# EUROPEAN PATENT SPECIFICATION

(11) **EP 4 271 345 B1**
(45) Date of publication and mention of the grant of the patent: **08.10.2025**
(21) Application number: 21914797.2
(22) Date of filing: 07.12.2021
(51) Int. Cl.: A61G 13/02, A61G 13/10, A61B 34/30, A61B 34/00, A61B 90/57

(54) **PENDANT FOR MOBILE MEDICAL PLATFORMS**
ANHÄNGER FÜR MOBILE MEDIZINISCHE PLATTFORMEN
PENDENTIF POUR PLATES-FORMES MÉDICALES MOBILES

(30) Priority: 30.12.2020 US 202063132437 P
(43) Date of publication of application: 08.11.2023
(73) Proprietor: Auris Health, Inc., Santa Clara, CA 95054 (US)
(72) Inventor: SCHMITT, Fabien Y., Redwood City, California 94065 (US); NORKOSKI, Christopher Michael, Redwood City, California 94065 (US); COVINGTON, Travis C., Redwood City, California 94065 (US); DOISNEAU, Anne Donahue, Redwood City, California 94065 (US); HASSAN, Alexander Tarek, San Francisco, California 94133 (US); LE ROUX, Eloi, San Francisco, California 94110 (US)
(74) Representative: Carpmaels & Ransford LLP
(86) International application number: PCT/IB2021/061420
(87) International publication number: WO 2022/144641

(56) References cited:
- EP-A1- 3 733 110
- EP-A1- 3 733 110
- EP-B1- 2 756 833
- CN-A- 111 956 402
- DE-A1- 102014 214 993
- US-A1- 2011 162 141
- US-A1- 2012 023 660
- US-A1- 2016 030 265
- US-A1- 2020 345 568

## Description

### TECHNICAL FIELD

The systems and methods disclosed herein are directed to control and operation of medical platforms, and more particularly to operation of mobility mechanisms and table top positioning functions of medical platforms, such as robotic surgical tables, mobile hospital beds, etc.

### BACKGROUND

Medical platforms, such as surgical tables or hospital beds, can be used to support a patient during a medical procedure. User control of a medical platform is important in operating the medical platform, including control of a table top orientation of the medical platform for patient positioning and movement of the medical platform for transportation. Conventionally, mobility (e.g., transportation) of a medical platform requires one or more persons to push or maneuver the medical platform manually, which is slow and laborious, and has a large footprint. During surgical setup, table top orientation of a medical platform is optionally controlled via a physicians' console or using a bedside pendent. In some cases, positioning of the medical platform, including positioning of table top of the medical platform, is performed manually.

EP3733110A1 describes a surgical robot terminal. US2016030265A1 describes a method and device for operating a mobile operating table. EP2756833B1 describes an operating table for an operating suite. DE102014214993A1 describes a patient support device and a method for displaying at least one state parameter of a patient support device.

### SUMMARY

Conventionally, transportation of the medical platform and adjustment of the table top orientation are typically performed by different people, and require scheduling coordination and communication during handoff, which can be cumbersome, time-consuming, and costly for hospitals. In addition, because more people may be required to handle the laborious process of transportation and positioning of the medical platform, the space requirement for the pathways and surgical rooms is high; otherwise, safety of the patient on the medical platform may be compromised during transportation or positioning of the medical platform in a cramped space. For at least these reasons, there is a need for methods and devices that provide centralized user control of mobility mechanisms for transporting the medical platform and table top mechanisms for adjusting an orientation of a table top of the medical platform. Furthermore, there is a need for methods and devices that provide bedside control of the powered mobility and orientation adjustment functions of the medical platform, and easy switching between the powered mobility control and orientation adjustment by a single user located at the bedside of the medical platform.

The invention is set out in the appended set of claims. Disclosed herein is a pendant (e.g., bedside pendant, pendant controller, handheld controller, input device, etc.) that allows a user to control both table top mechanisms and power-assisted mobility functions of the medical platform. Centralized control of the medical platform via the pendant allows a single user to seamlessly and effortlessly switch between operation of the medical platform in powered mobility mode (also referred to as bed control mode) (e.g., steering, driving, and transporting the medical platform as a whole) and in table top mode (also referred to as bed status mode) (e.g., orienting and positioning a table top of the medical platform into a desired pose). For example, a user may maneuver the medical platform down a hallway to a surgical suite using the pendant, adjust the position and orientation of the bed in the surgical suite relative to other equipment or personnel in the surgical suite; and after the medical platform is in place, use the pendant to change a position and/or orientation of the table top of the medical platform relative to the base of the medical platform to setup for a medical procedure, optionally, with the patient remaining on the table top of the medical platform throughout the entire process. The pendant also allows for precise movement and orientation of the medical platform and the table top of the medical platform, and can provide feedback to the user as the user provides inputs at the pendant for operating the medical platform. In addition, the user of the pendant is located close to the table top of the medical platform during operation of the pendant, such that the user could observe the patient's condition closely while controlling movement of the medical platform using the pendant. In some embodiments, the pendant is removably attached to (e.g., via a tether, a hook, or magnetically, etc.) a preset portion of the medical platform (e.g., head side of the table top, side railing, foot side of the table top, etc.) to allow easy access and easy storage of the pendant at or near the table top of the medical platform. In some embodiments, the pendant is, optionally, operable within a preset range (e.g., constrained via a tether, and/or preconfigured data communication range, etc.) from the table top of the medical platform, e.g., to ensure safe of patients and personnel during movement of the medical platform.

In accordance with some embodiments, a mobile medical platform (e.g., a surgical bed, a surgical table, a robotic surgical table, a medical platform, etc.) includes a table (e.g., a surgical table, a surgical bed, etc.), one or more robotic arms, one or more wheel assemblies, and a input device (e.g., a pendant, a pendant controller, a bed pendant, a portable device, a handheld input device, etc., coupled to the table). The table includes a base (e.g., a table base for a surgical bed, a rigid load-bearing housing, a chassis, a rigid base, etc.) and a table top (e.g., a platform or support surface that is rigidly coupled or movably coupled to the base). The one or more robotic arms are coupled (e.g., mechanically coupled, movably coupled, etc.) to the table (e.g., directly to the rigid base of the table, to the rigid base via a moveable arm support, or to the table top). The one or more wheel assemblies are coupled to the base to support and move the base in a physical environment, and a respective wheel assembly of the one or more wheel assemblies includes at least one motorized wheel (e.g., a wheel that is steered by a first motor, rolled by a second motor, or both). The input device is configured to receive user inputs of a first input type. The mobile medical platform also includes one or more processors and memory storing instructions, which, when executed by the one or more processors, cause the processors to perform one or more steps to operate the mobile medical platform. The mobile medical platform receives a first user input of the first input type (e.g., touch inputs (e.g., tap, swipe, press, touch and hold), movement of a joystick, press inputs, a combination of multiple preset input modalities) via the input device. In response to detecting the first user input of the first input type and in accordance with a determination that the first user input meets first criteria (e.g., criteria for recognizing an input that corresponds to a request to control mobility of the mobile medical platform by controlling the at least one motorized wheel (e.g., locking, unlocking, turning, rolling, stopping, and/or pivoting one or more of the at least one motorized wheel), including a combination of conditions, thresholds, and/or input type requirements), the mobile medical platform initiates first movement (e.g., steering or rolling movement, or both) of the at least one motorized wheel in accordance with the first user input of the first input type.

In some embodiments, the input device is configured to receive user inputs of a second input type (e.g., touch inputs (e.g., tap, swipe, press, touch and hold, etc.), movement of a joystick, press inputs, a combination of multiple preset input modalities, etc.) (e.g., same as the first input type, different from the first input type). The mobile medical platform receives a second user input of the second input type via the input device. In response to detecting the second user input of the first input type and in accordance with a determination that the second user input meets second criteria (e.g., criteria for recognizing an input that corresponds to a request to control the position and pose of the table top relative to the base (e.g., by controlling one or more motors coupled to the table (e.g., between the table top and the base, to the table top, etc.), including a combination of conditions, thresholds, and/or input type requirements), the mobile medical platform initiates second movement of the table top relative to the base (e.g., tilt, rotate, pan, translate vertically, translate horizontally the table top relative to the base, etc.) in accordance with the second user input of the second input type.

In some embodiments, the input device is a pendant that is coupled to the table (e.g., tethered by a wire, wireless communication such as Bluetooth, magnetically coupled to the frame of the table, etc.).

In some embodiments, the input device includes a first joystick and a second joystick.

In some embodiments, the input device includes a motion control affordance (e.g., motion enabled affordance). In accordance with a determination that the motion control affordance is activated (e.g., activated by a press input, pressed and continuously held down, etc.) while (e.g., concurrently with) user inputs are received by at least one of the first joystick and the second joystick, the mobile medical platform enables a movement control function of the first joystick and the second joystick (e.g., enables user control of the movement of the wheel assemblies via the first and second joysticks). In accordance with a determination that the motion control affordance is not activated while user inputs are received by at least one of the first joystick and the second joystick, the mobile medical platform disables the movement control function of the first joystick and the second joystick (e.g., disables user control of the movement of the wheel assemblies via the first and second joysticks, ignores the inputs received via the first and second joysticks, etc.).

In some embodiments, the first user input device includes a first visual indicator (e.g., a light ring, one or more lights, etc.) disposed adjacent to the first joystick (e.g., around a peripheral region of the first joystick, next to two or more boundary points of the input region of the first joystick, etc.), and a second visual indicator (e.g., a light ring, one or more lights, etc.) disposed adjacent to the second joystick (e.g., around a peripheral region of the first joystick, next to two or more boundary points of the input region of the first joystick, etc.). The first visual indicator is dynamically updated in accordance with characteristics of a movement input received via the first joystick, and the second visual indicator is dynamically updated in accordance with characteristics of a movement input received via the second joystick.

In some embodiments, the input device includes a touch sensitive display (e.g., a touch screen display, a touch-sensitive surface integrated with visual indicators that provide dynamically updated visual feedback and visual prompt for inputs, etc.). In accordance with a determination that the input device is in a first operation mode (e.g., bed mobility control mode), the mobile medical platform presents a first user interface for controlling mobility of the mobile medical platform, including one or more affordances for controlling the at least one powered wheel. In accordance with a determination that the input device is in a second operation mode (e.g., table top control mode), the mobile medical platform presents a second user interface for interacting with the table top, including one or more affordances for changing a position of the table top (e.g., pitch, roll, translate, move bed sections, flex head/foot sections, etc.) relative to the base.

In some embodiments, the input device includes a control affordance (e.g., a hardware lock/unlock button or switch). In accordance with a determination that the control affordance is activated (e.g., pressed, engaged, disengaged, etc.) at a first time, the mobile medical platform transitions from operating the input device in a first mode (e.g., mobility mode, bed control mode, etc.) to operating in a second mode (e.g., table top mode, bed status mode, etc.) that is different from the first mode. In accordance with a determination that the control affordance is activated (e.g., pressed again, disengaged, engaged, etc.) at a second time after the first time, the mobile medical platform transitions from operating the input device in the second mode to operating the input device in the first mode.

In some embodiments, the input device includes a display (e.g., a touch screen display, a LED display, an LCD display, etc.). In accordance with a determination that the input device is operating in the first mode, the mobile medical platform displays a first user interface with a first orientation (e.g., a landscape orientation) on the display. The first user interface includes one or more first affordances for controlling movement of the table. In accordance with a determination that the input device is operating in the second mode, the mobile medical platform displays a second user interface via the display with a second orientation (e.g., portrait orientation) on the display. The second orientation is different from the first orientation, and the second user interface includes one or more second affordances for reviewing a status of the mobile medical platform.

In some embodiments, in accordance with a determination the input device is operating in the first mode, the mobile medical platform orients the motorized wheels of the one or more wheel assemblies in a first predefined configuration (e.g., all wheels oriented in the same direction) to enable movement of the base in the physical environment. In accordance with a determination the input device is operating in the second mode, the mobile medical platform orients the motorized wheels of the one or more wheel assemblies in a second predefined configuration (e.g., braking configuration, preset braking configuration, a locked configuration, etc.) to restrict movement of the base in the physical environment, wherein the second predefined configuration is different from the first predefined configuration.

In some embodiments, the input device includes an accelerometer (e.g., 3-axis accelerometer, a gyro, a motion sensor, etc.). In response to detecting a falling motion of the input device using the accelerometer, the mobile medical platform activates a lock-out mode in which user inputs directed to the input device are ignored.

In accordance with some embodiments, an input device (e.g., a bed pendant, a portable device, a handheld input device, etc., coupled to the table) for controlling a mobile medical platform system (e.g., a surgical bed, a surgical table, a robotic surgical table, etc.) includes a bed, a base, and one or more robotic arms. The input device also includes a first input interface that is configured to receive user inputs of a first input type, a display, one or more processors, and memory storing instructions, which, when executed by the one or more processors, cause the processors to perform one or more steps to operate the input device. In accordance with a determination that the input device is operating in a first mode (e.g., mobility mode, bed control mode) (e.g., determined in accordance with a current orientation of the input device being the first orientation, and/or optionally, in accordance with activation of a motion enable control affordance, etc.), display, via the display, a first view of a user interface that corresponds to the first mode. The first view provides visual feedback regarding first movement (e.g., steering or rolling movement, or both) of the base of the mobile medical platform system in a physical environment that is initiated in accordance with a first user input of the first input type received through the first input interface. The memory further stores instructions, which, when executed by the one or more processors, cause the processors to detect a transition event that transitions the input device from the first mode to a second mode (e.g., table top mode, bed status mode) distinct from the first mode, and in response to detecting the transition event that transitions the input device from the first mode to the second mode, the input device displays a second view of the user interface that corresponds to the second mode. The second view provides visual feedback regarding a current positional status of the bed and/or the one or more robotic arms of the mobile medical platform system (e.g., arm positions relative to the bed, arm and bed positions relative to the base, etc.).

In some embodiments, the first view of the user interface that corresponds to the first mode has a first orientation (e.g., landscape format), and the second view of the user interface that corresponds to the second mode has a second orientation (e.g., landscape format) that is different from (e.g., orthogonal to) the first orientation.

In some embodiments, the input device includes a first joystick and a second joystick different from the first joystick.

In some embodiments, the input device includes a first visual indicator (e.g., a light ring, one or more lights, etc.) that is disposed adjacent to the first joystick (e.g., around a peripheral region of the first joystick, next to two or more boundary points of the input region of the first joystick, etc.), and a second visual indicator (e.g., a light ring, one or more lights, etc.) that is disposed adjacent to the second joystick (e.g., around a peripheral region of the first joystick, next to two or more boundary points of the input region of the first joystick, etc.). The first visual indicator is dynamically updated in accordance with characteristics of a movement input received via the first joystick, and the second visual indicator is dynamically updated in accordance with characteristics of a movement input received via the second joystick.

In some embodiments, the input device includes a motion control affordance (e.g., motion enable affordance, motion control affordance). In accordance with a determination that the motion control affordance is activated (e.g., activated by a press input, pressed and continuously held down, placed into or maintained in an engaged state, etc.) while (e.g., concurrently with) user inputs are received by at least one of the first joystick and the second joystick, the input device enables a movement control function of the first joystick and the second joystick (e.g., enabling user control of the movement of the wheel assemblies via the first and second joysticks). In accordance with a determination that the motion control affordance is not activated while user inputs are received by at least one of the first joystick and the second joystick, the user input device disables the movement control function of the first joystick and the second joystick.

In some embodiments, in accordance with a determination that the motion control affordance is activated while the input device is in the second mode, the input device automatically transitions from the second mode to the first mode.

In some embodiments, the input device includes a control affordance (e.g., lock/unlock button) for transitioning the input device between the first mode and the second mode. The transition event for transitioning the input device between the first mode and the second mode includes detection of a preset user input (e.g., pressing the button, flipping a switch, actuate and hold a button and switch, etc.) via the control affordance of the input device.

In some embodiments, the mobile medical platform system includes one or more wheel assemblies. A respective wheel assembly of the one or more wheel assemblies includes at least one motorized wheel. In response to detecting the transition event that transitions the input device from the first mode to the second mode and in accordance with a determination the input device is in the second mode, the motorized wheels of the one or more wheel assemblies are oriented in a predefined braking configuration to restrict movement of the mobile medical platform system. The memory further stores instructions, which, when executed by the one or more processors, cause the processors to detect a second transition event that transitions the input device from the second mode to a first mode. In response to detecting the second transition event that transitions the input device from the second mode to the first mode and in accordance with a determination the input device is in the first mode, the motorized wheels of the one or more wheel assemblies are oriented in a first predefined configuration (e.g., all wheels oriented in the same direction) to enable movement of the bed. The first predefined configuration is different from the predefined braking configuration.

In some embodiments, the input device includes an accelerometer (e.g., 3-axis accelerometer, a gyro, a motion sensor, etc.). The memory further stores instructions, which, when executed by the one or more processors, cause the processors to, in response to detecting a falling motion of the input device, activate a lock-out mode of the input device in which user inputs (e.g., button press, joystick movement, touch screen contact, any user input) directed to the input device are ignored (e.g., not detected, not processed, etc.).

### BRIEF DESCRIPTION OF THE DRAWINGS

The disclosed aspects will hereinafter be described in conjunction with the appended drawings, provided to illustrate and not to limit the disclosed aspects, wherein like designations denote like elements.
FIG. 1 illustrates an embodiment of a cart-based robotic system arranged for diagnostic and/or therapeutic bronchoscopy procedure(s).
FIG. 2 depicts further aspects of the robotic system of FIG. 1.
FIG. 3 illustrates an embodiment of the robotic system of FIG. 1 arranged for ureteroscopy.
FIG. 4 illustrates an embodiment of the robotic system of FIG. 1 arranged for a vascular procedure.
FIG. 5 illustrates an embodiment of a table-based robotic system arranged for a bronchoscopy procedure.
FIG. 6 provides an alternative view of the robotic system of FIG. 5.
FIG. 7 illustrates an example system configured to stow robotic arm(s).
FIG. 8 illustrates an embodiment of a table-based robotic system configured for a ureteroscopy procedure.
FIG. 9 illustrates an embodiment of a table-based robotic system configured for a laparoscopic procedure.
FIG. 10 illustrates an embodiment of the table-based robotic system of FIGS. 5-9 with pitch or tilt adjustment.
FIG. 11 provides a detailed illustration of the interface between the table and the column of the table-based robotic system of FIGS. 5-10.
FIG. 12 illustrates an alternative embodiment of a table-based robotic system.
FIG. 13 illustrates an end view of the table-based robotic system of FIG. 12.
FIG. 14 illustrates an end view of a table-based robotic system with robotic arms attached thereto.
FIG. 15 illustrates an exemplary instrument driver.
FIG. 16 illustrates an exemplary medical instrument with a paired instrument driver.
FIG. 17 illustrates an alternative design for an instrument driver and instrument where the axes of the drive units are parallel to the axis of the elongated shaft of the instrument.
FIG. 18 illustrates an instrument having an instrument-based insertion architecture.
FIG. 19 illustrates an exemplary controller.
FIG. 20 depicts a block diagram illustrating a localization system that estimates a location of one or more elements of the robotic systems of FIGS. 1-10, such as the location of the instrument of FIGS. 16-18, in accordance to an example embodiment.
FIG. 21A illustrates an embodiment of a mobile medical platform that includes one or more wheel assemblies in accordance with some embodiments.
FIG. 21B illustrates an embodiment of a mobile medical platform that includes an input device in accordance with some embodiments.
FIG. 22A - 22B illustrate embodiments of an input device for controlling operation of the mobile medical platform of FIGs. 21A and 21B in accordance with some embodiments.
FIG. 22C illustrates visual feedback at the input device of FIGs. 22A and 22B in accordance with some embodiments.
FIG. 22D illustrates a back view of the input device of FIGs. 22A and 22B in accordance with some embodiments.
FIG. 23A illustrates examples of user interfaces at a display of the input device of FIGs. 22A and 22B for controlling assisted mobility of the mobile medical platform of FIGs. 21A and 21B in accordance with some embodiments.
FIGs. 23B - 23D illustrate different wheel configurations the mobile medical platform 200 of FIGs. 21A and 21B in accordance with some embodiments.
FIG. 24A illustrates examples of user interfaces at a display of the input device of FIGs. 22A and 22B for operating a table top of the mobile medical platform of FIGs. 21A and 21B in accordance with some embodiments.
FIG. 24B illustrates vertical translation of a table top of the mobile medical platform of FIGs. 21A and 21B in accordance with some embodiments.
FIG. 24C illustrates lateral translation of a table top of the mobile medical platform of FIGs. 21A and 21B in accordance with some embodiments.
FIG. 24D illustrates rotation of a table top along a longitudinal axis of the mobile medical platform of FIGs. 21A and 21B in accordance with some embodiments.
FIG. 24E illustrates rotation of a table top along a lateral axis of the mobile medical platform of FIGs. 21A and 21B in accordance with some embodiments.
FIG. 24F illustrates an embodiment of a table top of the mobile medical platform of FIGs. 21A and 21B in accordance with some embodiments.
FIGS. 25A - 25D show a flowchart illustrating a method performed by an input device for controlling operations of the mobile medical platform of FIGs. 21A and 21B in accordance with some embodiments.
FIGS. 26A - 26C show a flowchart illustrating a method performed by an input device for controlling operations of the mobile medical platform of FIGs. 21A and 21B in accordance with some embodiments.
FIG. 27 is a schematic diagram illustrating electronic components of a mobile medical platform in accordance with some embodiments.

### DETAILED DESCRIPTION

### 1. Overview.

Aspects of the present disclosure may be integrated into a robotically-enabled medical system capable of performing a variety of medical procedures, including both minimally invasive, such as laparoscopy, and non-invasive, such as endoscopy, procedures. Among endoscopy procedures, the system may be capable of performing bronchoscopy, ureteroscopy, gastroscopy, etc.

In addition to performing the breadth of procedures, the system may provide additional benefits, such as enhanced imaging and guidance to assist the physician. Additionally, the system may provide the physician with the ability to perform the procedure from an ergonomic position without the need for awkward arm motions and positions. Still further, the system may provide the physician with the ability to perform the procedure with improved ease of use such that one or more of the instruments of the system can be controlled by a single user.

Various embodiments will be described below in conjunction with the drawings for purposes of illustration. It should be appreciated that many other embodiments of the disclosed concepts are possible, and various advantages can be achieved with the disclosed embodiments. Headings are included herein for reference and to aid in locating various sections. These headings are not intended to limit the scope of the concepts described with respect thereto. Such concepts may have applicability throughout the entire specification.

### A. Robotic System - Cart.

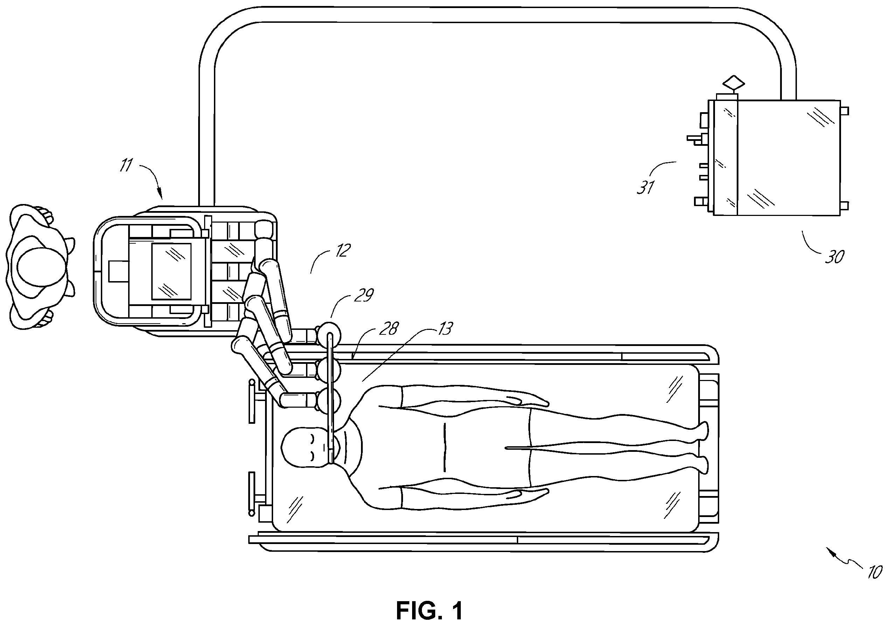

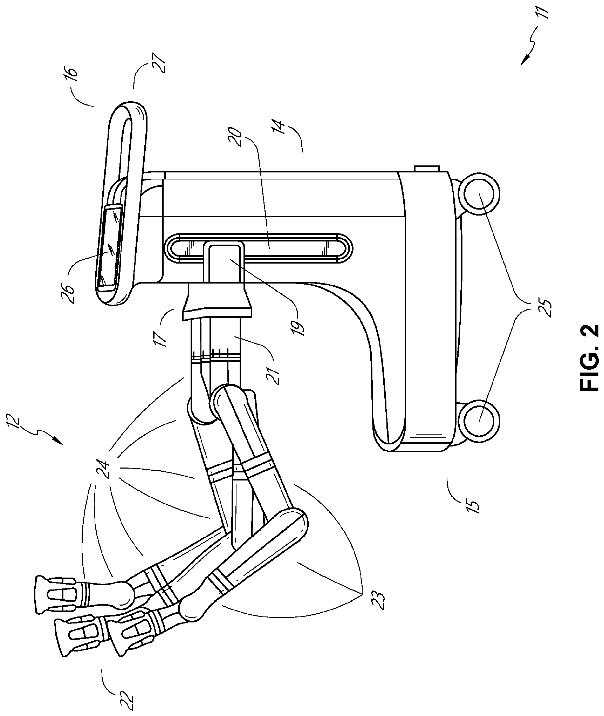

The robotically-enabled medical system may be configured in a variety of ways depending on the particular procedure. FIG. 1 illustrates an embodiment of a cart-based robotically-enabled system 10 arranged for a diagnostic and/or therapeutic bronchoscopy procedure. During a bronchoscopy, the system 10 may comprise a cart 11 having one or more robotic arms 12 to deliver a medical instrument, such as a steerable endoscope 13, which may be a procedure-specific bronchoscope for bronchoscopy, to a natural orifice access point (i.e., the mouth of the patient positioned on a table in the present example) to deliver diagnostic and/or therapeutic tools. As shown, the cart 11 may be positioned proximate to the patient's upper torso in order to provide access to the access point. Similarly, the robotic arms 12 may be actuated to position the bronchoscope relative to the access point. The arrangement in FIG. 1 may also be utilized when performing a gastro-intestinal (GI) procedure with a gastroscope, a specialized endoscope for GI procedures. FIG. 2 depicts an example embodiment of the cart in greater detail.

With continued reference to FIG. 1, once the cart 11 is properly positioned, the robotic arms 12 may insert the steerable endoscope 13 into the patient robotically, manually, or a combination thereof. As shown, the steerable endoscope 13 may comprise at least two telescoping parts, such as an inner leader portion and an outer sheath portion, each portion coupled to a separate instrument driver from the set of instrument drivers 28, each instrument driver coupled to the distal end of an individual robotic arm. This linear arrangement of the instrument drivers 28, which facilitates coaxially aligning the leader portion with the sheath portion, creates a "virtual rail" 29 that may be repositioned in space by manipulating the one or more robotic arms 12 into different angles and/or positions. The virtual rails described herein are depicted in the Figures using dashed lines, and accordingly the dashed lines do not depict any physical structure of the system. Translation of the instrument drivers 28 along the virtual rail 29 telescopes the inner leader portion relative to the outer sheath portion or advances or retracts the endoscope 13 from the patient. The angle of the virtual rail 29 may be adjusted, translated, and pivoted based on clinical application or physician preference. For example, in bronchoscopy, the angle and position of the virtual rail 29 as shown represents a compromise between providing physician access to the endoscope 13 while minimizing friction that results from bending the endoscope 13 into the patient's mouth.

The endoscope 13 may be directed down the patient's trachea and lungs after insertion using precise commands from the robotic system until reaching the target destination or operative site. In order to enhance navigation through the patient's lung network and/or reach the desired target, the endoscope 13 may be manipulated to telescopically extend the inner leader portion from the outer sheath portion to obtain enhanced articulation and greater bend radius. The use of separate instrument drivers 28 also allows the leader portion and sheath portion to be driven independent of each other.

For example, the endoscope 13 may be directed to deliver a biopsy needle to a target, such as, for example, a lesion or nodule within the lungs of a patient. The needle may be deployed down a working channel that runs the length of the endoscope to obtain a tissue sample to be analyzed by a pathologist. Depending on the pathology results, additional tools may be deployed down the working channel of the endoscope for additional biopsies. After identifying a nodule to be malignant, the endoscope 13 may endoscopically deliver tools to resect the potentially cancerous tissue. In some instances, diagnostic and therapeutic treatments can be delivered in separate procedures. In those circumstances, the endoscope 13 may also be used to deliver a fiducial to "mark" the location of the target nodule as well. In other instances, diagnostic and therapeutic treatments may be delivered during the same procedure.

The system 10 may also include a movable tower 30, which may be connected via support cables to the cart 11 to provide support for controls, electronics, fluidics, optics, sensors, and/or power to the cart 11. Placing such functionality in the tower 30 allows for a smaller form factor cart 11 that may be more easily adjusted and/or re-positioned by an operating physician and his/her staff. Additionally, the division of functionality between the cart / table and the support tower 30 reduces operating room clutter and facilitates improving clinical workflow. While the cart 11 may be positioned close to the patient, the tower 30 may be stowed in a remote location to stay out of the way during a procedure.

In support of the robotic systems described above, the tower 30 may include component(s) of a computer-based control system that stores computer program instructions, for example, within a non-transitory computer-readable storage medium such as a persistent magnetic storage drive, solid state drive, etc. The execution of those instructions, whether the execution occurs in the tower 30 or the cart 11, may control the entire system or sub-system(s) thereof. For example, when executed by a processor of the computer system, the instructions may cause the components of the robotics system to actuate the relevant carriages and arm mounts, actuate the robotics arms, and control the medical instruments. For example, in response to receiving the control signal, the motors in the joints of the robotics arms may position the arms into a certain posture.

The tower 30 may also include a pump, flow meter, valve control, and/or fluid access in order to provide controlled irrigation and aspiration capabilities to the system that may be deployed through the endoscope 13. These components may also be controlled using the computer system of tower 30. In some embodiments, irrigation and aspiration capabilities may be delivered directly to the endoscope 13 through separate cable(s).

The tower 30 may include a voltage and surge protector designed to provide filtered and protected electrical power to the cart 11, thereby avoiding placement of a power transformer and other auxiliary power components in the cart 11, resulting in a smaller, more moveable cart 11.

The tower 30 may also include support equipment for the sensors deployed throughout the robotic system 10. For example, the tower 30 may include opto-electronics equipment for detecting, receiving, and processing data received from the optical sensors or cameras throughout the robotic system 10. In combination with the control system, such opto-electronics equipment may be used to generate real-time images for display in any number of consoles deployed throughout the system, including in the tower 30. Similarly, the tower 30 may also include an electronic subsystem for receiving and processing signals received from deployed electromagnetic (EM) sensors. The tower 30 may also be used to house and position an EM field generator for detection by EM sensors in or on the medical instrument.

The tower 30 may also include a console 31 in addition to other consoles available in the rest of the system, e.g., console mounted on top of the cart. The console 31 may include a user interface and a display screen, such as a touchscreen, for the physician operator. Consoles in system 10 are generally designed to provide both robotic controls as well as pre-operative and real-time information of the procedure, such as navigational and localization information of the endoscope 13. When the console 31 is not the only console available to the physician, it may be used by a second operator, such as a nurse, to monitor the health or vitals of the patient and the operation of system, as well as provide procedure-specific data, such as navigational and localization information. In other embodiments, the console 31 is housed in a body that is separate from the tower 30.

The tower 30 may be coupled to the cart 11 and endoscope 13 through one or more cables or connections (not shown). In some embodiments, the support functionality from the tower 30 may be provided through a single cable to the cart 11, simplifying and decluttering the operating room. In other embodiments, specific functionality may be coupled in separate cabling and connections. For example, while power may be provided through a single power cable to the cart, the support for controls, optics, fluidics, and/or navigation may be provided through a separate cable.

FIG. 2 provides a detailed illustration of an embodiment of the cart from the cart-based robotically-enabled system shown in FIG. 1. The cart 11 generally includes an elongated support structure 14 (often referred to as a "column"), a cart base 15, and a console 16 at the top of the column 14. The column 14 may include one or more carriages, such as a carriage 17 (alternatively "arm support") for supporting the deployment of one or more robotic arms 12 (three shown in FIG. 2). The carriage 17 may include individually configurable arm mounts that rotate along a perpendicular axis to adjust the base of the robotic arms 12 for better positioning relative to the patient. The carriage 17 also includes a carriage interface 19 that allows the carriage 17 to vertically translate along the column 14.

The carriage interface 19 is connected to the column 14 through slots, such as slot 20, that are positioned on opposite sides of the column 14 to guide the vertical translation of the carriage 17. The slot 20 contains a vertical translation interface to position and hold the carriage at various vertical heights relative to the cart base 15. Vertical translation of the carriage 17 allows the cart 11 to adjust the reach of the robotic arms 12 to meet a variety of table heights, patient sizes, and physician preferences. Similarly, the individually configurable arm mounts on the carriage 17 allow the robotic arm base 21 of robotic arms 12 to be angled in a variety of configurations.

In some embodiments, the slot 20 may be supplemented with slot covers that are flush and parallel to the slot surface to prevent dirt and fluid ingress into the internal chambers of the column 14 and the vertical translation interface as the carriage 17 vertically translates. The slot covers may be deployed through pairs of spring spools positioned near the vertical top and bottom of the slot 20. The covers are coiled within the spools until deployed to extend and retract from their coiled state as the carriage 17 vertically translates up and down. The spring-loading of the spools provides force to retract the cover into a spool when carriage 17 translates towards the spool, while also maintaining a tight seal when the carriage 17 translates away from the spool. The covers may be connected to the carriage 17 using, for example, brackets in the carriage interface 19 to ensure proper extension and retraction of the cover as the carriage 17 translates.

The column 14 may internally comprise mechanisms, such as gears and motors, that are designed to use a vertically aligned lead screw to translate the carriage 17 in a mechanized fashion in response to control signals generated in response to user inputs, e.g., inputs from the console 16.

The robotic arms 12 may generally comprise robotic arm bases 21 and end effectors 22, separated by a series of linkages 23 that are connected by a series of joints 24, each joint comprising an independent actuator, each actuator comprising an independently controllable motor. Each independently controllable joint represents an independent degree of freedom available to the robotic arm. Each of the arms 12 have seven joints, and thus provide seven degrees of freedom. A multitude of joints result in a multitude of degrees of freedom, allowing for "redundant" degrees of freedom. Redundant degrees of freedom allow the robotic arms 12 to position their respective end effectors 22 at a specific position, orientation, and trajectory in space using different linkage positions and joint angles. This allows for the system to position and direct a medical instrument from a desired point in space while allowing the physician to move the arm joints into a clinically advantageous position away from the patient to create greater access, while avoiding arm collisions.

The cart base 15 balances the weight of the column 14, carriage 17, and arms 12 over the floor. Accordingly, the cart base 15 houses heavier components, such as electronics, motors, power supply, as well as components that either enable movement and/or immobilize the cart. For example, the cart base 15 includes one or more wheel assemblies that allow for the cart to easily move around the room prior to a procedure. After reaching the appropriate position, wheels of the one of more wheel assemblies may be immobilized using wheel locks or may be arranged in a preset braking configuration that maintains the cart 11 immobilized during the procedure.

Positioned at the vertical end of column 14, the console 16 allows for both a user interface for receiving user input and a display screen (or a dual-purpose device such as, for example, a touchscreen 26) to provide the physician user with both pre-operative and intra-operative data. Potential pre-operative data on the touchscreen 26 may include pre-operative plans, navigation and mapping data derived from pre-operative computerized tomography (CT) scans, and/or notes from pre-operative patient interviews. Intra-operative data on display may include optical information provided from the tool, sensor and coordinate information from sensors, as well as vital patient statistics, such as respiration, heart rate, and/or pulse. The console 16 may be positioned and tilted to allow a physician to access the console from the side of the column 14 opposite carriage 17. From this position, the physician may view the console 16, robotic arms 12, and patient while operating the console 16 from behind the cart 11. As shown, the console 16 also includes a handle 27 to assist with maneuvering and stabilizing cart 11.

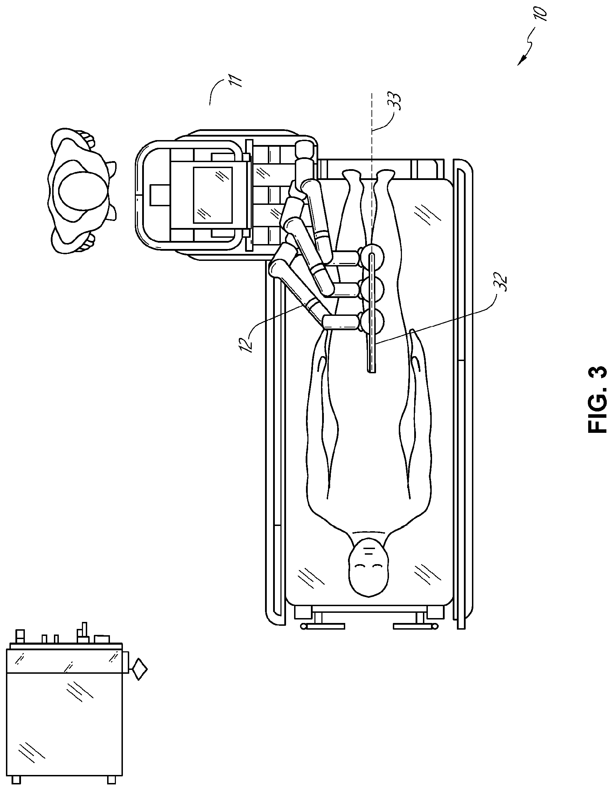

FIG. 3 illustrates an embodiment of a robotically-enabled system 10 arranged for ureteroscopy. In a ureteroscopic procedure, the cart 11 may be positioned to deliver a ureteroscope 32, a procedure-specific endoscope designed to traverse a patient's urethra and ureter, to the lower abdominal area of the patient. In a ureteroscopy, it may be desirable for the ureteroscope 32 to be directly aligned with the patient's urethra to reduce friction and forces on the sensitive anatomy in the area. As shown, the cart 11 may be aligned at the foot of the table to allow the robotic arms 12 to position the ureteroscope 32 for direct linear access to the patient's urethra. From the foot of the table, the robotic arms 12 may insert the ureteroscope 32 along the virtual rail 33 directly into the patient's lower abdomen through the urethra.

After insertion into the urethra, using similar control techniques as in bronchoscopy, the ureteroscope 32 may be navigated into the bladder, ureters, and/or kidneys for diagnostic and/or therapeutic applications. For example, the ureteroscope 32 may be directed into the ureter and kidneys to break up kidney stone build up using a laser or ultrasonic lithotripsy device deployed down the working channel of the ureteroscope 32. After lithotripsy is complete, the resulting stone fragments may be removed using baskets deployed down the ureteroscope 32.

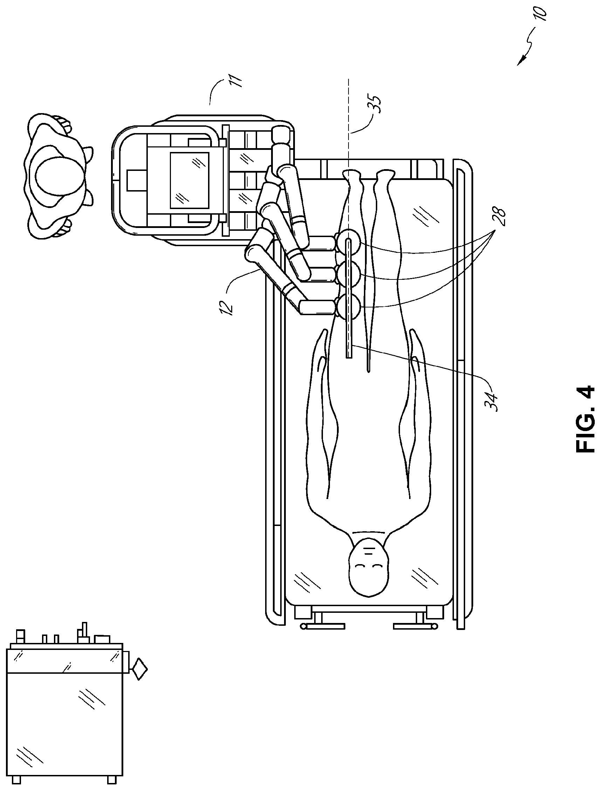

FIG. 4 illustrates an embodiment of a robotically-enabled system similarly arranged for a vascular procedure. In a vascular procedure, the system 10 may be configured such that the cart 11 may deliver a medical instrument 34, such as a steerable catheter, to an access point in the femoral artery in the patient's leg. The femoral artery presents both a larger diameter for navigation as well as a relatively less circuitous and tortuous path to the patient's heart, which simplifies navigation. As in a ureteroscopic procedure, the cart 11 may be positioned towards the patient's legs and lower abdomen to allow the robotic arms 12 to provide a virtual rail 35 with direct linear access to the femoral artery access point in the patient's thigh / hip region. After insertion into the artery, the medical instrument 34 may be directed and inserted by translating the instrument drivers 28. Alternatively, the cart may be positioned around the patient's upper abdomen in order to reach alternative vascular access points, such as, for example, the carotid and brachial arteries near the shoulder and wrist.

### B. Robotic System - Table.

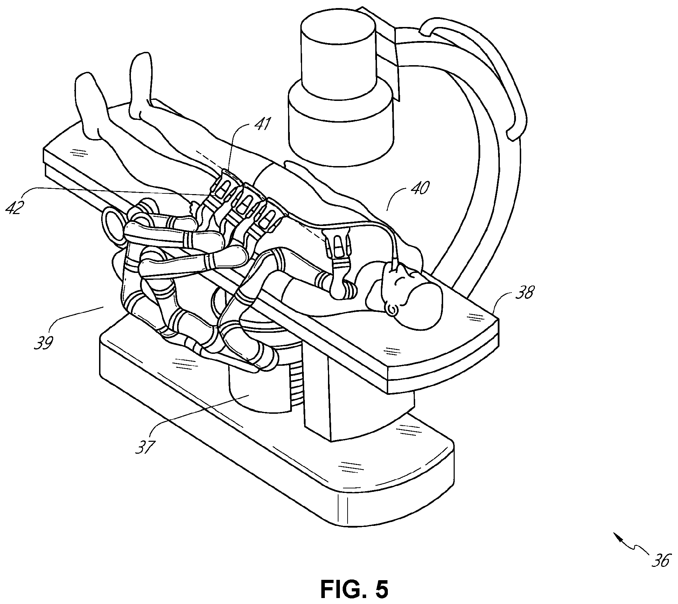

Embodiments of the robotically-enabled medical system may also incorporate the patient's table. Incorporation of the table reduces the amount of capital equipment within the operating room by removing the cart, which allows greater access to the patient. FIG. 5 illustrates an embodiment of such a robotically-enabled system arranged for a bronchoscopy procedure. System 36 includes a support structure or column 37 for supporting platform 38 (shown as a "table" or "bed") over the floor. Much like in the cart-based systems, the end effectors of the robotic arms 39 of the system 36 comprise instrument drivers 42 that are designed to manipulate an elongated medical instrument, such as a bronchoscope 40 in FIG. 5, through or along a virtual rail 41 formed from the linear alignment of the instrument drivers 42. In practice, a C-arm for providing fluoroscopic imaging may be positioned over the patient's upper abdominal area by placing the emitter and detector around table 38.

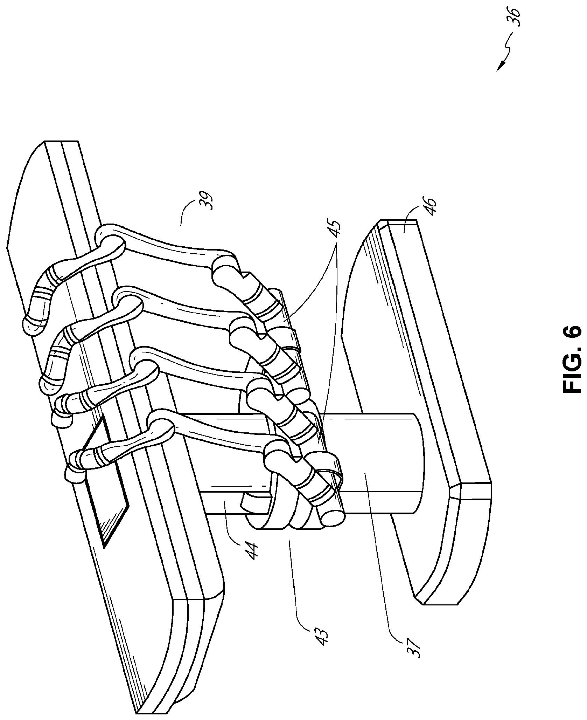

FIG. 6 provides an alternative view of the system 36 without the patient and medical instrument for discussion purposes. As shown, the column 37 may include one or more carriages 43 shown as ring-shaped in the system 36, from which the one or more robotic arms 39 may be based. The carriages 43 may translate along a vertical column interface 44 that runs the length of the column 37 to provide different vantage points from which the robotic arms 39 may be positioned to reach the patient. The carriage(s) 43 may rotate around the column 37 using a mechanical motor positioned within the column 37 to allow the robotic arms 39 to have access to multiples sides of the table 38, such as, for example, both sides of the patient. In embodiments with multiple carriages, the carriages may be individually positioned on the column and may translate and/or rotate independent of the other carriages. While carriages 43 need not surround the column 37 or even be circular, the ring-shape as shown facilitates rotation of the carriages 43 around the column 37 while maintaining structural balance. Rotation and translation of the carriages 43 allows the system to align the medical instruments, such as endoscopes and laparoscopes, into different access points on the patient. In other embodiments (not shown), the system 36 can include a patient table or bed with adjustable arm supports in the form of bars or rails extending alongside it. One or more robotic arms 39 (e.g., via a shoulder with an elbow joint) can be attached to the adjustable arm supports, which can be vertically adjusted. By providing vertical adjustment, the robotic arms 39 are advantageously capable of being stowed compactly beneath the patient table or bed, and subsequently raised during a procedure.

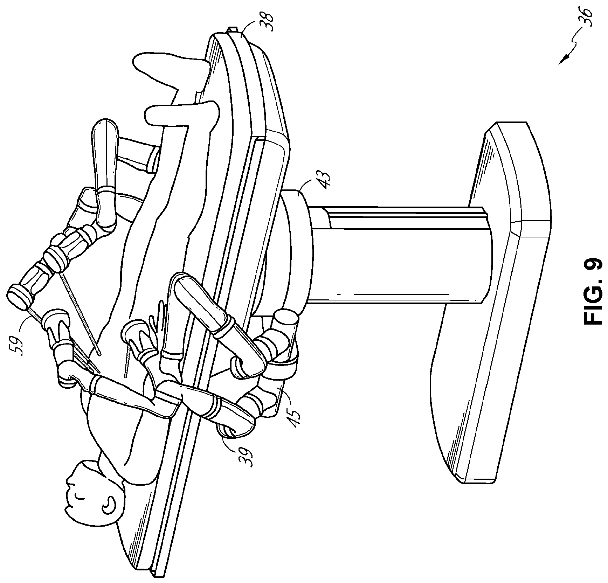

The arms 39 may be mounted on the carriages through a set of arm mounts 45 comprising a series of joints that may individually rotate and/or telescopically extend to provide additional configurability to the robotic arms 39. Additionally, the arm mounts 45 may be positioned on the carriages 43 such that, when the carriages 43 are appropriately rotated, the arm mounts 45 may be positioned on either the same side of table 38 (as shown in FIG. 6), on opposite sides of table 38 (as shown in FIG. 9), or on adjacent sides of the table 38 (not shown).

The column 37 structurally provides support for the table 38, and a path for vertical translation of the carriages. Internally, the column 37 may be equipped with lead screws for guiding vertical translation of the carriages, and motors to mechanize the translation of said carriages based the lead screws. The column 37 may also convey power and control signals to the carriage 43 and robotic arms 39 mounted thereon.

The table base 46 serves a similar function as the cart base 15 in cart 11 shown in FIG. 2, housing heavier components to balance the table/bed 38, the column 37, the carriages 43, and the robotic arms 39. The table base 46 may also incorporate rigid casters to provide stability during procedures. Deployed from the bottom of the table base 46, the casters may extend in opposite directions on both sides of the base 46 and retract when the system 36 needs to be moved.

Continuing with FIG. 6, the system 36 may also include a tower (not shown) that divides the functionality of system 36 between table and tower to reduce the form factor and bulk of the table. As in earlier disclosed embodiments, the tower may provide a variety of support functionalities to table, such as processing, computing, and control capabilities, power, fluidics, and/or optical and sensor processing. The tower may also be movable to be positioned away from the patient to improve physician access and de-clutter the operating room. Additionally, placing components in the tower allows for more storage space in the table base for potential stowage of the robotic arms. The tower may also include a master controller or console that provides both a user interface for user input, such as keyboard and/or pendant, as well as a display screen (or touchscreen) for pre-operative and intra-operative information, such as real-time imaging, navigation, and tracking information. In some embodiments, the tower may also contain holders for gas tanks to be used for insufflation.

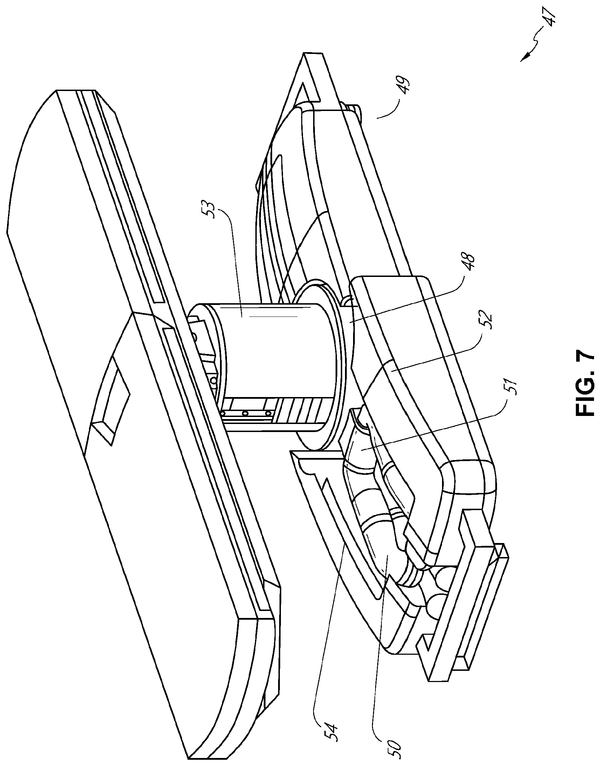

In some embodiments, a table base may stow and store the robotic arms when not in use. FIG. 7 illustrates a system 47 that stows robotic arms in an embodiment of the table-based system. In system 47, carriages 48 may be vertically translated into base 49 to stow robotic arms 50, arm mounts 51, and the carriages 48 within the base 49. Base covers 52 may be translated and retracted open to deploy the carriages 48, arm mounts 51, and arms 50 around column 53, and closed to stow to protect them when not in use. The base covers 52 may be sealed with a membrane 54 along the edges of its opening to prevent dirt and fluid ingress when closed.

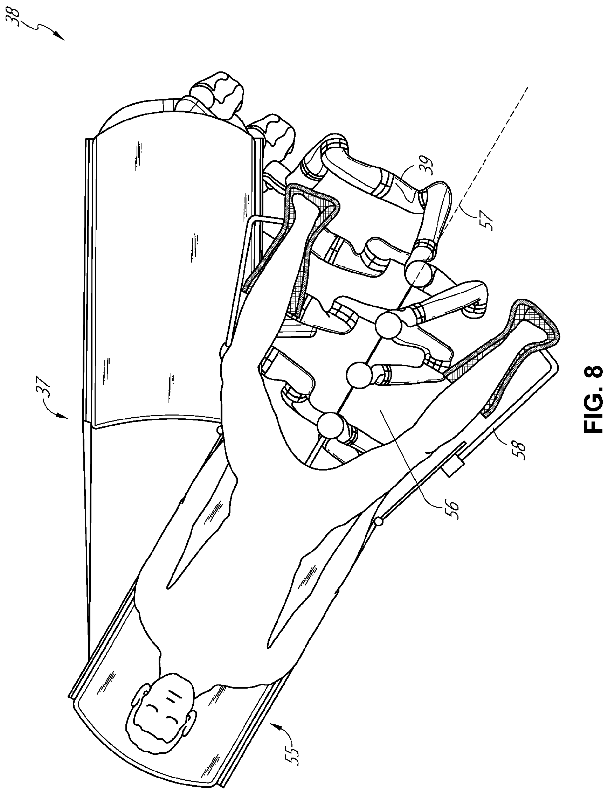

FIG. 8 illustrates an embodiment of a robotically-enabled table-based system configured for a ureteroscopy procedure. In a ureteroscopy, the table 38 may include a swivel portion 55 for positioning a patient off-angle from the column 37 and table base 46. The swivel portion 55 may rotate or pivot around a pivot point (e.g., located below the patient's head) in order to position the bottom portion of the swivel portion 55 away from the column 37. For example, the pivoting of the swivel portion 55 allows a C-arm (not shown) to be positioned over the patient's lower abdomen without competing for space with the column (not shown) below table 38. By rotating the carriage (not shown) around the column 37, the robotic arms 39 may directly insert a ureteroscope 56 along a virtual rail 57 into the patient's groin area to reach the urethra. In a ureteroscopy, stirrups 58 may also be fixed to the swivel portion 55 of the table 38 to support the position of the patient's legs during the procedure and allow clear access to the patient's groin area.

In a laparoscopic procedure, through small incision(s) in the patient's abdominal wall, minimally invasive instruments may be inserted into the patient's anatomy. In some embodiments, the minimally invasive instruments comprise an elongated rigid member, such as a shaft, which is used to access anatomy within the patient. After inflation of the patient's abdominal cavity, the instruments may be directed to perform surgical or medical tasks, such as grasping, cutting, ablating, suturing, etc. In some embodiments, the instruments can comprise a scope, such as a laparoscope. FIG. 9 illustrates an embodiment of a robotically-enabled table-based system configured for a laparoscopic procedure. As shown in FIG. 9, the carriages 43 of the system 36 may be rotated and vertically adjusted to position pairs of the robotic arms 39 on opposite sides of the table 38, such that instrument 59 may be positioned using the arm mounts 45 to be passed through minimal incisions on both sides of the patient to reach his/her abdominal cavity.

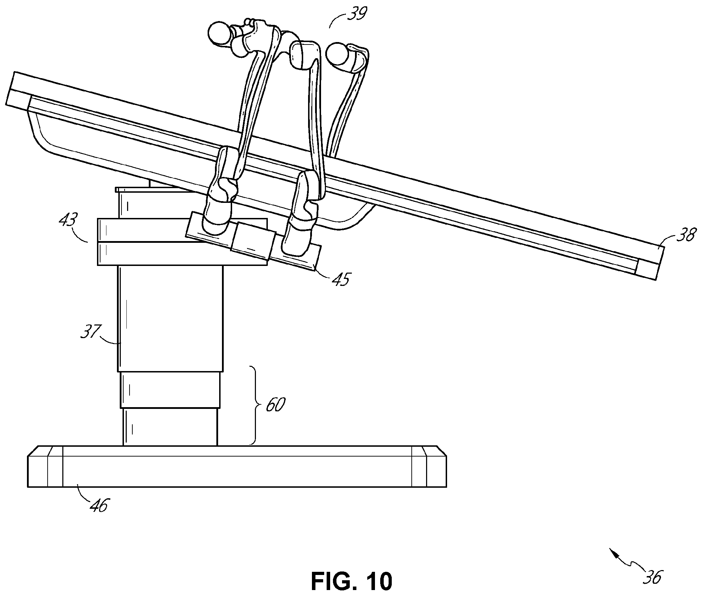

To accommodate laparoscopic procedures, the robotically-enabled table system may also tilt the platform to a desired angle. FIG. 10 illustrates an embodiment of the robotically-enabled medical system with pitch or tilt adjustment. As shown in FIG. 10, the system 36 may accommodate tilt of the table 38 to position one portion of the table at a greater distance from the floor than the other. Additionally, the arm mounts 45 may rotate to match the tilt such that the arms 39 maintain the same planar relationship with table 38. To accommodate steeper angles, the column 37 may also include telescoping portions 60 that allow vertical extension of column 37 to keep the table 38 from touching the floor or colliding with base 46.

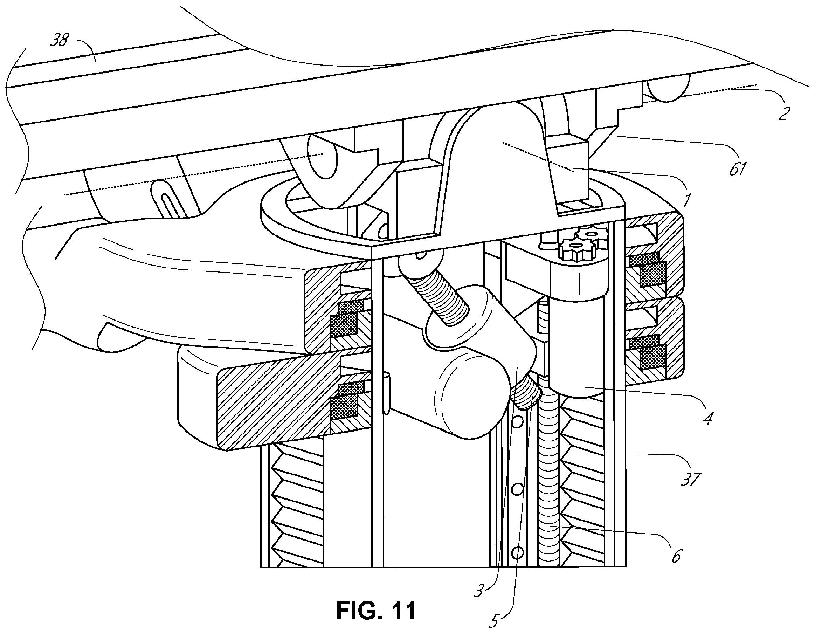

FIG. 11 provides a detailed illustration of the interface between the table 38 and the column 37. Pitch rotation mechanism 61 may be configured to alter the pitch angle of the table 38 relative to the column 37 in multiple degrees of freedom. The pitch rotation mechanism 61 may be enabled by the positioning of orthogonal axes 1, 2 at the column-table interface, each axis actuated by a separate motor 3, 4 responsive to an electrical pitch angle command. Rotation along one screw 5 would enable tilt adjustments in one axis 1, while rotation along the other screw 6 would enable tilt adjustments along the other axis 2. In some embodiments, a ball joint can be used to alter the pitch angle of the table 38 relative to the column 37 in multiple degrees of freedom.

For example, pitch adjustments are particularly useful when trying to position the table in a Trendelenburg position, i.e., position the patient's lower abdomen at a higher position from the floor than the patient's lower abdomen, for lower abdominal surgery. The Trendelenburg position causes the patient's internal organs to slide towards his/her upper abdomen through the force of gravity, clearing out the abdominal cavity for minimally invasive tools to enter and perform lower abdominal surgical or medical procedures, such as laparoscopic prostatectomy.

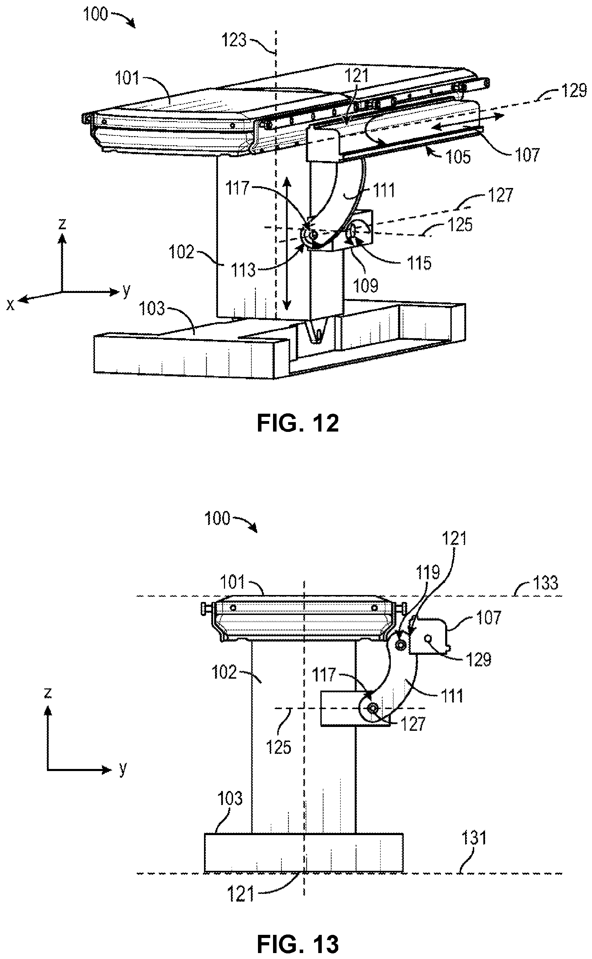

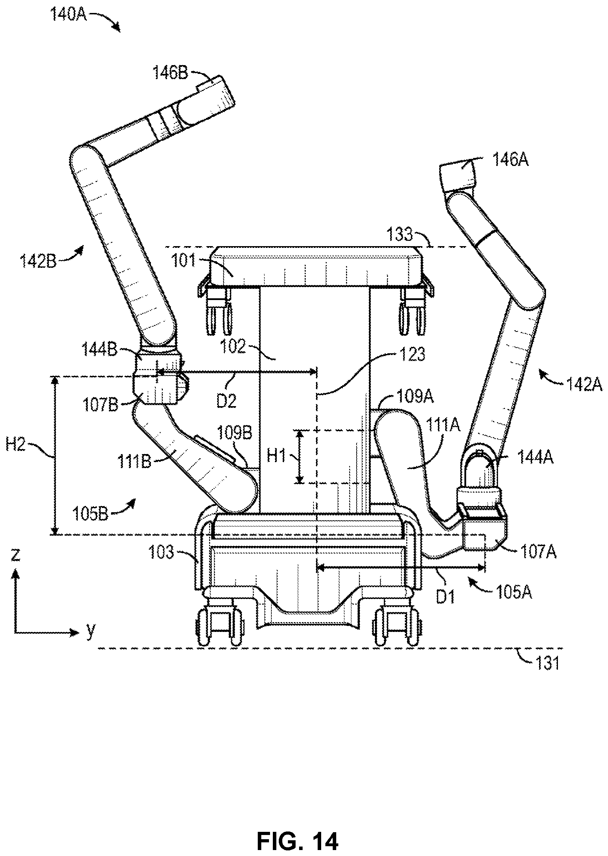

FIGS. 12 and 13 illustrate isometric and end views of an alternative embodiment of a table-based surgical robotics system 100. The surgical robotics system 100 includes one or more adjustable arm supports 105 that can be configured to support one or more robotic arms (see, for example, FIG. 14) relative to a table 101. In the illustrated embodiment, a single adjustable arm support 105 is shown, though an additional arm support can be provided on an opposite side of the table 101. The adjustable arm support 105 can be configured so that it can move relative to the table 101 to adjust and/or vary the position of the adjustable arm support 105 and/or any robotic arms mounted thereto relative to the table 101. For example, the adjustable arm support 105 may be adjusted one or more degrees of freedom relative to the table 101. The adjustable arm support 105 provides high versatility to the system 100, including the ability to easily stow the one or more adjustable arm supports 105 and any robotics arms attached thereto beneath the table 101. The adjustable arm support 105 can be elevated from the stowed position to a position below an upper surface of the table 101. In other embodiments, the adjustable arm support 105 can be elevated from the stowed position to a position above an upper surface of the table 101.

The adjustable arm support 105 can provide several degrees of freedom, including lift, lateral translation, tilt, etc. In the illustrated embodiment of FIGS. 12 and 13, the arm support 105 is configured with four degrees of freedom, which are illustrated with arrows in FIG. 12. A first degree of freedom allows for adjustment of the adjustable arm support 105 in the z-direction ("Z-lift"). For example, the adjustable arm support 105 can include a carriage 109 configured to move up or down along or relative to a column 102 supporting the table 101. A second degree of freedom can allow the adjustable arm support 105 to tilt. For example, the adjustable arm support 105 can include a rotary joint, which can allow the adjustable arm support 105 to be aligned with the bed in a Trendelenburg position. A third degree of freedom can allow the adjustable arm support 105 to "pivot up," which can be used to adjust a distance between a side of the table 101 and the adjustable arm support 105. A fourth degree of freedom can permit translation of the adjustable arm support 105 along a longitudinal length of the table.

The surgical robotics system 100 in FIGS. 12 and 13 can comprise a table supported by a column 102 that is mounted to a base 103. The base 103 and the column 102 support the table 101 relative to a support surface. A floor axis 131 and a support axis 133 are shown in FIG. 13.

The adjustable arm support 105 can be mounted to the column 102. In other embodiments, the arm support 105 can be mounted to the table 101 or base 103. The adjustable arm support 105 can include a carriage 109, a bar or rail connector 111 and a bar or rail 107. In some embodiments, one or more robotic arms mounted to the rail 107 can translate and move relative to one another.

The carriage 109 can be attached to the column 102 by a first joint 113, which allows the carriage 109 to move relative to the column 102 (e.g., such as up and down a first or vertical axis 123). The first joint 113 can provide the first degree of freedom ("Z-lift") to the adjustable arm support 105. The adjustable arm support 105 can include a second joint 115, which provides the second degree of freedom (tilt) for the adjustable arm support 105. The adjustable arm support 105 can include a third joint 117, which can provide the third degree of freedom ("pivot up") for the adjustable arm support 105. An additional joint 119 (shown in FIG. 13) can be provided that mechanically constrains the third joint 117 to maintain an orientation of the rail 107 as the rail connector 111 is rotated about a third axis 127. The adjustable arm support 105 can include a fourth joint 121, which can provide a fourth degree of freedom (translation) for the adjustable arm support 105 along a fourth axis 129.

FIG. 14 illustrates an end view of the surgical robotics system 140A with two adjustable arm supports 105A, 105B mounted on opposite sides of a table 101. A first robotic arm 142A is attached to the bar or rail 107A of the first adjustable arm support 105B. The first robotic arm 142A includes a base 144A attached to the rail 107A. The distal end of the first robotic arm 142A includes an instrument drive mechanism 146A that can attach to one or more robotic medical instruments or tools. Similarly, the second robotic arm 142B includes a base 144B attached to the rail 107B. The distal end of the second robotic arm 142B includes an instrument drive mechanism 146B. The instrument drive mechanism 146B can be configured to attach to one or more robotic medical instruments or tools.

In some embodiments, one or more of the robotic arms 142A, 142B comprises an arm with seven or more degrees of freedom. In some embodiments, one or more of the robotic arms 142A, 142B can include eight degrees of freedom, including an insertion axis (1-degree of freedom including insertion), a wrist (3-degrees of freedom including wrist pitch, yaw and roll), an elbow (1-degree of freedom including elbow pitch), a shoulder (2-degrees of freedom including shoulder pitch and yaw), and base 144A, 144B (1-degree of freedom including translation). In some embodiments, the insertion degree of freedom can be provided by the robotic arm 142A, 142B, while in other embodiments, the instrument itself provides insertion via an instrument-based insertion architecture.

### C. Instrument Driver & Interface.

The end effectors of the system's robotic arms comprise (i) an instrument driver (alternatively referred to as "instrument drive mechanism" or "instrument device manipulator") that incorporate electro-mechanical means for actuating the medical instrument and (ii) a removable or detachable medical instrument, which may be devoid of any electro-mechanical components, such as motors. This dichotomy may be driven by the need to sterilize medical instruments used in medical procedures, and the inability to adequately sterilize expensive capital equipment due to their intricate mechanical assemblies and sensitive electronics. Accordingly, the medical instruments may be designed to be detached, removed, and interchanged from the instrument driver (and thus the system) for individual sterilization or disposal by the physician or the physician's staff. In contrast, the instrument drivers need not be changed or sterilized, and may be draped for protection.

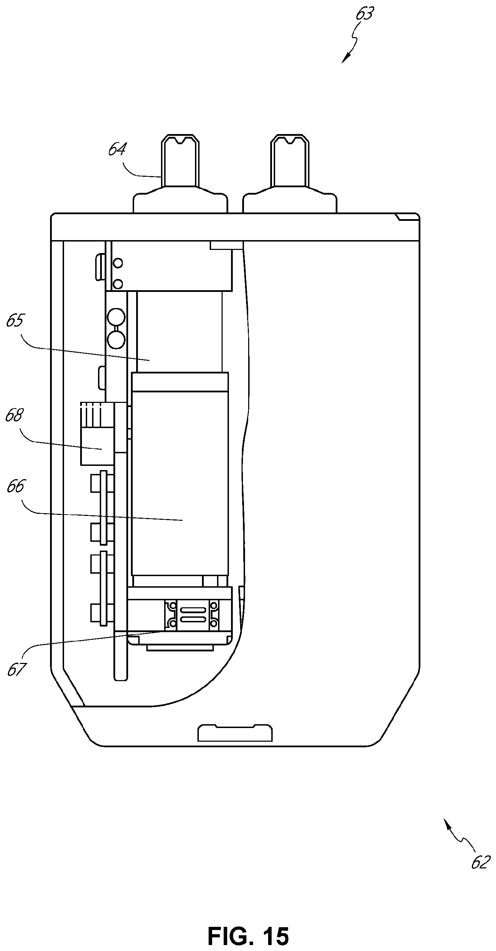

FIG. 15 illustrates an example instrument driver. Positioned at the distal end of a robotic arm, instrument driver 62 comprises of one or more drive units 63 arranged with parallel axes to provide controlled torque to a medical instrument via drive shafts 64. Each drive unit 63 comprises an individual drive shaft 64 for interacting with the instrument, a gear head 65 for converting the motor shaft rotation to a desired torque, a motor 66 for generating the drive torque, an encoder 67 to measure the speed of the motor shaft and provide feedback to the control circuitry, and control circuity 68 for receiving control signals and actuating the drive unit. Each drive unit 63 being independent controlled and motorized, the instrument driver 62 may provide multiple (four as shown in FIG. 15) independent drive outputs to the medical instrument. In operation, the control circuitry 68 would receive a control signal, transmit a motor signal to the motor 66, compare the resulting motor speed as measured by the encoder 67 with the desired speed, and modulate the motor signal to generate the desired torque.

For procedures that require a sterile environment, the robotic system may incorporate a drive interface, such as a sterile adapter connected to a sterile drape, that sits between the instrument driver and the medical instrument. The chief purpose of the sterile adapter is to transfer angular motion from the drive shafts of the instrument driver to the drive inputs of the instrument while maintaining physical separation, and thus sterility, between the drive shafts and drive inputs. Accordingly, an example sterile adapter may comprise of a series of rotational inputs and outputs intended to be mated with the drive shafts of the instrument driver and drive inputs on the instrument. Connected to the sterile adapter, the sterile drape, comprised of a thin, flexible material such as transparent or translucent plastic, is designed to cover the capital equipment, such as the instrument driver, robotic arm, and cart (in a cart-based system) or table (in a table-based system). Use of the drape would allow the capital equipment to be positioned proximate to the patient while still being located in an area not requiring sterilization (i.e., non-sterile field). On the other side of the sterile drape, the medical instrument may interface with the patient in an area requiring sterilization (i.e., sterile field).

### D. Medical Instrument.

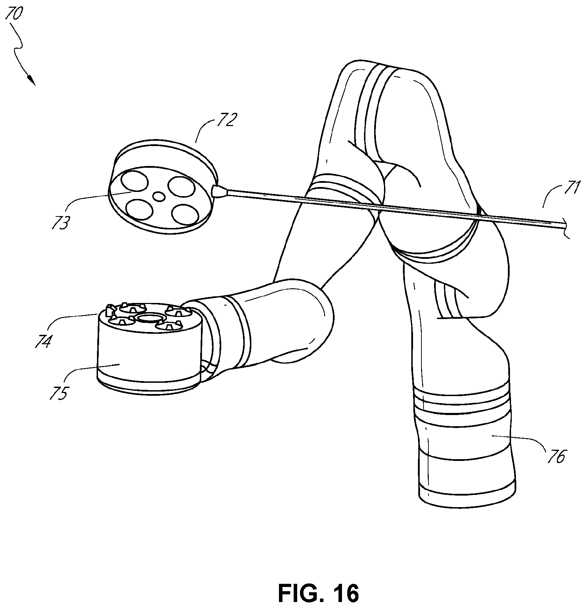

FIG. 16 illustrates an example medical instrument with a paired instrument driver. Like other instruments designed for use with a robotic system, medical instrument 70 comprises an elongated shaft 71 (or elongate body) and an instrument base 72. The instrument base 72, also referred to as an "instrument handle" due to its intended design for manual interaction by the physician, may generally comprise rotatable drive inputs 73, e.g., receptacles, pulleys or spools, that are designed to be mated with drive outputs 74 that extend through a drive interface on instrument driver 75 at the distal end of robotic arm 76. When physically connected, latched, and/or coupled, the mated drive inputs 73 of instrument base 72 may share axes of rotation with the drive outputs 74 in the instrument driver 75 to allow the transfer of torque from drive outputs 74 to drive inputs 73. In some embodiments, the drive outputs 74 may comprise splines that are designed to mate with receptacles on the drive inputs 73.

The elongated shaft 71 is designed to be delivered through either an anatomical opening or lumen, e.g., as in endoscopy, or a minimally invasive incision, e.g., as in laparoscopy. The elongated shaft 71 may be either flexible (e.g., having properties similar to an endoscope) or rigid (e.g., having properties similar to a laparoscope) or contain a customized combination of both flexible and rigid portions. When designed for laparoscopy, the distal end of a rigid elongated shaft may be connected to an end effector extending from a jointed wrist formed from a clevis with at least one degree of freedom and a surgical tool or medical instrument, such as, for example, a grasper or scissors, that may be actuated based on force from the tendons as the drive inputs rotate in response to torque received from the drive outputs 74 of the instrument driver 75. When designed for endoscopy, the distal end of a flexible elongated shaft may include a steerable or controllable bending section that may be articulated and bent based on torque received from the drive outputs 74 of the instrument driver 75.

Torque from the instrument driver 75 is transmitted down the elongated shaft 71 using tendons along the shaft 71. These individual tendons, such as pull wires, may be individually anchored to individual drive inputs 73 within the instrument handle 72. From the handle 72, the tendons are directed down one or more pull lumens along the elongated shaft 71 and anchored at the distal portion of the elongated shaft 71, or in the wrist at the distal portion of the elongated shaft. During a surgical procedure, such as a laparoscopic, endoscopic or hybrid procedure, these tendons may be coupled to a distally mounted end effector, such as a wrist, grasper, or scissor. Under such an arrangement, torque exerted on drive inputs 73 would transfer tension to the tendon, thereby causing the end effector to actuate in some way. In some embodiments, during a surgical procedure, the tendon may cause a joint to rotate about an axis, thereby causing the end effector to move in one direction or another. Alternatively, the tendon may be connected to one or more jaws of a grasper at distal end of the elongated shaft 71, where tension from the tendon cause the grasper to close.

In endoscopy, the tendons may be coupled to a bending or articulating section positioned along the elongated shaft 71 (e.g., at the distal end) via adhesive, control ring, or other mechanical fixation. When fixedly attached to the distal end of a bending section, torque exerted on drive inputs 73 would be transmitted down the tendons, causing the softer, bending section (sometimes referred to as the articulable section or region) to bend or articulate. Along the non-bending sections, it may be advantageous to spiral or helix the individual pull lumens that direct the individual tendons along (or inside) the walls of the endoscope shaft to balance the radial forces that result from tension in the pull wires. The angle of the spiraling and/or spacing there between may be altered or engineered for specific purposes, wherein tighter spiraling exhibits lesser shaft compression under load forces, while lower amounts of spiraling results in greater shaft compression under load forces, but also exhibits limits bending. On the other end of the spectrum, the pull lumens may be directed parallel to the longitudinal axis of the elongated shaft 71 to allow for controlled articulation in the desired bending or articulable sections.

In endoscopy, the elongated shaft 71 houses a number of components to assist with the robotic procedure. The shaft may comprise of a working channel for deploying surgical tools (or medical instruments), irrigation, and/or aspiration to the operative region at the distal end of the shaft 71. The shaft 71 may also accommodate wires and/or optical fibers to transfer signals to/from an optical assembly at the distal tip, which may include of an optical camera. The shaft 71 may also accommodate optical fibers to carry light from proximallylocated light sources, such as light emitting diodes, to the distal end of the shaft.

At the distal end of the instrument 70, the distal tip may also comprise the opening of a working channel for delivering tools for diagnostic and/or therapy, irrigation, and aspiration to an operative site. The distal tip may also include a port for a camera, such as a fiberscope or a digital camera, to capture images of an internal anatomical space. Relatedly, the distal tip may also include ports for light sources for illuminating the anatomical space when using the camera.

In the example of FIG. 16, the drive shaft axes, and thus the drive input axes, are orthogonal to the axis of the elongated shaft. This arrangement, however, complicates roll capabilities for the elongated shaft 71. Rolling the elongated shaft 71 along its axis while keeping the drive inputs 73 static results in undesirable tangling of the tendons as they extend off the drive inputs 73 and enter pull lumens within the elongated shaft 71. The resulting entanglement of such tendons may disrupt any control algorithms intended to predict movement of the flexible elongated shaft during an endoscopic procedure.

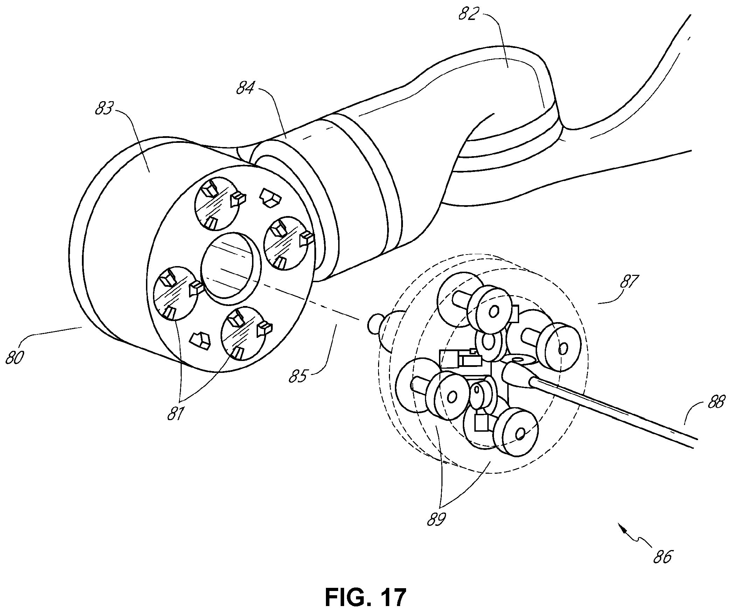

FIG. 17 illustrates an alternative design for an instrument driver and instrument where the axes of the drive units are parallel to the axis of the elongated shaft of the instrument. As shown, a circular instrument driver 80 comprises four drive units with their drive outputs 81 aligned in parallel at the end of a robotic arm 82. The drive units, and their respective drive outputs 81, are housed in a rotational assembly 83 of the instrument driver 80 that is driven by one of the drive units within the assembly 83. In response to torque provided by the rotational drive unit, the rotational assembly 83 rotates along a circular bearing that connects the rotational assembly 83 to the non-rotational portion 84 of the instrument driver. Power and controls signals may be communicated from the non-rotational portion 84 of the instrument driver 80 to the rotational assembly 83 through electrical contacts may be maintained through rotation by a brushed slip ring connection (not shown). In other embodiments, the rotational assembly 83 may be responsive to a separate drive unit that is integrated into the non-rotatable portion 84, and thus not in parallel to the other drive units. The rotational mechanism 83 allows the instrument driver 80 to rotate the drive units, and their respective drive outputs 81, as a single unit around an instrument driver axis 85.

Like earlier disclosed embodiments, an instrument 86 may comprise an elongated shaft portion 88 and an instrument base 87 (shown with a transparent external skin for discussion purposes) comprising a plurality of drive inputs 89 (such as receptacles, pulleys, and spools) that are configured to receive the drive outputs 81 in the instrument driver 80. Unlike prior disclosed embodiments, instrument shaft 88 extends from the center of instrument base 87 with an axis substantially parallel to the axes of the drive inputs 89, rather than orthogonal as in the design of FIG. 16.

When coupled to the rotational assembly 83 of the instrument driver 80, the medical instrument 86, comprising instrument base 87 and instrument shaft 88, rotates in combination with the rotational assembly 83 about the instrument driver axis 85. Since the instrument shaft 88 is positioned at the center of instrument base 87, the instrument shaft 88 is coaxial with instrument driver axis 85 when attached. Thus, rotation of the rotational assembly 83 causes the instrument shaft 88 to rotate about its own longitudinal axis. Moreover, as the instrument base 87 rotates with the instrument shaft 88, any tendons connected to the drive inputs 89 in the instrument base 87 are not tangled during rotation. Accordingly, the parallelism of the axes of the drive outputs 81, drive inputs 89, and instrument shaft 88 allows for the shaft rotation without tangling any control tendons.

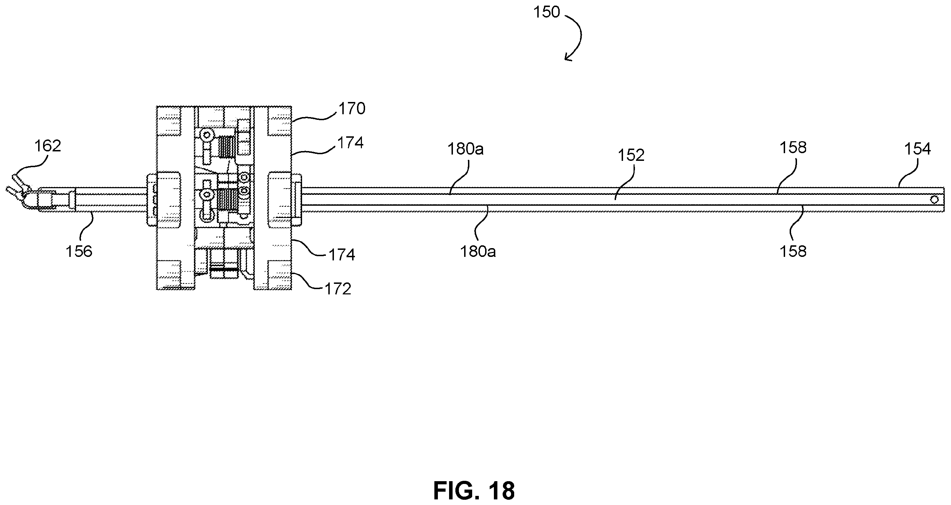

FIG. 18 illustrates an instrument having an instrument based insertion architecture in accordance with some embodiments. The instrument 150 can be coupled to any of the instrument drivers discussed above. The instrument 150 comprises an elongated shaft 152, an end effector 162 connected to the shaft 152, and a handle 170 coupled to the shaft 152. The elongated shaft 152 comprises a tubular member having a proximal portion 154 and a distal portion 156. The elongated shaft 152 comprises one or more channels or grooves 158 along its outer surface. The grooves 158 are configured to receive one or more wires or cables 180 therethrough. One or more cables 180 thus run along an outer surface of the elongated shaft 152. In other embodiments, cables 180 can also run through the elongated shaft 152. Manipulation of the one or more cables 180 (e.g., via an instrument driver) results in actuation of the end effector 162.

The instrument handle 170, which may also be referred to as an instrument base, may generally comprise an attachment interface 172 having one or more mechanical inputs 174, e.g., receptacles, pulleys or spools, that are designed to be reciprocally mated with one or more torque couplers on an attachment surface of an instrument driver.

In some embodiments, the instrument 150 comprises a series of pulleys or cables that enable the elongated shaft 152 to translate relative to the handle 170. In other words, the instrument 150 itself comprises an instrument-based insertion architecture that accommodates insertion of the instrument, thereby minimizing the reliance on a robot arm to provide insertion of the instrument 150. In other embodiments, a robotic arm can be largely responsible for instrument insertion.

### E. Controller.

Any of the robotic systems described herein can include an input device or controller for manipulating an instrument attached to a robotic arm. In some embodiments, the controller can be coupled (e.g., communicatively, electronically, electrically, wirelessly and/or mechanically) with an instrument such that manipulation of the controller causes a corresponding manipulation of the instrument e.g., via master slave control.

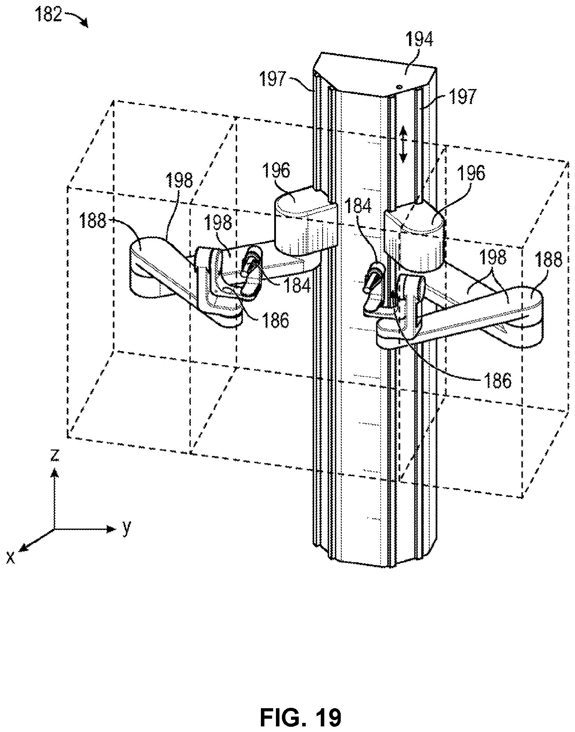

FIG. 19 is a perspective view of an embodiment of a controller 182. In the present embodiment, the controller 182 comprises a hybrid controller that can have both impedance and admittance control. In other embodiments, the controller 182 can utilize just impedance or passive control. In other embodiments, the controller 182 can utilize just admittance control. By being a hybrid controller, the controller 182 advantageously can have a lower perceived inertia while in use.

In the illustrated embodiment, the controller 182 is configured to allow manipulation of two medical instruments, and includes two handles 184. Each of the handles 184 is connected to a gimbal 186. Each gimbal 186 is connected to a positioning platform 188.

As shown in FIG. 19, each positioning platform 188 includes a SCARA arm (selective compliance assembly robot arm) 198 coupled to a column 194 by a prismatic joint 196. The prismatic joints 196 are configured to translate along the column 194 (e.g., along rails 197) to allow each of the handles 184 to be translated in the z-direction, providing a first degree of freedom. The SCARA arm 198 is configured to allow motion of the handle 184 in an x-y plane, providing two additional degrees of freedom.

In some embodiments, one or more load cells are positioned in the controller. For example, in some embodiments, a load cell (not shown) is positioned in the body of each of the gimbals 186. By providing a load cell, portions of the controller 182 are capable of operating under admittance control, thereby advantageously reducing the perceived inertia of the controller while in use. In some embodiments, the positioning platform 188 is configured for admittance control, while the gimbal 186 is configured for impedance control. In other embodiments, the gimbal 186 is configured for admittance control, while the positioning platform 188 is configured for impedance control. Accordingly, for some embodiments, the translational or positional degrees of freedom of the positioning platform 188 can rely on admittance control, while the rotational degrees of freedom of the gimbal 186 rely on impedance control.

### F. Navigation and Control.

Traditional endoscopy may involve the use of fluoroscopy (e.g., as may be delivered through a C-arm) and other forms of radiation-based imaging modalities to provide endoluminal guidance to an operator physician. In contrast, the robotic systems contemplated by this disclosure can provide for non-radiation-based navigational and localization means to reduce physician exposure to radiation and reduce the amount of equipment within the operating room. As used herein, the term "localization" may refer to determining and/or monitoring the position of objects in a reference coordinate system. Technologies such as preoperative mapping, computer vision, real-time EM tracking, and robot command data may be used individually or in combination to achieve a radiation-free operating environment. In other cases, where radiation-based imaging modalities are still used, the pre-operative mapping, computer vision, real-time EM tracking, and robot command data may be used individually or in combination to improve upon the information obtained solely through radiation-based imaging modalities.

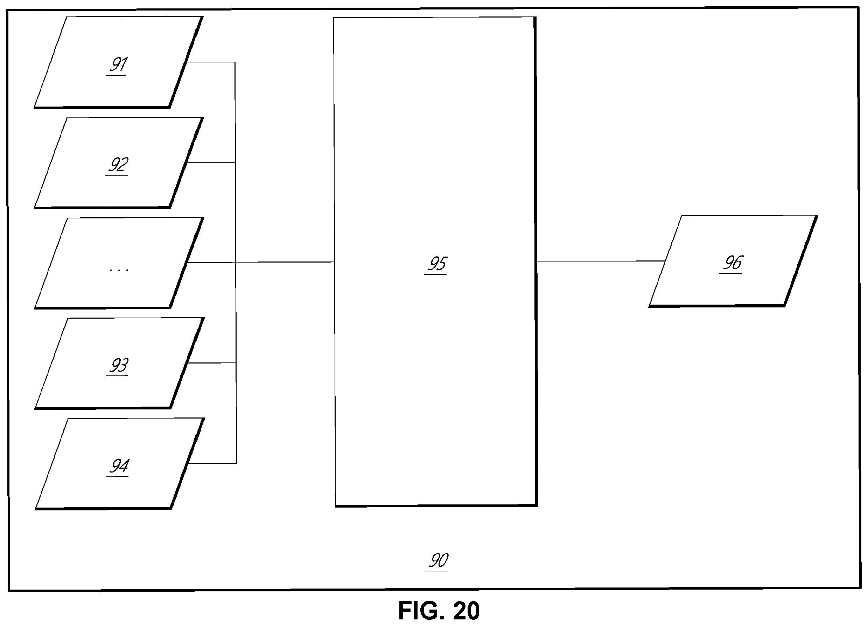

FIG. 20 is a block diagram illustrating a localization system 90 that estimates a location of one or more elements of the robotic system, such as the location of the instrument, in accordance to an example embodiment. The localization system 90 may be a set of one or more computer devices configured to execute one or more instructions. The computer devices may be embodied by a processor (or processors) and computer-readable memory in one or more components discussed above. By way of example and not limitation, the computer devices may be in the tower 30 shown in FIG. 1, the cart shown in FIGS. 1-4, the beds shown in FIGS. 5-14, etc.

As shown in FIG. 20, the localization system 90 may include a localization module 95 that processes input data 91-94 to generate location data 96 for the distal tip of a medical instrument. The location data 96 may be data or logic that represents a location and/or orientation of the distal end of the instrument relative to a frame of reference. The frame of reference can be a frame of reference relative to the anatomy of the patient or to a known object, such as an EM field generator (see discussion below for the EM field generator).

The various input data 91-94 are now described in greater detail. Preoperative mapping may be accomplished through the use of the collection of low dose CT scans. Pre-operative CT scans are reconstructed into three-dimensional images, which are visualized, e.g. as "slices" of a cutaway view of the patient's internal anatomy. When analyzed in the aggregate, image-based models for anatomical cavities, spaces and structures of the patient's anatomy, such as a patient lung network, may be generated. Techniques such as center-line geometry may be determined and approximated from the CT images to develop a three-dimensional volume of the patient's anatomy, referred to as model data 91 (also referred to as "preoperative model data" when generated using only preoperative CT scans). The use of center-line geometry is discussed in U.S. Pat. App. No. 14/523,760. Network topological models may also be derived from the CT-images, and are particularly appropriate for bronchoscopy.

In some embodiments, the instrument may be equipped with a camera to provide vision data 92. The localization module 95 may process the vision data to enable one or more vision-based location tracking. For example, the preoperative model data may be used in conjunction with the vision data 92 to enable computer vision-based tracking of the medical instrument (e.g., an endoscope or an instrument advance through a working channel of the endoscope). For example, using the preoperative model data 91, the robotic system may generate a library of expected endoscopic images from the model based on the expected path of travel of the endoscope, each image linked to a location within the model. Intra-operatively, this library may be referenced by the robotic system in order to compare real-time images captured at the camera (e.g., a camera at a distal end of the endoscope) to those in the image library to assist localization.

Other computer vision-based tracking techniques use feature tracking to determine motion of the camera, and thus the endoscope. Some features of the localization module 95 may identify circular geometries in the preoperative model data 91 that correspond to anatomical lumens and track the change of those geometries to determine which anatomical lumen was selected, as well as the relative rotational and/or translational motion of the camera. Use of a topological map may further enhance vision-based algorithms or techniques.

Optical flow, another computer vision-based technique, may analyze the displacement and translation of image pixels in a video sequence in the vision data 92 to infer camera movement. Examples of optical flow techniques may include motion detection, object segmentation calculations, luminance, motion compensated encoding, stereo disparity measurement, etc. Through the comparison of multiple frames over multiple iterations, movement and location of the camera (and thus the endoscope) may be determined.

The localization module 95 may use real-time EM tracking to generate a real-time location of the endoscope in a global coordinate system that may be registered to the patient's anatomy, represented by the preoperative model. In EM tracking, an EM sensor (or tracker) comprising of one or more sensor coils embedded in one or more locations and orientations in a medical instrument (e.g., an endoscopic tool) measures the variation in the EM field created by one or more static EM field generators positioned at a known location. The location information detected by the EM sensors is stored as EM data 93. The EM field generator (or transmitter), may be placed close to the patient to create a low intensity magnetic field that the embedded sensor may detect. The magnetic field induces small currents in the sensor coils of the EM sensor, which may be analyzed to determine the distance and angle between the EM sensor and the EM field generator. These distances and orientations may be intra-operatively "registered" to the patient anatomy (e.g., the preoperative model) in order to determine the geometric transformation that aligns a single location in the coordinate system with a position in the pre-operative model of the patient's anatomy. Once registered, an embedded EM tracker in one or more positions of the medical instrument (e.g., the distal tip of an endoscope) may provide real-time indications of the progression of the medical instrument through the patient's anatomy.

Robotic command and kinematics data 94 may also be used by the localization module 95 to provide localization data 96 for the robotic system. Device pitch and yaw resulting from articulation commands may be determined during pre-operative calibration. Intra-operatively, these calibration measurements may be used in combination with known insertion depth information to estimate the position of the instrument. Alternatively, these calculations may be analyzed in combination with EM, vision, and/or topological modeling to estimate the position of the medical instrument within the network.

As FIG. 20 shows, a number of other input data can be used by the localization module 95. For example, although not shown in FIG. 20, an instrument utilizing shape-sensing fiber can provide shape data that the localization module 95 can use to determine the location and shape of the instrument.

The localization module 95 may use the input data 91-94 in combination(s). In some cases, such a combination may use a probabilistic approach where the localization module 95 assigns a confidence weight to the location determined from each of the input data 91-94. Thus, where the EM data may not be reliable (as may be the case where there is EM interference) the confidence of the location determined by the EM data 93 can be decrease and the localization module 95 may rely more heavily on the vision data 92 and/or the robotic command and kinematics data 94.

As discussed above, the robotic systems discussed herein may be designed to incorporate a combination of one or more of the technologies above. The robotic system's computer-based control system, based in the tower, bed and/or cart, may store computer program instructions, for example, within a non-transitory computer-readable storage medium such as a persistent magnetic storage drive, solid state drive, or the like, that, upon execution, cause the system to receive and analyze sensor data and user commands, generate control signals throughout the system, and display the navigational and localization data, such as the position of the instrument within the global coordinate system, anatomical map, etc.

### 2. Surgical Bed Pendant for Mobile Medical Platforms.

As shown in several of the examples described above, robotic medical systems can include a medical platform that includes a bed or table top. The medical platform can be configured to support a patient during a medical procedure, such as robotic endoscopy, robotic laparoscopy, open procedures, or others (see, for example, FIGS. 1, 3, 4, 5, 8, and 9, described above). In some cases, operation of the medical platform may be controlled by an input device, such as a pendant (e.g., pendant controller, bed pendant, surgical bed pendant, etc.). The input device provides a user interface via one or more of: joystick controller(s), touch screen display, and affordances (e.g., buttons), and other types of hardware controls, etc. that allow a user to operate the medical platform. In some embodiments, the input device is configured to receive user input to provide powered assisted mobility to the medical platform by steering, driving, and/or maneuvering (e.g., via powered steering and motorized propulsion of motorized wheels) the medical platform. In some embodiments, the input device is configured to receive user input to control orientation and/or positioning of the bed or table top of the medical platform relative to the base of the medical platform. In some embodiments, the input device is configured to receive user input to place the medical platform in a specific mode, such as stowing robotic arms for mobilizing the medical platform, or other operational modes during set-up of or transition between procedures.

Disclosed herein are mobile medical platforms that include a user input device, such as a controller, console, or pendant, to provide user control of at least some operations of the mobile medical platform. The input device allows a user to control the medical platform in different modes of operation, such as in an assisted mobility mode for providing power-assisted steering and propulsion to move the medical platform and in a table top control mode for positioning and orienting the table top for surgical procedures.

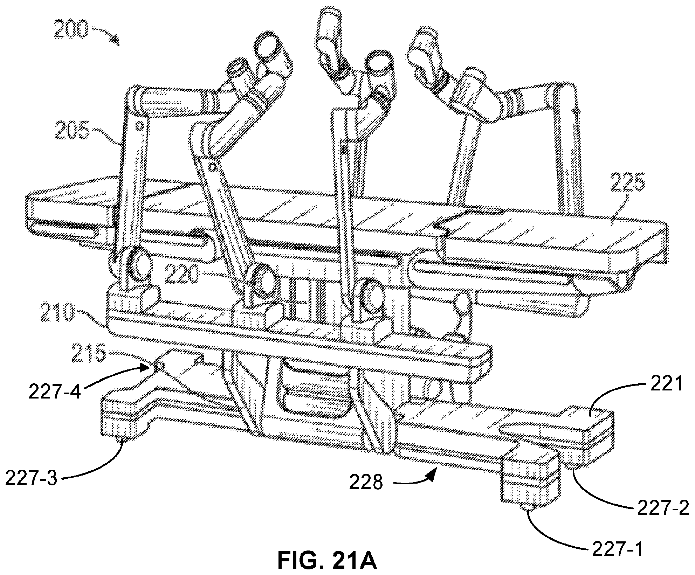

FIG. 21A illustrates an embodiment of a mobile medical platform 200 that includes one or more wheel assemblies 227 in accordance with some embodiments. The mobile medical platform 200 (e.g., medical platform, patient platform, robotic surgical platform) includes a table, which includes a table top 225 (e.g., surgical bed, surgical table, robotic surgical table, a platform, a support surface), a bed column 220 to support the table top 225, and a rigid base 221 (e.g., a table base for a surgical bed, a rigid load-bearing housing, a chassis) that supports the table top 225 and the bed column 220. The rigid base 221 is rigidly coupled or movably coupled to the table top 225 and is configured to support the table top 225 (e.g., with a bed column 220). The table top 225 is configured to support a patient and serve as a hospital bed or a surgical bed. The table top 225 is manually, teleoperatively, and/or automatically movable (e.g., capable of tilting, panning, rotating, and/or translating) relative to the rigid base 221. The mobile medical platform 200 also includes one or more mechanisms for adjusting a position and orientation of the table top 225, such as raising or lowering the table top 225, or tilting the table top 225, for example.

The mobile medical platform 200 also includes one or more powered wheel assemblies 227 (e.g., 227-1 through 227-4) coupled (e.g., rigidly coupled) to a first side 228 (e.g., a bottom side). The one or more powered wheel assemblies 227 are configured to provide power-assisted movement and transportation of the entire mobile medical platform 200, and to support and move the rigid base 221 in a physical environment. A respective wheel assembly of the one or more wheel assemblies 227 includes at least one motorized wheel (e.g., a wheel that is steered by a first motor, rolled by a second motor, or both). Additional details regarding mechanisms associated with functions and operations of the powered wheel assemblies 227 are described in U.S. Provisional Application Serial No. 63/086,043, filed September 30, 2020.

In some embodiments, the mobile medical platform 200 also includes one or more robotic arms 205 (e.g., a single robotic arm, two or more robotic arms, a plurality of robotic arms), one or more adjustable arm supports 210, and/or one or more set-up joints 215. A respective robotic arm of the robotic arms 205 may be supported by one of the adjustable arm supports 210 and the adjustable arm support(s) 210 may be in turn be supported by the setup joint(s) 215. A respective robotic arm of the robotic arms 205 may be coupled (e.g., mechanically coupled, movably coupled, etc.) to the table (e.g., via the rigid base 221 and bed column 220, etc.), to the rigid base 221 (e.g., via a moveable arm support), or to the table top 225. In some embodiments, the robotic arms 205 are manually, teleoperatively, and/or automatically movable (e.g., capable of tilting, panning, rotating, bending, and/or translating) relative to the rigid base 221, the arm support 210, and/or the table top 225.

In some embodiments, the mobile medical platform 200 includes medical equipment such as monitoring or imaging equipment attached to the one or more robotic arms 205. The mobile medical platform 200 may also include an onboard battery for wireless operation of the mobile medical platform 200 and/or an onboard power supply that can be plugged into an electrical socket to provide electricity for operation of the mobile medical platform 200. The one or more power wheel assemblies 227 are configured to provide power-assisted mobility to the mobile medical platform 200 and any equipment or persons that are support by, mounted on, coupled to, and/or on onboard, the mobile medical platform 200.

The mobile medical platform 200 also includes an input device (e.g., controller, pendant, bed pendant, portable device, handheld input device, etc.) that is configured to receive user inputs for controlling operations and functions of the mobile medical platform 200. For example, a user may provide user inputs at the input device for controlling a motion of the mobile medical platform 200 during power-assisted transportation of the mobile medical platform 200 (e.g., driving the mobile medical platform 200). In another example, the user may provide user inputs at the input device for adjusting a position of the table top 225 of the mobile medical platform 200.

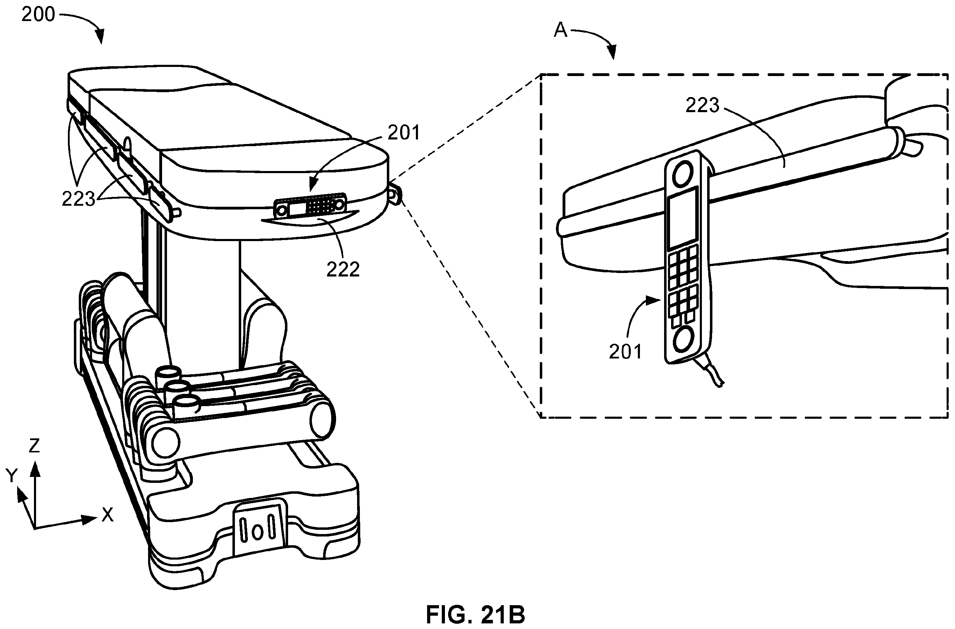

FIG. 21B illustrates an embodiment of a mobile medical platform 200 that includes an input device 201 in accordance with some embodiments. The input device 201 is in communication with the mobile medical platform 200 (e.g., the mobile medical platform system). In some embodiments, the input device 201 is electrically coupled (e.g., connected, physically coupled to) to the mobile medical platform 200, such as via one or more wired connections. In some embodiments, the input device 201 is wirelessly connected to the mobile medical platform 200 via wireless communication methods and technology, such as over a wireless communication network, or a near-range peer-to-peer connection, etc. In some embodiments, the input device 201 is able to communicate with the mobile medical platform 200 within a predefined range of operation (e.g., the mobile medical platform 200 and the input device 201 are within 5 feet, 10 feet, 20 feet, 50 feet, etc. of one another). For example, the input device 201 may be tethered to the mobile medical platform 200 by a wire or connected to the mobile medical platform 200 via wireless communication methods. In some embodiments, the amount of separation between the input device 201 and the table top 225 is such that, while a person is within a preset range of (e.g., remaining stationary relative to, or moving within the preset range, etc.) the mobile medical platform 200 that is stationary or moving as a whole in a physical environment, the person will be able to reach and control the input device 201 when the input device 201 is mounted on or connected to a portion of the mobile medical platform 200.

In some embodiments, the mobile medical platform 200 includes one or more bars 223 disposed around a periphery of the mobile medical platform 200, such as around a periphery of the table top 225. The bar 223 is configured to stow (e.g., house, stow, store) the input device 201. In some embodiments, the mobile medical platform 200 includes a single bar 223 that surrounds a periphery of the mobile medical platform 200. In some embodiments, the mobile medical platform 200 includes a plurality of bars 223 that are disposed around the periphery of the mobile medical platform 200.

In some embodiments, the mobile medical platform 200 also includes a mount 222 disposed at a head end and/or a foot end of the mobile medical platform 200. In some embodiments, a user is able to provide user inputs at the input device 201 to control power-assisted mobility of the mobile medical platform 200 (e.g., provide user inputs to drive the mobile medical platform 200) while the input device 201 is mounted or stored on the mount 222. For example, a user may be able to provide one or more inputs at the input device 201 to steer the mobile medical platform 200 while pushing the mobile medical platform 200. In some embodiments, an operator of the mobile medical platform 200 may push the mobile medical platform 200 or control movement of the mobile medical platform 200 while walking alongside the mobile medical platform 200 while the mobile medical platform 200 is, for example, used to transport a patient.

In some embodiments, the input device 201 is configurable to be magnetically coupled (e.g., magnetically and removably mounted) onto the bar 223 and/or the mount 222 of the mobile medical platform 200.

The input device 201 can be stored (e.g., mounted, hooked, attached, etc.) on the bar 223 and the mount 222 in either a horizontal (e.g., landscape) orientation, or in a vertical (e.g., portrait) orientation, as shown in inset A. In some embodiments, the orientation of the input device 201 is locked when mounted. In some embodiments, the mount 222 is configured to allow the input device 201 to have any of a range or set of orientations and angles relative to the mount 222.

The input device 201 is configured to advantageously operate in at least two modes, includinga mobility mode (also referred to herein as bed control mode) and a table top mode (also referred to herein as bed status mode). The input device 201 is configured to operate in a horizontal format while in the mobility mode, and to operate in vertical format while in the table top mode. In some embodiments, the operation in the vertical format is associated with a first range of angles and orientations (e.g., up to 60 degrees tilted backward, up to 30 degrees tilted left or right, etc., relative to an axis in the direction of gravity) with the input device held in the portrait orientation. In some embodiments, the operation in the horizontal format is associated with a second range of angles and orientations (e.g., up to 90 degrees tilted backward, up to 60 degrees tilted left or right, etc., relative to an axis in the direction of gravity) with the input device held in the landscape orientation.

In some embodiments, the mode of operation of the input device 201 is independent of the physical orientation of the input device 201. For example, while in the mobility mode, the input device 201 continues to operate in the mobility mode with a horizontal format regardless of a physical orientation of the input device 201 in space. Similarly, while in the table top mode, the input device 201 continues to operate in the table top mode with a vertical format even if a user rotates a physical orientation of the input device 201 from a vertical physical orientation to a horizontal or diagonal physical orientation. In such cases, changing a physical orientation of the input device 201 does not change an operational mode or operational format of the input device 201. In some embodiments, locking the operation mode of the input device 201 reduces accidental switching of operation modes when the user is operating the input device 201 and moving the input device 201 in the physical environment.

In some embodiments, the input device 201 is configured to operate in either the mobility mode or the table top mode based on a physical orientation of the input device 201. In such cases, the input device 201 is configured to transition (e.g., automatically switching) between the mobility mode and the table top mode based on a physical orientation of the input device 201. For example, when a user changes a physical orientation of the input device 201 from a vertical physical orientation to a horizontal physical orientation, the input device 201 transitions from the mobility mode of operation to the table top mode of operation. In some embodiments, the vertical physical orientation is associated with a first range of angles and orientations (e.g., up to 60 degrees tilted backward, up to 30 degrees tilted left or right, etc., relative to an axis in the direction of gravity) with the input device held in the portrait orientation. In some embodiments, the horizontal physical orientation is associated with a second range of angles and orientations (e.g., up to 90 degrees tilted backward, up to 60 degrees tilted left or right, etc., relative to an axis in the direction of gravity) with the input device held in the landscape orientation. In some embodiments, the automatic switching require one or more confirmation inputs or conditions to be met, such as a time threshold that the input device is held in a respective orientation, or a confirmation input provided on a touch-screen of the input device, etc..

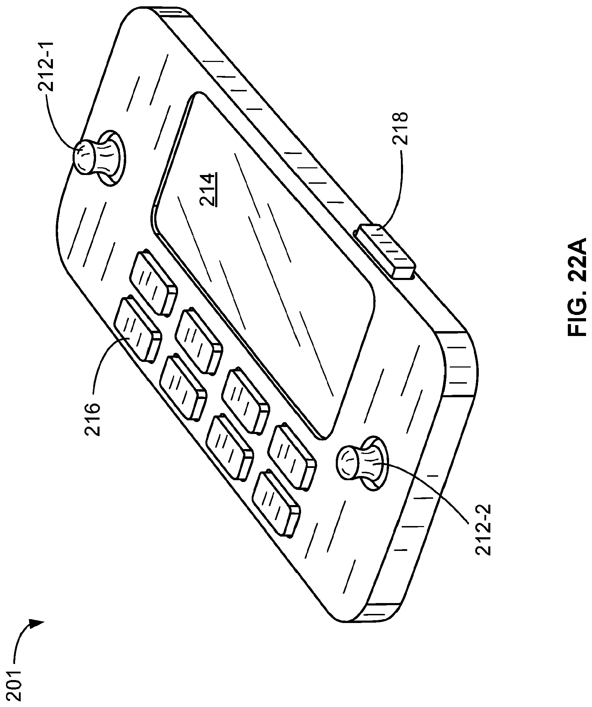

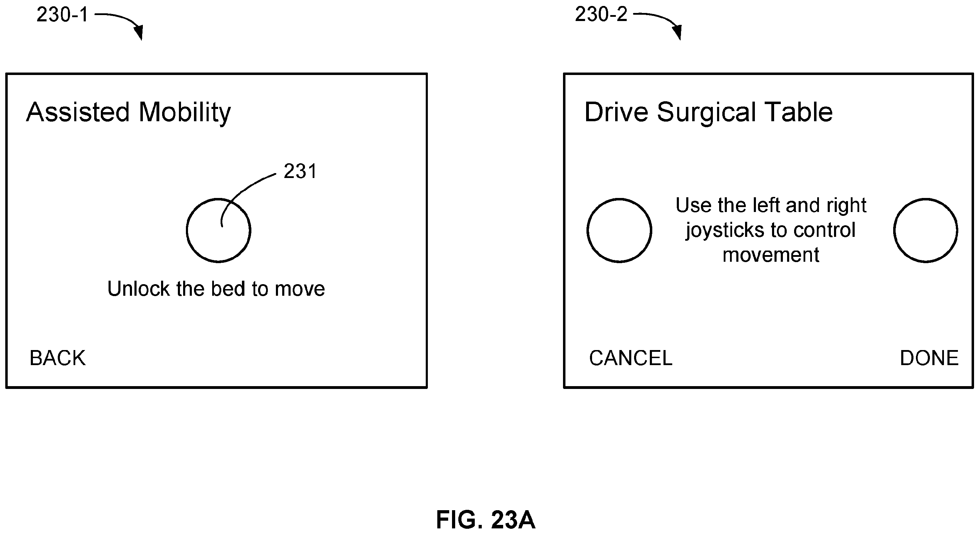

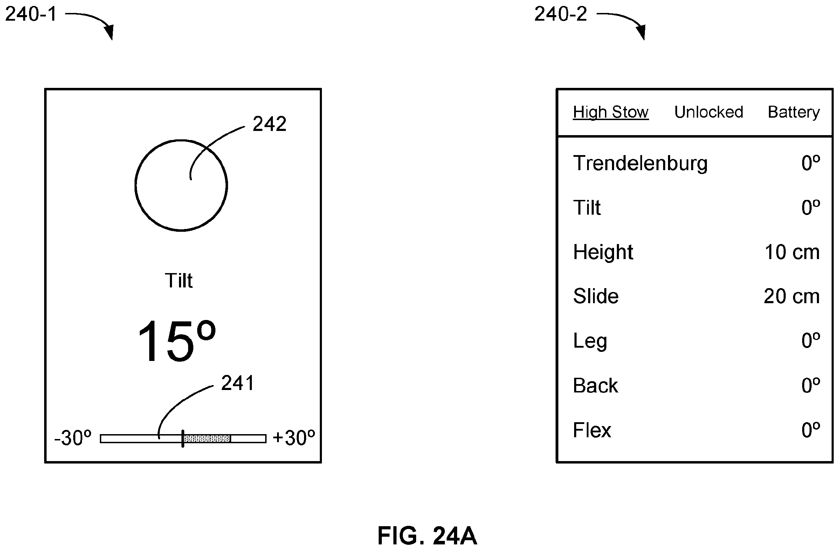

FIGs. 22A - 22B provide details regarding features of the input device 201, FIGs. 23A - 23D provide details regarding operation of the input device 201 in the mobility mode, and FIGs. 24A - 24F provide details regarding operation of the input device 201 in the table top mode, in accordance with various embodiments.

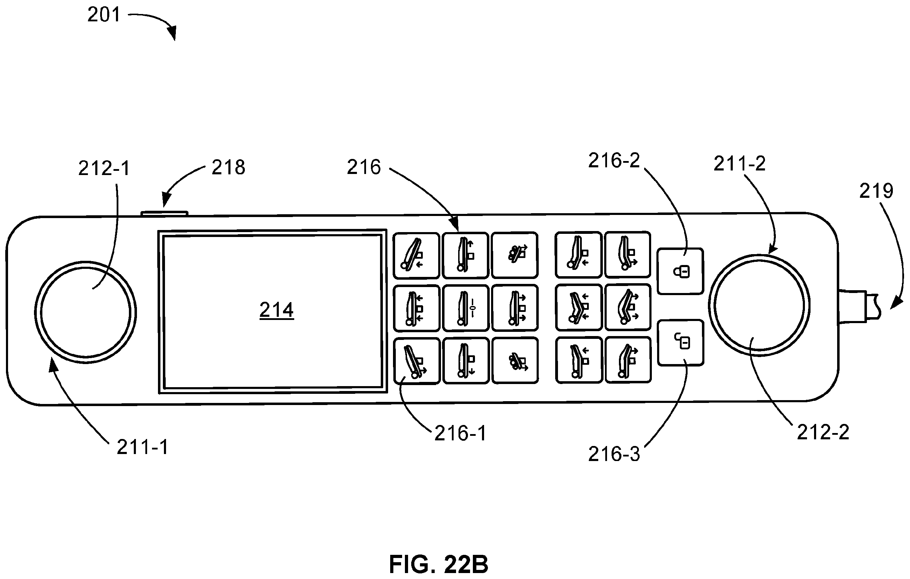

FIG. 22A - 22B illustrate embodiments of an input device 201 for controlling operation of the mobile medical platform 200 of FIGs. 21A and 21B in accordance with some embodiments. FIG. 22A illustrates perspective view of an example of input device 201, and FIG. 22B illustrates front view of another example of input device 201, in accordance with some embodiments. Input device 201 includes one or more joysticks 212 (such as joysticks 212-1 and 212-2), a display 214, one more affordances 216 (e.g., buttons, control affordances), and a motion control affordance 218 (e.g., motion enabled affordance), in accordance with some embodiments.

Joysticks 212 are a respective type of user interface that includes a user interface, a hardware controller, or a combination thereof, that detects physical inputs provided by a user's hand(s) (e.g., via one or more sensors or mechanical means) and translates one or more characteristics of the inputs (e.g., magnitudes, directions, type, etc. of forces, moments, and/or movements, etc.) to corresponding control parameters and instructions for moving a controlled object, in accordance with some embodiments. The one or more joysticks 212 are configured to receive user input, such as movement and/or displacement of the joystick 212 from a center location or a neutral position of the joystick 212, in accordance with some embodiments. In some embodiments, the input device 201 is configured to receive various combinations of user inputs at the first joystick 212-1 and the second joystick 212-2, including receiving concurrent inputs and receiving sequential inputs.

In some embodiments, the input device 201 is configured to receive user input at the joystick 212 while operating in one or both of the mobility mode and the table top mode. In some embodiments, function and execution of the user input at the joystick 212 is dependent on which mode the input device 201 is currently operating in while the user input is received. In some embodiments, inputs that are received at the joystick 212 (either joystick 212-1 or 212-2) while input device is operating in the mobility mode corresponds to mobility functions of the mobile medical platform 200, such as power-assisted movement of the mobile medical platform 200, and/or such as steering and propulsion. Inputs that are received at the joystick 212 (either joystick 212-1 or 212-2) while input device 201 is operating in the table top mode corresponds to table top positioning and table top orientation functions of the mobile medical platform 200, such as translating and rotating the table top 225 relative to the rigid base 221.

In some embodiments, while the input device 201 is in the mobility mode, inputs received at the joysticks 212 correspond to mobility functions of the mobile medical platform 200. In some embodiments, the direction and magnitude of the movement of the one or more wheel assemblies 227 are determined based on the direction (e.g., left, right, forward, backward, clockwise, counterclockwise, etc.) and/or magnitude (e.g., duration, displacement, etc.) the inputs received via the first joystick 212-1, based on the direction (e.g., left, right, forward, backward, clockwise, counterclockwise, etc.) and/or magnitude (e.g., duration, displacement, etc.) the inputs received via the second joystick 212-2, or based on both. In some embodiments, while in the mobility mode, a first joystick 212-1 is configured to control an orientation (e.g., direction, heading, alignment) of the mobile medical platform 200 by controlling an orientation (e.g., steering) of the wheels of the one or more wheel assemblies 227. Thus, when user inputs are received at the first joystick 212-1, wheels of the one or more wheel assemblies 227 are steered or oriented in accordance with the user inputs at the first joystick 212-1. For example, by providing an input via the first joystick 212-1, a user may cause the mobile medical platform 200 to rotate or pivot clockwise or counterclockwise with negligible lateral movement (e.g., without lateral translation of the mobile medical platform 200). In some embodiments, while in the mobility mode, a second joystick 212-2 is configured to control propulsion (e.g., motion) of the mobile medical platform 200 by controlling propulsion of the wheels of the one or more wheel assemblies 227. Thus, when user inputs are received at the second joystick 212-2, wheels of the one or more wheel assemblies 227 are propelled in accordance with the user inputs at the second joystick 212-2 (e.g., in accordance with a direction or magnitude of the user input received at the second joystick 212-2). For example, by providing an input via the second joystick 212-2, a user may cause the mobile medical platform 200 to move laterally in any direction (left, right, forwards, backwards, diagonally, etc.) without rotating the mobile medical platform 200. In some embodiments, one or more processors of the mobile medical platform 200 determines respective movements for each wheel of the one or more wheel assemblies 227 based on a desired movement of the base specified by the inputs detected by the first and second joysticks 212-1 and 212-2, and instructs the one or more wheel assemblies 227 to move in a coordinated manner to achieve the desired movement of the base. Thus, operations of each wheel assembly 227 of the mobile medical platform 200 are automatically controlled and coordinated to achieve a movement or action as requested by a user via the input device 201.

In some embodiments, while the input device 201 is in the table top mode, inputs received at the joysticks 212 correspond to table top functions of the mobile medical platform 200. For example, while in the table top mode, the first joystick 212-1 is configured to control a position (e.g., control translation, raise/lower and slide towards head/feet, etc.) of the table top 225, in accordance with some embodiments. Thus, when user inputs are received at the first joystick 212-1, the table top 225 may be raised and lowered (e.g., moved up and down along the z-direction) and/or moved towards the head end or feet end of the mobile medical platform 200 (e.g., slide along a y-direction). In another example, while in the table top mode, the second joystick 212-2 is configured to control an orientation of the table top 225 (e.g., rotate the table top 225) around a lateral axis (e.g., pitch, Trendelenburg angle, into a Trendelenburg position or reverse-Trendelenburg position) and a longitudinal axis (e.g., roll, left/right rotation). In some embodiments, a single joystick (e.g., joystick 212-2) may be configured to receive user inputs for both translating and rotating the table top 225, and function of inputs received at the joystick 212 may be toggled via affordance(s) displayed on the display 214 or by depressing the joystick 212, for example.

In some embodiments, functions described with respect to the first joystick 212-1 and the second joystick 212-2 may be reversed. For example, while in the mobility mode, the first joystick 212-1 may be configured to provide steering of the mobile medical platform 200 and the second joystick 212-2 may be configured to provide propulsion of the mobile medical platform 200, or vice versa. Similarly, while in the table top mode, the first joystick 212-1 may be configured to provide translation of the table top 225 and the second joystick 212-2 may be configured to provide rotation of the table top 225, or vice versa.

In some embodiments, the joysticks 212-1 and 212-2 may be implemented as physical joysticks, as shown in FIG. 22A, or as touch sensitive joystick surfaces (e.g., touch pads).

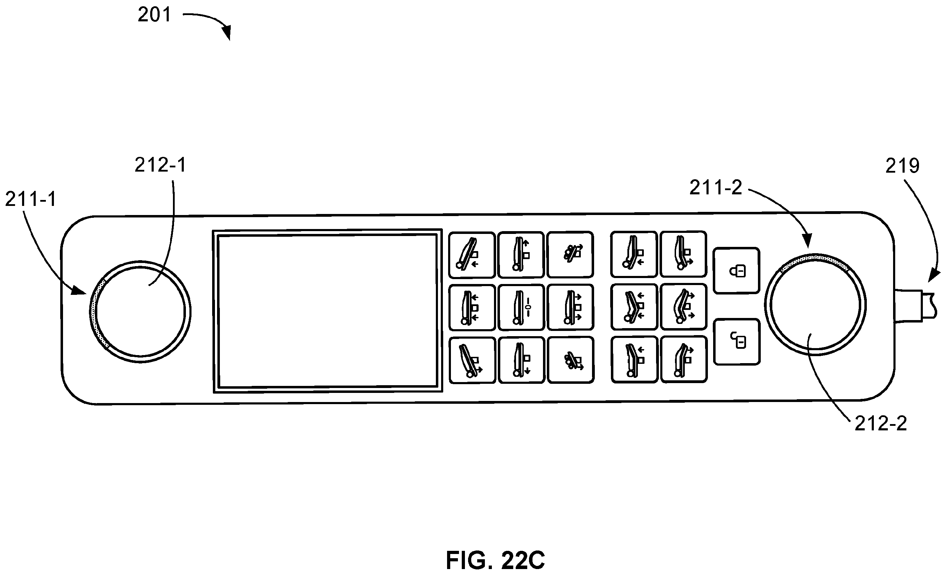

In some embodiments, as shown in FIG. 22B, an input device 201 can also include a first visual indicator 211-1 (e.g., light ring, one or more lights) configured to provide a visual feedback (e.g., visual indication) corresponding to inputs received at the first joystick 212-1, and a second visual indicator 211-2 (e.g., light ring) configured to provide a visual feedback corresponding to inputs received at the second joystick 212-2. In some embodiments, the first visual indicator 211-1 is dynamically updated in accordance with characteristics of a movement input received via the first joystick 212-1, and the second visual indicator 211-2 is dynamically updated in accordance with characteristics of a movement input received via the second joystick 212-2. For example, in response to receiving input at the first joystick 212-2, at least a portion of the first visual indicator 211-1 will provide a visual signal (e.g., visual indication, such as lighting up, outputting light, emitting light, generating light) to indicate that an input is received or detected at the first joystick 212-1. Absent detection of an input at the first joystick 212-1, the visual indicator 211-1 does not provide a visual indication (e.g., does not output light, does not light up). FIG. 22C provide examples of operation of the visual indicators 211-1 and 211-2, in accordance with some embodiments.

In some embodiments, the dynamically updated appearance of the visual indicators 211 corresponds to a direction of the movement input, and/or a direction of the requested movement of the mobile medical platform 200 as a whole. In some embodiments, the first visual indicator 211-1 and the second visual indicator 211-2 include light emitting devices that are configured to output a light of one or more of multiple selected colors, and visual indications provided by the first visual indicator 211-1 and the second visual indicator 211-2 are color coded such that a visual indication corresponding to a first color has a different meaning or different indication than a visual indication corresponding to a second color different from the first color. For example, outputting green light may be used as a visual feedback indicating that the pendant is receiving input and will execute the received input. In contrast, outputting red light may be used as a visual feedback indicating that the input device 201 is receiving input (e.g., the input is detected by the input device 201) but is unable to execute the received input (e.g., due to restrictions, input error), in accordance with some embodiments.

In some embodiments, the first visual indicator 211-1 associated with the first joystick 212-1 is disposed adjacent to the first joystick 212-1 (e.g., around a peripheral region of the first joystick 212-1, next to two or more boundary points of the input region of the first joystick 212-1, etc.). In some embodiments, the second visual indicator 211-2 associated with the second joystick 212-2 is disposed adjacent to the first joystick 212-2 (e.g., around a peripheral region of the second joystick 212-2, next to two or more boundary points of the input region of the second joystick 212-2).

In some embodiments, the display 214 is configured to provide (e.g., display) information reflecting a current operating mode and/or provide information regarding a status of the mobile medical platform 200 or the table top 225 of the mobile medical platform 200 while the input device 201 is operating in any of the mobility mode and the table top mode. In some embodiments, the display 214 is a touch-sensitive display (e.g., a touch screen display, a touch-sensitive surface integrated with visual indicators that provide dynamically updated visual feedback and visual prompt for inputs) that is configured to receive user inputs and gestures such as a tap (e.g., press), double tap, a swipe, etc. In such cases, the display 214 is, optionally, configured to display one or more affordances for viewing a status of the mobile medical platform 200 and/or controlling functions of the medical platform 200. For example, the display 214 may provide (e.g., display) an affordance to lock the input device 201 (e.g., ignore inputs at the input device 201 until unlocked) or an affordance to switch between operating the input device 201 the mobility mode and the table top mode, in accordance with some embodiments. In another example, the display 214 may provide (e.g., display) affordances for a user to view different statuses of the medical platform 200, such as viewing a tilt of the table top 225, or viewing a height of the table top 225, etc. In yet another example, the display 214 may provide (e.g., display) affordances for a user to view different mobility parameters of the mobile medical platform 200, such as a current speed, a turning radius, etc. In some embodiments, the display 214 is configured to provide display menu settings and/or input device 201 menu settings, in accordance with some embodiments.

In some embodiments, the display 214 is configured to display a first user interface (e.g., a first view of the user interface) while the input device 201 is in the mobility mode. While the input device 201 is in the mobility mode, the input device is configured to operate in a horizontal format such that the display 214 provides user interfaces corresponding to the mobility mode (e.g., first user interface, first view of the user interface, etc.) in a landscape orientation (e.g., corresponding to a first range of angles and orientations relative to a vertical axis in the direction of gravity), and joystick movement is based on a horizontal orientation (e.g., corresponding to a first range of angles and orientations relative to a vertical axis in the direction of gravity) of the input device 201. The first user interface is configured to display information (and optionally, affordances) related to mobility functions of the mobile medical platform 200. For example, while the input device 201 is operating in the mobility mode, the display 214 may display information regarding a speed of the mobile medical platform 200, a turning radius of the mobile medical platform 200, or an expected pathway of the mobile medical platform 200 based on user input at the input device 201. In another example, the display 214 may provide (e.g., display) one or more affordances, such as an affordance to place the wheels of the wheel assemblies 227 in a preset configuration (e.g., a preset braking configuration, a preset roll forward configuration, a preset circular motion configuration, etc.), in accordance with some embodiments.

In some embodiments, the display 214 is configured to display a second user interface (e.g., a second view of the user interface), that is different from the first user interface, while the input device 201 is in the table top mode. While the input device 201 is in the table top mode, the input device 201 is configured to operate in a vertical format such that the display 214 provides user interfaces corresponding to the table top mode (e.g., second user interface, second view of the user interface) in a vertical orientation (e.g., portrait orientation, a second range of angles and orientations relative to a vertical axis in the direction of gravity, etc.), and joystick movement is based on a vertical orientation (e.g., corresponding to a second range of angles and orientations relative to a vertical axis in the direction of gravity) of the input device 201. The second user interface is configured to display information (and optionally, affordances) related to table top functions of the mobile medical platform 200, in some embodiments. For example, while the input device 201 is operating in the table top mode, the display 214 may display information regarding a current position or orientation of the table top 225 relative to the rigid base 221. In another example, the display 214 may provide (e.g., display, enable, etc.) one or more affordances, that allows the user translate or rotate the table top 225 based on a gesture provided at the touch-sensitive display 214, in accordance with some embodiments.

In some embodiments, the display 214 may display information regarding an executed function, such as a maximum speed at which the mobile medical platform 200 was moved or an angle or position at which the table top 225 was placed. In some embodiments, the display 214 may display an error message or a warning message, such as when the input device 201 is disconnected from the mobile medical platform 200, when a user input is not allowed or not possible, if an obstruction is detected, or if a component of the mobile medical platform 200 is not functioning properly (e.g., wheel is not rotating). In some embodiments, the display 214 may display a notification or alert. For example, the display 214 may display an alert when the mobile medical platform 200 is reaching a safety speed limit or if a user input is received to initiate power-assisted mobility of the mobile medical platform 200 while the table top 225 or one or more robotic arms 205 is not in a position conducive to transportation of the mobile medical platform 200 (e.g., table top 225 is in a Trendelenburg or reverse Trendelenburg position, and/or one or more robotic arms are not stowed, etc.)

In some embodiments, the display 214 may provide (e.g., display) an affordance for switching from the mobility mode to the table top mode, or vice versa. In some embodiments, the display 214 may provide (e.g., display, enable, etc.) an affordance for locking and/or unlocking the input device 201 (e.g., placing the input device 201 in a locked mode such that inputs received at the input device 201 are ignored until the input device 201 is unlocked, and unlocking the input device 201 from the locked mode such that the input device 201 responds to received user inputs). In some embodiments, the display 214 may provide (e.g., display, enable, etc.) an affordance for locking and/or unlocking mobility functions the mobile medical platform 200 (e.g., placing and/or releasing the wheels of the wheel assemblies 227 in/from a preset braking configuration to prohibit or restrict motion of the mobile medical platform 200).

In some embodiments, the display 214 is a color display, such as a display based on LCD, LED, and/or other presently available or future developed display technologies, etc.

In some embodiments, the one or more affordances 216 (e.g., control affordances) of input device 201 may correspond to specific functions of the mobile medical platform 200, such as changing a height of the table top 225 relative to the rigid base 221 of the mobile medical platform 200 or a preset set orientation of the table top 225 of the mobile medical platform 200. Each affordance of the one or more affordances 216 includes an icon that illustrates the function of the affordance. For example, affordance 216-1 corresponds to tilting the table top 225 into a Trendelenburg position such that the head end of the table top 225 (e.g., an end of the table top 225 where a patient's head would be located) is lower than the feet end (e.g., an end of the table top 225 where a patient's feet would be located) of the table top 225, in accordance with some embodiments.

In some embodiments, the one or more affordances 216 of input device 201 may include an emergency stop (e.g., hardwired emergency stop) affordance configured to halt all operations of the mobile medical platform 200 when activated (e.g., when pressed, depressed). For example, in response to activation (e.g., pressing, depression) of the emergency stop affordance, any functions of the mobile medical platform 200 that are being executed are immediately halted. Halting functions of the mobile medical platform 200 may include manual and power-assisted mobility functions such that the mobile medical platform 200 is immediately immobilized (e.g., brakes are applied, wheels are oriented in a predefined braking configuration) or propulsion of the mobile medical platform 200 is ceased. Halting functions of the mobile medical platform 200 may also include movement of the table top 225 and/or movement of the one or more robotic arms 205.

In some embodiments, the one or more affordances 216 includes one or more control affordances, such as lock control affordance 216-2 and unlock control affordance 216-3. The lock control affordance 216-2 corresponds to locking the mobile medical platform 200 (e.g., placing the wheels of the wheel assemblies 227 in a preset braking configuration to prohibit or restrict motion of the mobile medical platform 200), and the unlock control affordance 216-3 corresponds to unlocking the mobile medical platform 200 (e.g., releasing the wheels of the wheel assemblies 227 from the preset braking configuration to allow motion of the mobile medical platform 200, or placing the wheels of the wheel assemblies 227 in a preset mobile configuration such as a roll forward configuration or a circular motion configuration to allow for motion of the mobile medical platform 200, etc.). In some embodiments, the input device 201 automatically operates in the mobility mode in response to activation (e.g., depression) of the unlock control affordance 216-3. For example, depression of the unlock control affordance 216-3 while the input device 201 is in the mobility mode allows the input device 201 to continue to operate in the mobility mode, and depression of the unlock control affordance 216-3 while the input device 201 is in the table top mode automatically transitions (e.g., switches) the input device 201 to operate in the mobility mode. In some embodiments, the input device 201 automatically operates in the table top mode in response to activation (e.g., depression) of the lock control affordance 216-2. For example, depression of the lock control affordance 216-2 while the input device 201 is in the table top mode allows the input device 201 to continue to operate in the table top mode, and depression of the lock control affordance 216-2 while the input device 201 is in the mobility mode automatically transitions (e.g., switches) the input device 201 to operate in the table top mode, in accordance with some embodiments.

In some embodiments, the lock control affordance 216-2 and the unlock control affordance 216-3 are presented (e.g., combined, incorporated) as a single control affordance (e.g., one button). In such cases, depression of the lock/unlock control affordance at a first time corresponds to locking the mobile medical platform 200 (e.g., placing the mobile medical platform 200 in a locked or stationary mode) and depression of the lock/unlock control affordance at a second time after the first time corresponds to unlocking the mobile medical platform 200 (e.g., placing the mobile medical platform 200 in an unlocked or transport mode) such that subsequent depressions of the lock/unlock control affordance toggles between locking and unlocking the mobile medical platform 200.

In some embodiments, activation (e.g., pressing, engaging, disengaging, toggling, etc.) of the control affordance (e.g., the lock control affordance 216-2, unlock control affordance 216-3, etc.) switches (e.g., toggles) between multiple modes (e.g., the first and second modes, and optionally, additional mode(s)) of operation. In some embodiments, the control affordance is a physical button or a physical switch, lever, or other multi-state physical control.

In some embodiments, the motion control affordance 218 is associated with preset criteria for mobilizing or braking the mobile medical platform 200. For example, the preset criteria may require detection of user input at the motion control affordance 218 (e.g., the motion control affordance 218 is activated, initiated, pressed, depressed, etc.) in order to initiate and/or allow continued movement of the mobile medical platform 200 or in order to initiate and/or continue propulsion and orientation of the wheels of the wheel assemblies 227. In another example, the preset criteria may require that user input at the motion control affordance 218 is maintained (e.g., constantly depressed, activated, pressed, etc.) while the mobile medical platform 200 and/or the wheels of the wheel assemblies 227 are in motion. For example, in accordance with a determination that the motion control affordance 218 is activated (e.g., is pressed, depressed, etc.) and a determination that user input is received at a joystick 212, motion (e.g., movement, transport, steering, etc.) of the mobile medical platform 200 is allowed (e.g., the wheels can be rolled manually or via power-assisted mobility, movement of the wheels are not restricted, etc.). In another example, in accordance with a determination that the motion control affordance 218 is not activated and a determination that user input is received at a joystick 212, motion (e.g., movement, transport, steering, etc.) of the mobile medical platform 200 is prohibited (e.g., the wheels are locked, wheels cannot be rolled manually or via power-assisted mobility, movement of the wheels are restricted, movement of the wheels are prohibited, etc.).

In some embodiments, activation (e.g., pressing, engaging, disengaging, toggling, etc.) of the motion control affordance 218 automatically transitions the input device 201 to the mobility mode independently of whether or not the unlock control affordance 216-3 is activated (e.g., pressed, depressed, etc.).

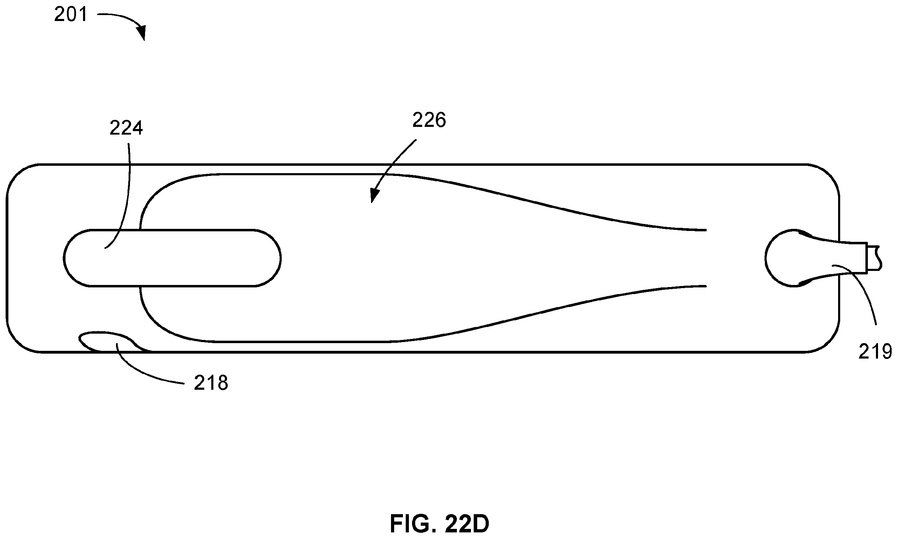

In some embodiments, the first and second joysticks 212-1 and 212-2 are disposed on a first side (e.g., front side, display side, etc.) of the input device 201, as shown in FIG. 22A and 22B, and the motion control affordance 218 is disposed on a second side of the input device 201, such as along the edge of the input device 201, as shown in FIGS. 22A and 22B, or on back side of the input device 201, shown in FIG. 22D. In some embodiments, the joysticks and the motion control affordances are disposed on the same side of the input device 201.

The input device 201 is configured to receive different user input types, including touch inputs (such as gestures, swipes, taps, press and hold, etc.), joystick inputs (such as joystick movement, joystick displacement, joystick tilt and rotation relative to an axis or pivot, etc.), and touch inputs (such as touch and hold, pressing, or depressing on a physical affordance, a touch-sensitive surface or button, etc.). The input device 201 is configured to receive any combination of the different input types, such as a combination of multiple preset input modalities. In some embodiments, the input device 201 is configured to simultaneously receive a plurality of inputs of different input types, such as touching or depression of the motion control affordance 218 detected in conjunction with (e.g., concurrently with, within a preset time window of, during overlapping time periods, etc.) movement of a joystick 212.

In some embodiments, the input device 201 is configured to receive user inputs corresponding to movement of the mobile medical platform 200 while the input device 201 is in the mobility mode, and to receive user inputs corresponding to positioning and orientation of the table top 225 while the input device 201 is in the table top mode. Thus, while the input device 201 is in the mobility mode, inputs received at affordances corresponding to table top 225 positioning or table top 225 orientation are ignored (e.g., locked, disabled), in accordance with some embodiments. For example, while the input device 201 is in the mobility mode, a button press at affordance 216-1 is ignored (e.g., does not result in an executed function at the mobile medical system 200), in accordance with some embodiments. Similarly, while the input device 201 is in the table top mode, inputs received at affordances corresponding to movement of the mobile medical platform 200 are ignored (e.g., locked, disabled, not executed), in accordance with some embodiments. For example, while in the table top mode, wheels of the wheel assemblies 227 are locked or in aligned (e.g., oriented) in a preset braking configuration to prohibit movement (e.g., transportation) of the mobile medical platform 200; however, inputs at the motion control affordance 218, the lock control affordance 216-2, and the unlock control affordance 216-3 are accepted (e.g., not ignored) during operation of the input device 201 in any of the mobility mode and the table top mode as those affordances can be used to transition (e.g., toggle, switch) between modes of operation, in accordance with some embodiments. In some embodiments, the input device 201 is configured to operate in one mode at any given time. Thus, operation of the input device 201 in the mobility mode concurrently with operation of the input device 201 in the table top mode is not allowed (e.g., cannot simultaneously control movement of the mobile medical platform 200 and positioning/orientation of the table top 225), in accordance with some embodiments. For example, while in the mobility mode, the input device 201 is configured to receive inputs to control mobility or movement the mobile medical platform 200 without changing the position or orientation of the table top 225 (e.g., without changing the status of the mobile medical platform 200, while prohibiting changes to the position and orientation of the table top 225), in accordance with some embodiments. While in the table top mode, the input device 201 is configured to receive inputs to control position and orientation of the table top 225 without changing moving or mobilizing the mobile medical platform 200 (e.g., the mobile medical platform remains stationary and cannot be moved while controls to change a position or orientation of the table top 225 received at the input device 201 are executed), in accordance with some embodiments.

In some embodiments, the input device 201 includes one or more actuators for providing haptic feedback (e.g., vibrations) in response to user input. The haptic feedback can be provided in addition to or in lieu of visual feedback, in some embodiments. For example, in some embodiments, in response to receiving user input that cannot be executed, the input device 201 vibrates to alert to user that the requested function(s) cannot be executed. Depending on the input received at the input device 201, the input device 201 may provide visual feedback, haptic feedback, or a combination or both visual and haptic feedback, in accordance with some embodiments.

In some embodiments, the input device 201 includes an accelerometer (e.g., 3-axis accelerometer, a gyro, a motion sensor) that can detect a motion of the input device 201 (including changes in a physical orientation of the input device 201). For example, the input device 201 may determine that the input device 201 is in a falling motion when the accelerometer reading exceeds a threshold value or when a rate of change in the movement speed of the input device 201 is greater than a threshold rate, in accordance with some embodiments. In some embodiments, in response to detecting a falling motion of the input device 201 (e.g., via the accelerometer), the input device 201 activates a lock-out mode in which user inputs (e.g., any user inputs, including button presses, joystick movements, touch screen contacts) directed to the input device 201 are ignored (e.g., not detected, not processed). In some embodiments, the lock-out mode may also include orienting motorized wheels of the wheel assemblies 227 in a predefined braking configuration.

In some embodiments, a mode of operation of the input device 201 is independent on a physical orientation of the input device 201. In such cases, while the input device is operating in a specific mode (either the mobility mode or the table top mode), the input device 201 continues to operate in the same mode until a user provides an input to switch operation in a different mode, regardless of a physical orientation of the input device 201. In some embodiments, a mode of operation of the input device 201 is dependent on a physical orientation of the input device 201. In such cases, the input device 201 automatically operates in the mobility mode when a horizontal (e.g., landscape) orientation of the input device 201 is detected, and automatically operates in the table top mode when a vertical (e.g., portrait) orientation of the input device 201 is detected. In such cases, in response to detecting a change in the physical orientation of the input device 201, such as a change from the from a horizontal orientation to a vertical orientation, the input device 201 automatically transitions from operation in the mobility mode to operating in the table top mode.

In some embodiments, the input device 201 includes a wired connection 219 (e.g., electrical wire) that provides an electrical connection between the input device 201 and the mobile medical platform 200.

FIG. 22C illustrates representative visual feedback that can occur at the input device 201 of FIGs. 22A and 22B in accordance with some embodiments. The visual indicators 211 (e.g., visual indicators 211-1 and 211-2) are configured to provide visual feedback (e.g., a visual indication) to a user of the input device 201 by outputting light (e.g., emitting light, generating light, lighting up, etc.) in response to detection of user input. In this example, visual indicator 211-1 is associated with joystick 212-1 and thus, is configured to output light in response to detecting input at joystick 212-1. Similarly, visual indicator 211-2 is associated with joystick 212-2 and thus, is configured to output light in response to detecting input at joystick 212-2.

In some embodiments, the visual indicator 211 is divided into a plurality of portions. For example, the visual indicator 211 may be divided into quadrants corresponding to the up, down, left, and right directions. In another example, the visual indicator 211 may be divided into eight portions corresponding to the up, down, left, right, and diagonal directions. In the example shown in FIG. 22C, the visual indicator 211 is divided into quadrants. As shown, the left quadrant of the visual indicator 211-1 is illuminated (e.g., is outputting light is lit up), indicating that the input device 201 detects user input corresponding to a movement to the left of the joystick 212-1. Similarly, an upper quadrant of the visual indicator 211-2 is illuminated (e.g., is outputting light, is lit up), indicating that the input device 201 detects user input corresponding to an upwards displacement of the joystick 212-2.

In some embodiments, the visual indicator 211 may be configured to output light having a plurality of colors. For example, the visual indicator 211 may output red light in response to detection of user input that is not allowed (e.g., is not accepted, is not executed). Alternatively, the visual indicator 211 may output green light in response to detection of user input that is allowed (e.g., is accepted, is executed).

FIG. 22D illustrates a back view of the input device 201 of FIGs. 22A and 22B in accordance with some embodiments. The input device 201 includes a hook 224 for hanging (e.g., storing, attaching) the input device 201 to the table top 225, such as by mounting (e.g., hanging) the input device 201 on the bar 223 or on the mount 222 shown in FIG. 21B, in accordance with some embodiments. The input device 201 also includes a cut-out region 226 that provides an ergonomic contoured back surface for user comfort when holding and using the input device 201. In some embodiments, as shown in FIG. 22D, the motion control affordance 218 is disposed on the back of the input device 201 and can be easily activated (e.g., pressed, held down) by a user while using other functions of the input device 201.

FIGs. 23A - 23D provide details regarding operation of the input device 201 in the mobility mode, in accordance with some embodiments. Inputs received at the input device 201 while the input device 201 is in the mobility mode correspond to movement of the mobile medical platform 200, in accordance with some embodiments. While in the mobility mode, the input device 201 is configured to receive inputs to control movement of the mobile medical platform 200 at any of the joysticks 212 and the touch-sensitive display 214 of the input device 201, in accordance with some embodiments. While in the mobility mode, the input device 201 is also configured to display mobility information regarding the mobile medical platform 200, in accordance with some embodiments.

FIG. 23A illustrates examples of user interfaces 230 at a display 214 of the input device 201 of FIGs. 22A and 22B for controlling assisted mobility of the mobile medical platform 200 of FIGs. 21A and 21B, in accordance with some embodiments. The input device 201 is configured to provide (e.g., display, enable, etc.) a first user interface 230 (e.g., a first view 230 of the user interface) while the input device 201 is operating in the mobility mode. For example, the input device 201 provides user interface 230-1 to unlock the mobile medical platform 200. The user interface 230-1 includes a "BACK" affordance to return to a previous screen (e.g., a previous interface or interface view, such as a menu screen, for example), and an affordance 231 that, when activated (e.g., pressed, press and hold, press and slide), unlocks the mobile medical platform 200 (e.g., permits movement and transportation of the mobile medical platform 200). In another example, the input device 201 may display user interface 230-2 while the input device 201 is operating in the mobility mode to provide instructions on how to use the joysticks 212-1 and 212-2 to control movement of the mobile medical platform 200. The user interface 230-2 also provides (e.g., displays, enable, etc.) a "CANCEL" affordance and a "DONE" affordance for canceling an action or confirming execution of an action at the mobile medical system 200. In both examples, the user can interact directly with the affordances provided by the display 214 as part of the user interface. For example, the user can directly tap (or press and hold) affordance 231 to unlock the mobile medical platform 200 and allow movement (e.g., mobility, transportation) of the mobile medical platform 200. Alternately, the user may interact with another affordance, such as affordance 216-3 (shown in FIG. 22B) to unlock the mobile medical platform 200.

In some embodiments, while the input device 201 is in the mobility mode, the display 214 may display a user interface 230 that provides information and/or feedback regarding inputs received (e.g., detected) at the input device 201. For example, the display 214 may display a current speed of the mobile medical platform 200 while a user is moving (e.g., driving) the mobile medical platform 200 via inputs provided at the input device 201.

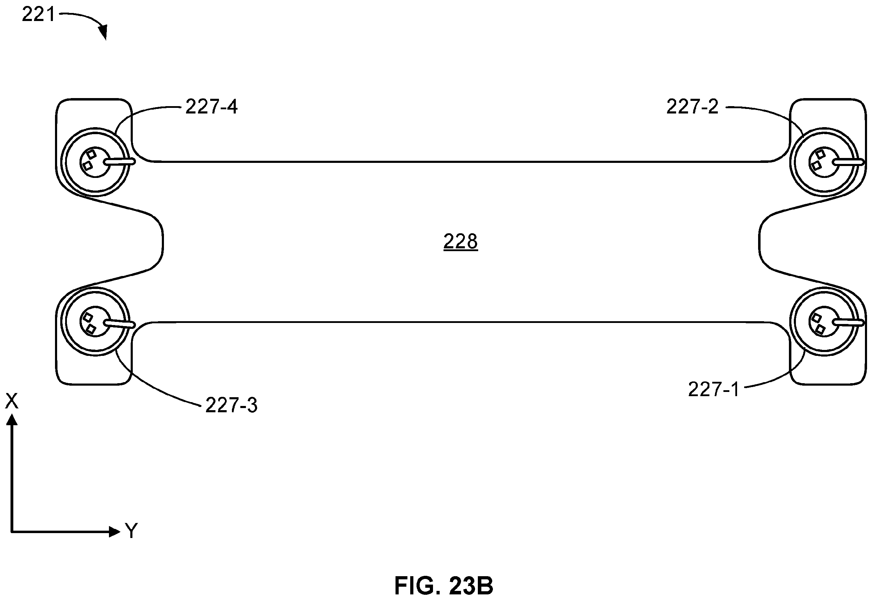

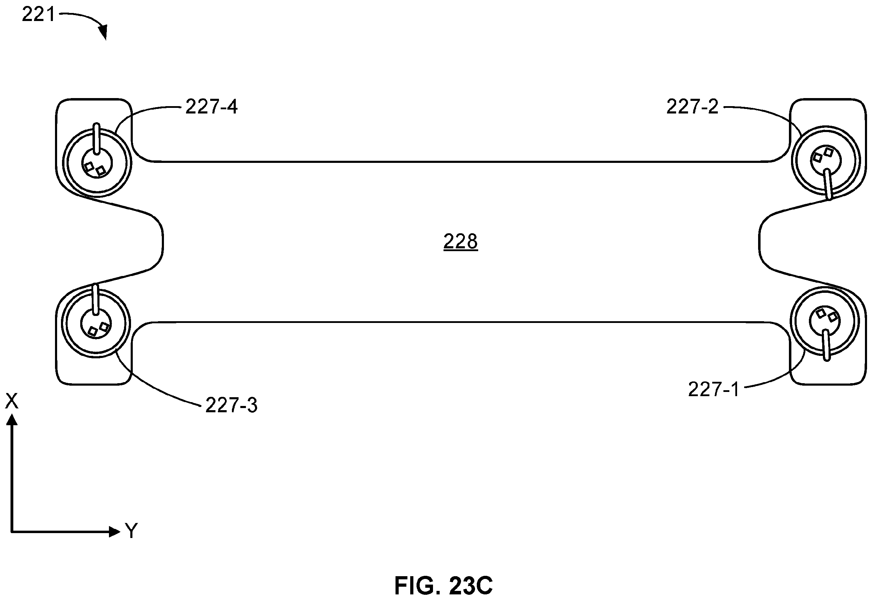

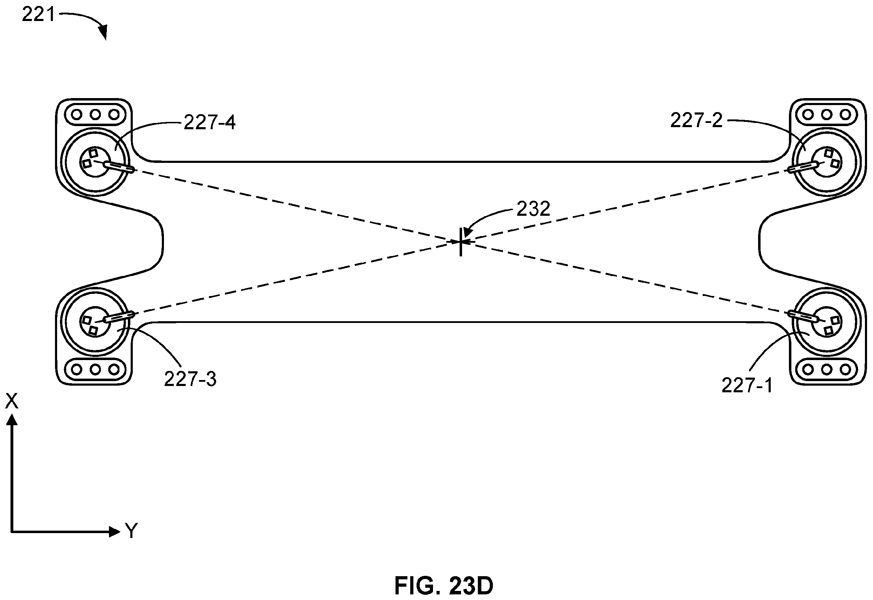

FIGs. 23B - 23D illustrate different wheel configurations of the mobile medical platform 200 of FIGs. 21A and 21B, in accordance with some embodiments. In some embodiments, a user can provide inputs at the input device 201 to orient wheels of the wheel assemblies 227 in a specific configuration, including any of a linear motion configuration (also referred to herein as a roll-forward configuration) shown in FIG. 23B, a circular motion configuration shown in FIG. 23C, or a preset braking configuration shown in FIG. 23D, for example. In some embodiments, a configuration of the wheels of the wheel assemblies 227 is determined based on user inputs at the input device 201. For example, the wheels of the wheel assemblies 227 may be placed in a specific configuration in response to user inputs corresponding to a change in a mode of operation of the input device 201 (e.g., transitioning between the mobility and table top modes of operation) or in response to user inputs corresponding to a change in a mode of the mobile medical platform (e.g., stationary mode versus transport mode). For example, in some embodiments, in response to initiating (or transitioning to) the mobility mode on the input device 201, wheels of the wheel assemblies 227 of the mobile medical platform 200 are oriented in a linear motion configuration (also referred to herein as a roll-forward configuration) shown in FIG. 23B or a circular motion configuration shown in FIG. 23C. In another example, in response to initiating (or transitioning to) the table top mode on the input device 201, wheels of the wheel assemblies 227 of the mobile medical platform 200 are oriented in a preset braking configuration shown in FIG. 23D, in accordance with some embodiments.

Referring to FIG. 23B, in the linear motion configuration, the wheels are oriented in a same direction, allowing for linear movement of the mobile medical platform 200 either via manual (e.g., user pushing the mobile medical platform 200) or motorized propulsion (e.g., power-assisted propulsion of the mobile medical platform 200) without rotation of the mobile medical platform 200, in accordance with some embodiments.

Referring to FIG. 23C, in the circular motion configuration, the wheels at one end (e.g., head end) of the mobile medical platform 200 are oriented in a different direction than the wheels at another end (e.g., feet end, an opposite end, etc.) of the mobile medical platform 200, allowing for circular movement (e.g., rotation, pivot, etc.) of the mobile medical platform 200 either via manual (e.g., user pushing the mobile medical platform 200) and/or motorized propulsion (e.g., power-assisted propulsion of the mobile medical platform 200) around without lateral translation of the mobile medical platform 200, in accordance with some embodiments.

In some embodiments, the wheels of the wheel assemblies 227 of the mobile medical platform 200 are automatically oriented in the linear motion configuration shown in FIG. 23B or the circular configuration shown in FIG 23C in response to activation (e.g., depression, press, press and hold, etc.) of the motion control affordance 218, in accordance with some embodiments. In some embodiments, the wheels of the wheel assemblies 227 of the mobile medical platform 200 are automatically oriented in the linear motion configuration shown in FIG. 23B or the circular configuration shown in FIG 23C in response to activation (e.g., depression, press, press and hold) of the unlock control affordance 216-3.

In some embodiments, a user may provide input at the input device 201 for the mobile medical platform 200 to be manually moveable, where power-assisted mobility may be used for orienting the wheels of the wheel assemblies 227 but not for propelling (e.g., rolling) the wheels.

Referring to FIG. 23D, in the preset braking motion configuration, the wheels at the head end of the mobile medical platform 200 are oriented in different directions (represented by dashed lines) so that the respective wheels are not parallel to one another and movement of the mobile medical platform 200 is not possible, in accordance with some embodiments. In some embodiments, the wheels of the wheel assemblies 227 are directed to a common point, such as a centroid 232 of the wheel assemblies 227, while in the preset braking configuration. For example, as shown in FIG. 23D, the wheels of the wheel assemblies 227 are oriented to form an "X" shape when in the preset braking configuration, in accordance with some embodiments.

In some embodiments, the wheels of the wheel assemblies 227 are automatically oriented in the preset braking configuration shown in FIG. 23D while the input device 201 is in the table top mode. In some embodiments, the wheels of the wheel assemblies 227 are automatically oriented in the preset braking configuration shown in FIG. 23D while the mobile medical platform 200 is in a stationary mode. In some embodiments, the wheels of the wheel assemblies 227 are automatically oriented in the preset braking configuration shown in FIG. 23D whenever activation of the motion control affordance 218 is not detected (e.g., whenever the motion control affordance 218 is not pressed, held down, depressed). In some embodiments, the mobile medical system 200 is configured to determine whether or not user input is received at the input device 201 within a predetermined time period, and in accordance with a determination that user input has not been received at the input device 201 within the predetermined time period, the motorized wheels of the one or more wheel assemblies 227 are oriented in the braking configuration to restrict movement of the mobile medical system 200. For example, the wheels of the wheel assemblies 227 are automatically oriented in the preset braking configuration shown in FIG. 23D in response to the input device 201 being idle after a predetermined period of time (e.g., when user inputs are not detected or received at the input device 201 after a predetermined period of time). In some embodiments, the wheels of the wheel assemblies 227 are automatically oriented in the preset braking configuration shown in FIG. 23D in response to detecting that the mobile medical platform 200 is stationary for a predetermined period of time (e.g., when the mobile medical platform 200 is not moved after a predetermined period of time).

FIGs. 24A - 24F provide details regarding operation of the input device 201 in the table top mode, in some embodiments.

FIG. 24A illustrate examples of user interfaces 240 at a display 214 of the input device 201 of FIGs. 22A and 22B for operating a table top 225 of the mobile medical platform 200 of FIGs. 21A and 21B, in accordance with some embodiments. The input device 201 is configured to provide (e.g., display, enable, etc.) a second user interface 240 (e.g., a second view 240 of the user interface) while the input device 201 is operating in the table top mode, in some embodiments. The user interfaces 240 corresponding to the table top mode are different from the user interfaces 230 corresponding to the mobility mode. The user interfaces 240 corresponding to the table top mode provide information and/or feedback regarding operation of the input device 201 (and executed functions regarding the table top 225 of the mobile medical platform 200) while the input device 201 is in the table top mode, in accordance with some embodiments. For example, the input device 201 provides user interface 240-1 indicating a current tilt angle of the table top 225 relative to the rigid base 221. Additionally, the display 214 also provides affordances 241 and 242, allowing a user to interact with either affordance 241 (e.g., a sliding bar) or affordance 242 to adjust a tilt angle of the table top 225. A user can, optionally, adjust a tilt angle of the table top via other inputs on the input device 201, such as a joystick 212 or a pre-programmed affordance 216. In another example, the input device 201 may display user interface 240-2 while the input device 201 is operating in the table top mode. The user interface 240-2 allows a user to select which status of the table top 225 to view or control. For example, the user may touch the "Tilt" button in order to imitate display of the user interface 240-1, allowing the user to view and adjust the tilt angle of the table top 225, in accordance with some embodiments.

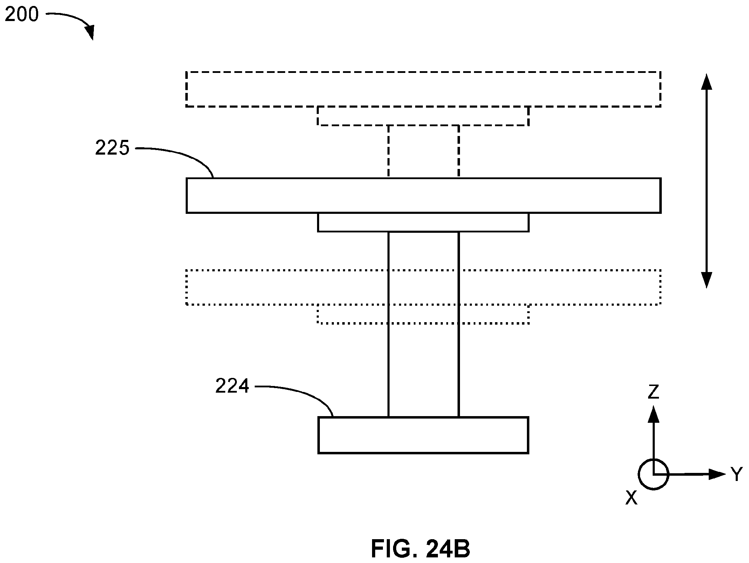

FIG. 24B illustrates vertical translation of a table top 225 of the mobile medical platform 200 of FIGs. 21A and 21B, in accordance with some embodiments. FIG. 24B is a side view of the mobile medical platform system 200, with the x-axis extending into the page, that illustrates axial translation along the z-direction of the table top 225 relative to the rigid base 221. Movement of the table top 225 in the axial direction, represented by the double-ended arrow, allows the table top 225 to be raised or lowered relative to a position of the rigid base 221. The table top 225 is illustrated in three positions: the solid lines represent the table top 225 in an untranslated position, the dashed lines represent the table top 225 in a raised position, and the dotted lines represent the table top 225 in a lowered position.

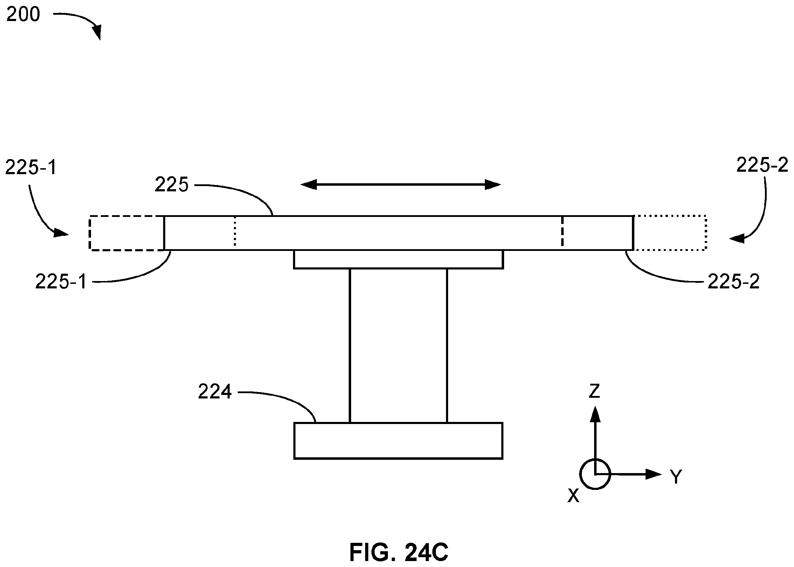

FIG. 24C illustrates lateral translation of a table top 225 of the mobile medical platform 200 of FIGs. 21A and 21B, in accordance with some embodiments. FIG. 24C is a side view of the patient platform system 200, with the x-axis extending into the page, that illustrates longitudinal translation (along the y-direction) of the table top 225 relative to the rigid base 221. Movement of the table top 225 along the y-direction, as represented by the double-ended arrows, allows the table top 225 to slide towards a head end 225-1 or a feet end 225-2 of the patient platform system 200 relative to the rigid base 221. The table top 225 is illustrated in three positions: the solid lines represent the table top 225 in an untranslated position (e.g., a center of the table top 225 is aligned with a center of the rigid base 221), the dashed lines represent a position where the table top 225 is translated towards the head end 225-1 of the patient platform system 200, the dotted lines represent a position where the table top 225 is translated towards the feet end 225-2 of the patient platform system 200.

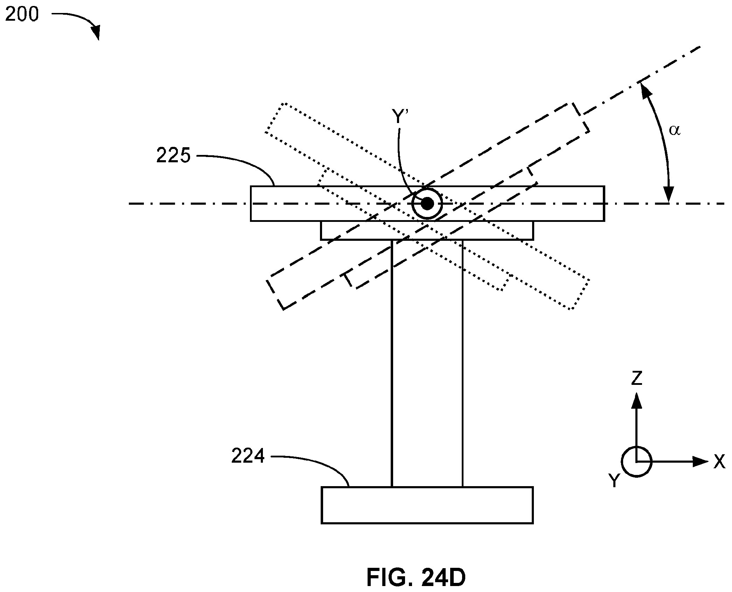

FIG. 24D illustrates rotation of a table top 225 along a longitudinal axis of the mobile medical platform 200 of FIGs. 21A and 21B in accordance with some embodiments. FIG. 24D is an end view of the patient platform system 200, with the y-axis extending into the page, that illustrates rolling the table top 225 around a longitudinal axis Y' that is parallel to the y-axis (e.g., extends in the y-direction) of the patient platform system 200. This allows the table top 225 to be rolled or rotated left to right. The table top 225 is illustrated in three positions: the solid lines representing in an un-rotated position where the table top 225 is substantially parallel to the rigid base 221, the dashed lines representing a first rotated position, and the dotted lines representing a second rotated position. In the first rotated position, the table top 225 forms an angle α with respect to the x-axis, and in the second rotated position, the table top 225 forms a negative angle α with respect to the x-axis. In some embodiments, the table top 225 can be configured to allow at least a rotational angle α of about 5 degrees, 10 degrees, 15 degrees, 20 degrees, 25 degrees, 30 degrees, 35 degrees, 40 degrees, 45 degrees or more relative to the un-rotated position. In some embodiments, the table top 225 can be rotated in both transverse directions from the un-rotated position (e.g., the rotational angle α can be positive or negative). In some embodiments, the table top 225 can be rotated to any angle between the positions illustrated in dashed and dotted lines.

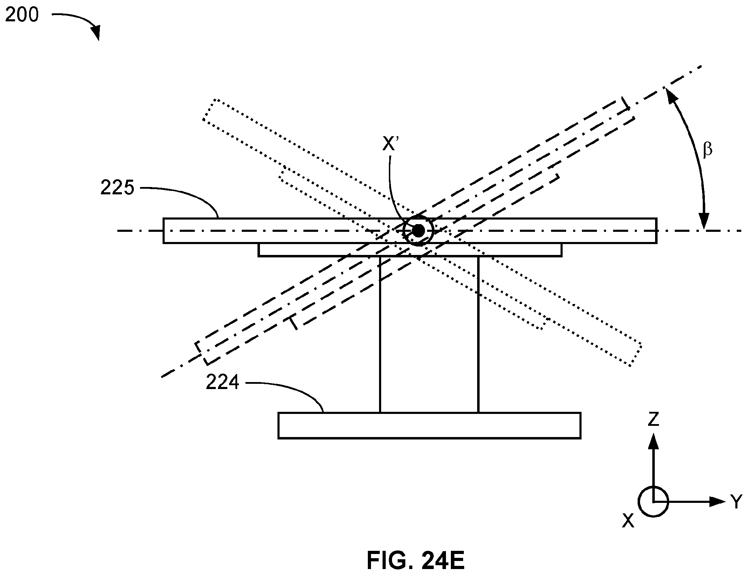

FIG. 24E illustrates rotation of a table top 225 along a lateral axis of the mobile medical platform 200 of FIGs. 21A and 21B, in accordance with some embodiments. FIG. 24E is a side view of the patient platform system 200, with the x-axis extending into the page, that illustrates pitching the table top 225 around a transverse axis X' that is parallel to the x-axis (e.g., extends in the x-direction) of the patient platform system 200. This allows the table top 225 to be pitched or tilted such the head of the table top 225 is elevated relative to the feet of the table top 225, or vice versa. The table top 225 is illustrated in three positions: the solid lines representing an un-tilted position where the table top 225 is substantially parallel to the rigid base 221, the dashed lines representing a first tilted position, and the dotted lines representing a second tilted position. In the first tilted position, the table top 225 forms an angle β with respect to the y-axis, and in the second tilted position, the table top 225 forms a negative angle β with respect to the y-axis. In some embodiments, the table top 225 can be configured to allow at least a tilt angle β of about 15 degrees, 20 degrees, 25 degrees, 30 degrees, 35 degrees, 40 degrees, 45 degrees, 50 degrees, 55 degrees, 60 degrees or more relative to the un-tilted position. In some embodiments, the table top 225 can be tilted in both longitudinal directions from the un-tilted position (e.g., the lateral tilt angle β can be positive or negative). In some embodiments, the table top 225 can be tilted to any angle between the positions illustrated in dashed and dotted lines.

Additional details regarding mechanisms associated with movement of the table top 225 are described in U.S. Patent Application Serial No. 16/810,469, filed March 5, 2020, and U.S. Provisional Application Serial No. 63/086,038, filed September 30, 2020.

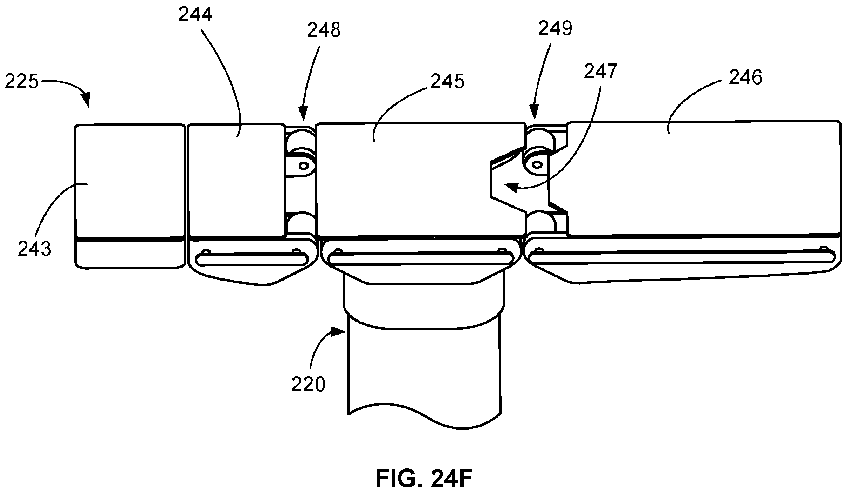

FIG. 24F illustrates an embodiment of a table top 225 of the mobile medical platform 200 of FIGs. 21A and 21B in accordance with some embodiments. In some embodiments, the table top 225 includes a plurality of sections to support different portions of the patient, such as a head board 243, an upper back support 244, a lower back support 245, and a leg board 246. Each of the plurality of sections (e.g., sections 243 - 246) are movable relative to one another, allowing table top 225 to accommodate and support different body sizes and shapes. In some embodiments, the head board 243 is removable. In some embodiments, the leg board 246 is removable. In some embodiments, the lower back support 245 includes a perineal cut-out 247. In some embodiments, the table top 225 includes a powered mechanism 248 between the upper back support 244 and the lower back support 245. The powered mechanism 248 allows the upper back support 244 to be moved (e.g., slid) or flexed (e.g., tilted) relative to the lower back support 245. In some embodiments, the table top 225 includes a powered mechanism 249 between the lower back support 245 and the leg board 246. The powered mechanism 249 allows the leg board 246 to be moved (e.g., slid) or flexed (e.g., be tilted) relative to the lower back support 245. In some embodiments, a user can control the position and orientation of the sections of the table top 225 via the input device 201. For example, the input device 201 may be configured to receive input corresponding to movement of the upper back support 244 relative to the lower back support 245, such as sliding the upper back support 244 to increase or decrease space between the upper back support 244 and the lower back support 245 and tilting the upper back support 244 relative to the lower back support 245.

In some embodiments, the input device 201 may include one or more affordances (such as affordances 216 or an affordance provided via the display 214) to position the sections 243 - 246 of the table top 225 in predefined positions or preset poses, such as a Trendelenburg position or a reverse Trendelenburg position, for example.

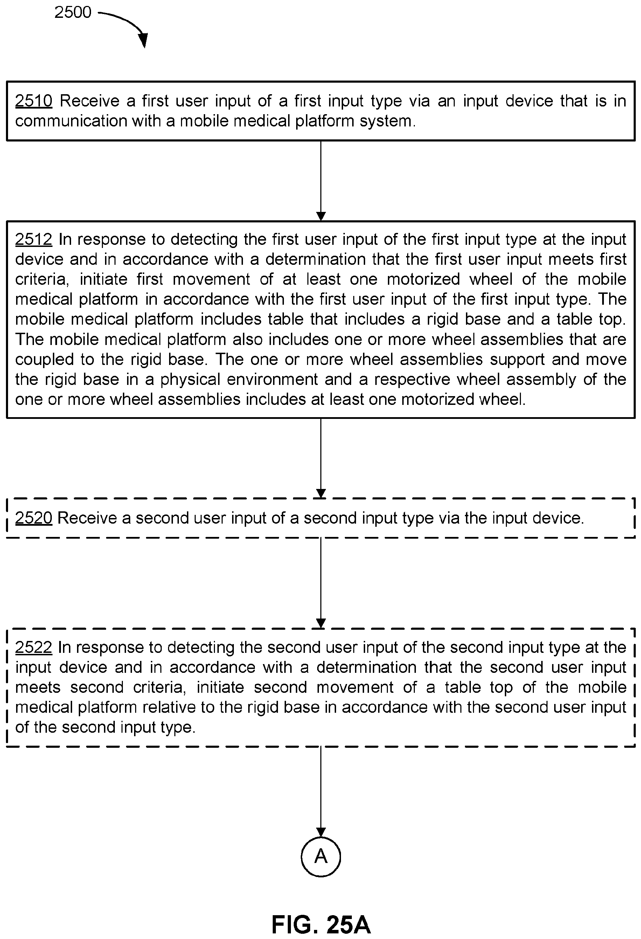

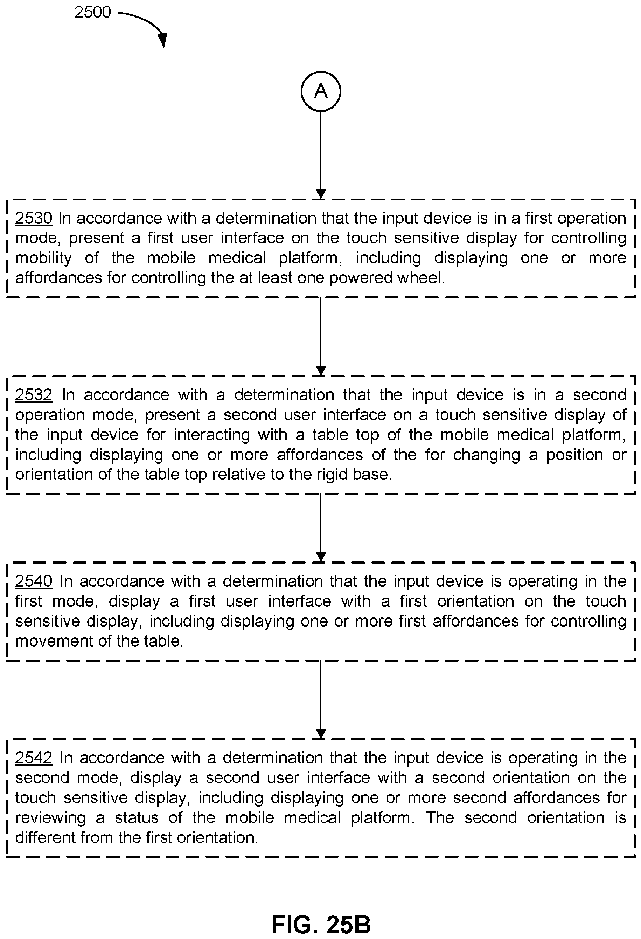

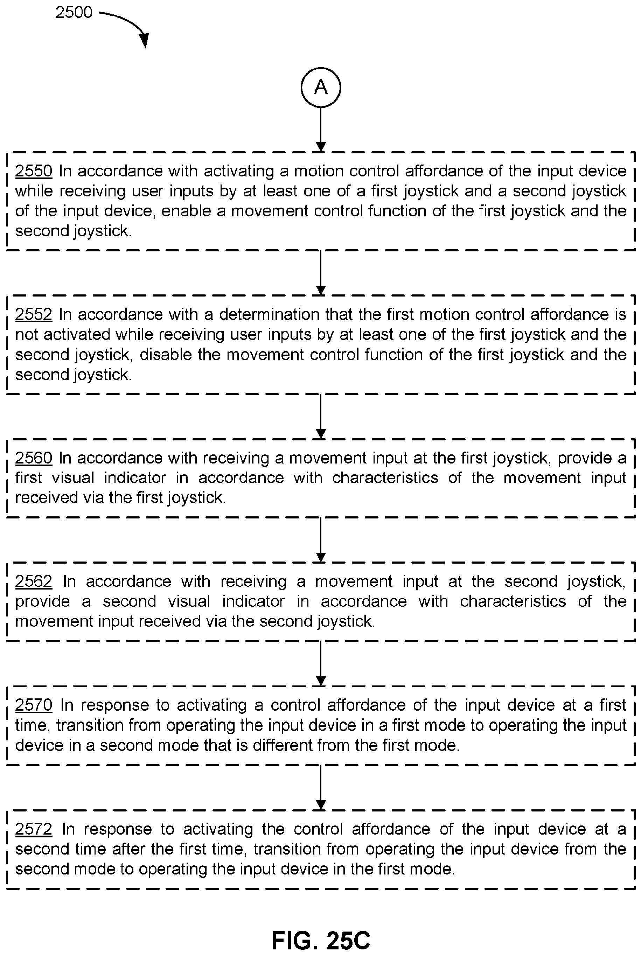

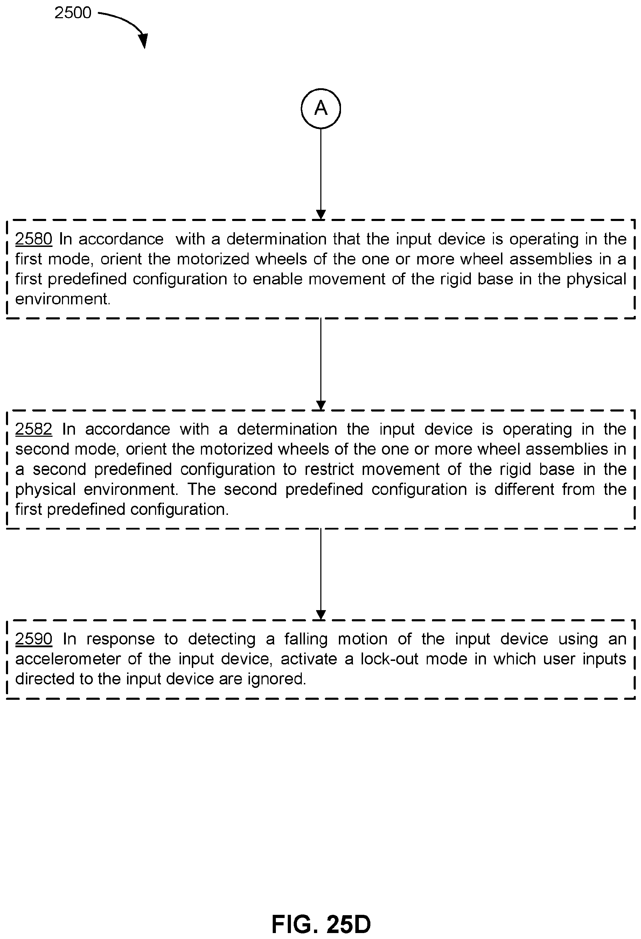

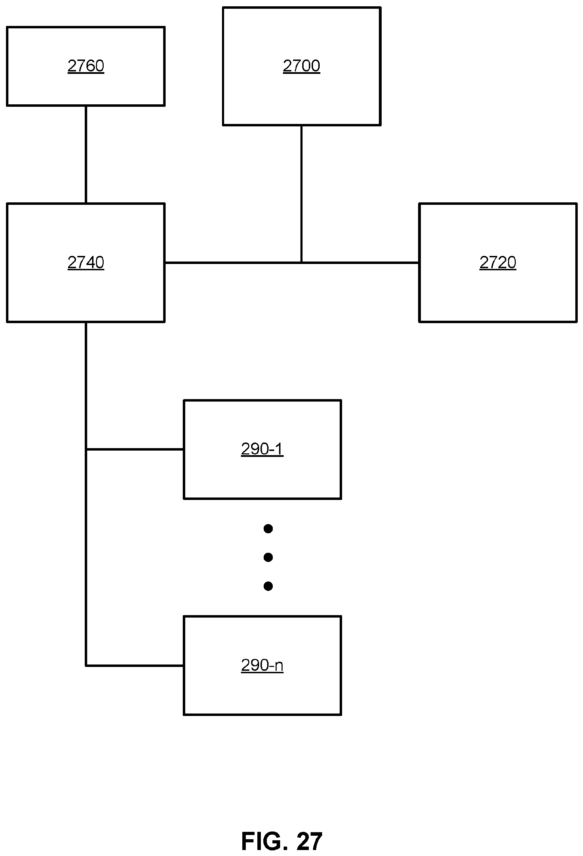

FIGS. 25A - 25D are a flowchart illustrating a method 2500 performed by an input device 201 for controlling operations of the mobile medical platform 200 of FIGs. 21A and 21B, in accordance with some embodiments. In some embodiments, the method 2500 may be performed at an electronic device (such as mobile medical platform 200 or a computer system associated with mobile medical platform 200) having one or more processors and memory storing instructions for execution by the one or more processors (details of which are provided below with respect to FIG. 27). In some embodiments, the method 2500 is performed by executing instructions stored in the memory of the electronic device.

The method 2500 includes receiving (2510) a first user input of a first input type (e.g., touch inputs such as tap, swipe, press, touch and hold; joystick movement; press inputs; a combination of multiple preset input modalities) at the input device 201 that is in communication with the mobile medical platform 200. The method 2500 also includes, in response to receiving the first user input of the first input type at the input device 201 and in accordance with a determination that the first user input meets first criteria, initiating (2512) first movement of at least one motorized wheel of the mobile medical platform 200 in accordance with the first user input of the first input type. In some embodiments, the medical platform includes table that includes a rigid base 221 and a table top 225. The mobile medical platform includes one or more wheel assemblies 227 that are coupled to the rigid base 221. The one or more wheel assemblies 227 support and move the rigid base 221 in a physical environment and a respective wheel assembly of the one or more wheel assemblies 227 includes at least one motorized wheel. The first criteria may include, for example, criteria for recognizing an input that corresponds to a request to control the mobility of the mobile medical platform 200 (e.g., control movement or transport of the mobile medical platform 200) by controlling the at least one motorized wheel (e.g., locking, unlocking, turning, rolling, stopping, pivoting, etc. one or more of the at least one motorized wheel), including a combination of conditions, thresholds, and/or input type requirements, etc.). For example, the first criteria may include detecting activation (e.g., pressing, depression, press and hold) of the motion control affordance 218 in combination with joystick movement, in accordance with some embodiments.

In some embodiments, the method 2500 also includes receiving (2520) a second user input of a second input type via the input device 201. In some embodiments, the second input is a separate or different input of than the first input. The second input type may be a same input type as the first input type (e.g., both input types are movements or a joystick or pressing of button), or the second input type may be a different input type from the first input type (e.g., the first input type is a button press and the second input type is a joystick movement). For example, the first input may be a joystick movement and the second input may be button press. In another example, the first and second inputs may be button presses at an affordance (such button press at one of the affordances 216) of the input device 201. The method 2500 further includes, in response to receiving the second user input of the second input type at the input device 201 and in accordance with a determination that the second user input meets second criteria, initiating (2522) second movement of a table top 225 of the mobile medical platform 200 relative to the rigid base 221 in accordance with the second user input of the second input type (e.g., tilting, rotating, translating vertically, translating horizontally the table top 225 relative to the rigid base 221). For example, second criteria for recognizing an input that corresponds to a request to control the position and orientation of the table top 225 relative to the rigid base 221 (e.g., by controlling one or more motors coupled to the table top 225 (e.g., between the table top 225 and the rigid base 221, to the table top 225), including a combination of conditions, thresholds, and/or input type requirements).

In some embodiments, the method 2500 includes, in accordance with a determination that the input device 201 is in a first operation mode (e.g., mobility mode, bed control mode), presenting (e.g., 2530) a first user interface 230 (e.g., user interface 230-1 or 230-2) on a touch sensitive display 214 of the input device 201 for controlling mobility of the mobile medical platform 200, including displaying one or more affordances (e.g., affordance 231) for controlling the at least one powered wheel. FIG. 23A shows examples of first user interfaces 230-1 and 230-2, including an example of a first user interface 230-1 that includes an affordance 231. The method also includes, in accordance with a determination that the input device 201 is in a second operation mode (e.g., table top mode, bed status mode), presenting (2532) a second user interface 240 on the touch sensitive display 214 for interacting with a table top 225 of the mobile medical platform 200, including displaying one or more affordances (e.g., affordance 241) of the for changing a position or orientation of the table top 225 relative to the rigid base 221. FIG. 24A shows examples of second user interfaces 240-1 and 240-2, including an example of a second user interface 240-1 that includes an affordance 241, in accordance with some embodiments.

In some embodiments, the method 2500 includes, in accordance with a determination that the input device 201 is operating in the first mode (e.g., mobility mode, bed control mode), displaying (2540) a first user interface 230 with a first orientation (e.g., horizontal orientation, landscape orientation, etc.) on the touch sensitive display 214, including displaying one or more first affordances (such as affordance 231, shown in FIG. 23A) for controlling movement of the table of the mobile medical platform 200. The method 2500 also includes, in accordance with a determination that the input device 201 is operating in the second mode (e.g., table top mode, bed status mode), displaying (2542) a second user interface 240 with a second orientation (e.g., vertical orientation, portrait orientation, etc.) on the touch sensitive display 214, including displaying one or more second affordances (such as affordance 241, shown in FIG. 24A) for reviewing a status of the mobile medical platform 200. The second orientation is different from the first orientation.

In some embodiments, the method 2500 includes, in accordance with a determination that the input device 201 is operating in a third mode (e.g., arm control mode), displaying a third user interface for controlling arms (e.g., robotic arms 205) of the mobile medical platform 200, including one or more affordances for placing the one or more robotic arms 205 in one or more predefined positions (e.g., dock arms for transport, dock arms for stowing, etc.), one or more affordances corresponding saved user settings that are stored in the memory (e.g., less commonly used motions, stored movement commands, saved memory settings, customized functions, etc.). In some embodiments, while the input device 201 has a vertical format while operating in the third mode. In such cases, display 214 provides the third user interface in a vertical orientation (e.g., portrait orientation). In some embodiments, the third user interface includes one or more affordances for controlling movement of the one or more robotic arms 205 (e.g., stowing the robotic arms, docking the robotic arms, etc.).

In some embodiments, the method 2500 further includes, in accordance with activating a motion control affordance 218 of the input device 201 while receiving user inputs by at least one of a first joystick 212-1 and a second joystick 212-2 of the input device 201, enabling (2550) a movement control function of the first joystick 212-1 and the second joystick 212-2. For example, in accordance with a determination that user inputs are received at both a joystick 212 and the motion control affordance 218, execute a movement of the mobile medical platform 200 in accordance with the user input received at the joystick 212. The method also includes, in accordance with a determination that the first motion control affordance 218 is not activated while receiving user inputs by at least one of the first joystick 212-1 and the second joystick 212-2, disabling (2552) the movement control function of the first joystick 212-1 and the second joystick 212-2. For example, in accordance with a determination that user input is received at a joystick 212 and user input is not received at the motion control affordance 218, the user input received at the joystick 212 is not executed (e.g., ignored). In some embodiments, the display 214 may provide an indication or reminder for the user to activate the motion control affordance 218 in order to execute inputs received at the input device 201.

In some embodiments, the method 2500 further includes, in accordance with receiving a movement input at the first joystick 212-1, providing (2560) a first visual indicator in accordance with characteristics of the movement input received via the first joystick 212-1. The method 2500 also includes, in accordance with receiving a movement input at the second joystick 212-2, providing (2562) a second visual indicator in accordance with characteristics of the movement input received via the second joystick 212-2. For example, the visual indicator 211-1 may output light to indicate detection of an input at the first joystick 212-1 associated with the visual indicator 211-1. In another example, at least a portion of visual indicator 211-2 may output light to indicate detection of an input at the second joystick 212-2 associated with the visual indicator 211-2.

In some embodiments, the method 2500 also includes, in response to activating (e.g., pressing, engaging, disengaging) a control affordance (e.g., an affordance that combines the functions of lock control affordance 216-2 and unlock control affordance 216-3) of the input device at a first time, transition from operating the input device 201 in a first mode to operating the input device 201 in a second mode that is different from the first mode. The method also includes, in response to activating (e.g., pressing, engaging, disengaging) the control affordance of the input device at a second time after the first time, transitioning (2572) from operating the input device 201 from the second mode to operating the input device 201 in the first mode.

In some embodiments, the method 2500 further includes, in accordance with a determination that the input device 201 is operating in the first mode, orienting (2580) the motorized wheels of the one or more wheel assemblies 227 in a first predefined configuration (e.g., linear motion configuration shown in FIG. 23B, circular motion configuration shown in FIG. 23C) to enable movement of the rigid base 221 in the physical environment. The method also includes, in accordance with a determination the input device 201 is operating in the second mode, orienting (2582) the motorized wheels of the one or more wheel assemblies 227 in a second predefined configuration (e.g., preset braking configuration shown in FIG. 23D) to restrict movement of the rigid base 221 in the physical environment. The second predefined configuration is different from the first predefined configuration.

In some embodiments, the first predefined configuration includes any of: (i) orienting the wheels in the same direction for manual linear movement (as shown in FIG. 23B), (ii) orienting the wheels at predefined angles to one another for manual turning movement (as shown in FIG. 23C), (iii) orienting the wheels at predefined angles to one another for manual pivoting movement, and/or (iv) orienting the wheels in a configuration ready for power-assisted mobility.

In some embodiments, the method further includes, in response to detecting a falling motion of the input device 201 using an accelerometer of the input device 201, activating (2590) a lock-out mode in which user inputs directed to the input device 201 are ignored.

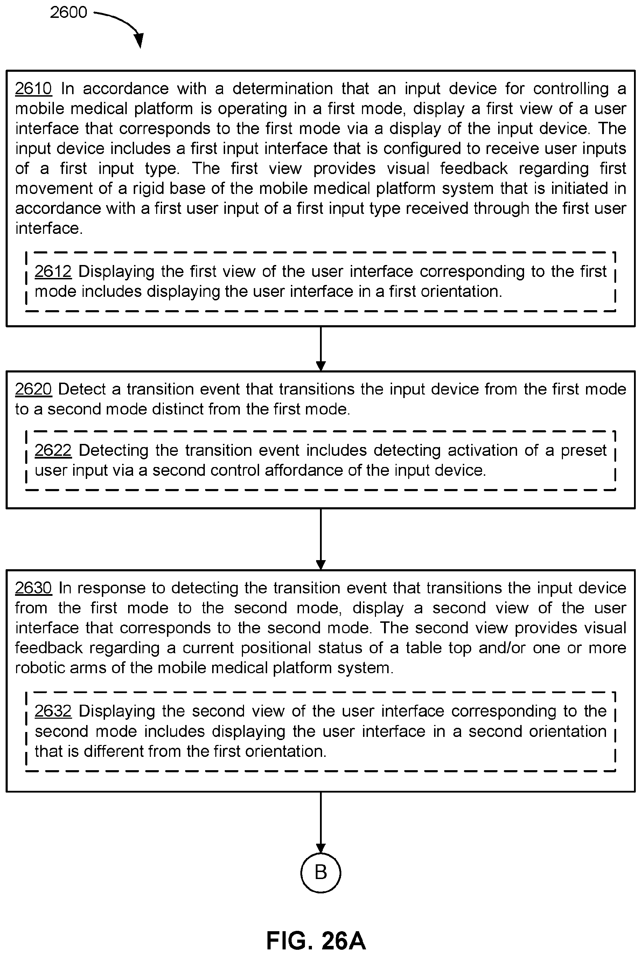

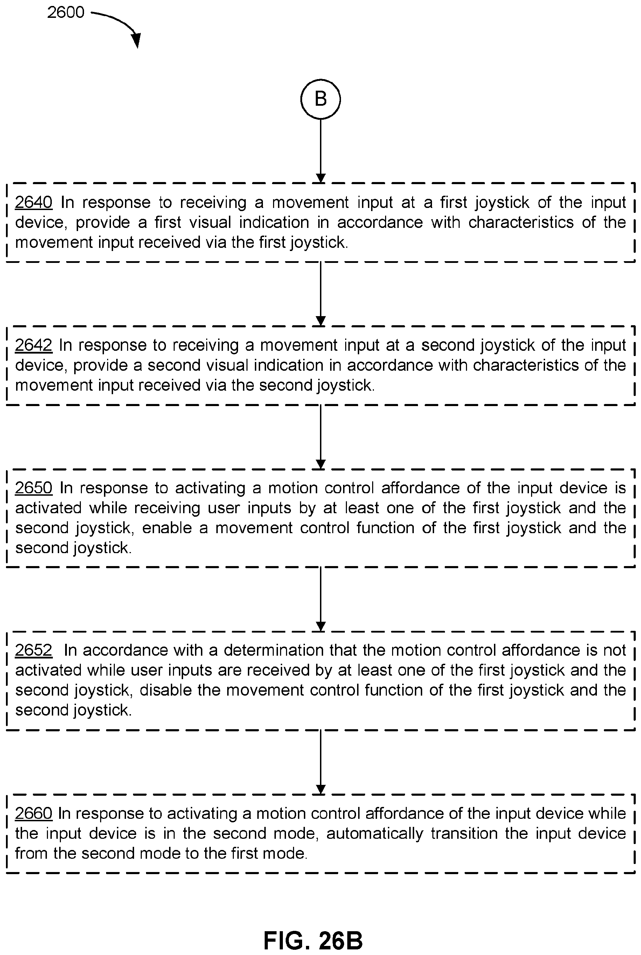

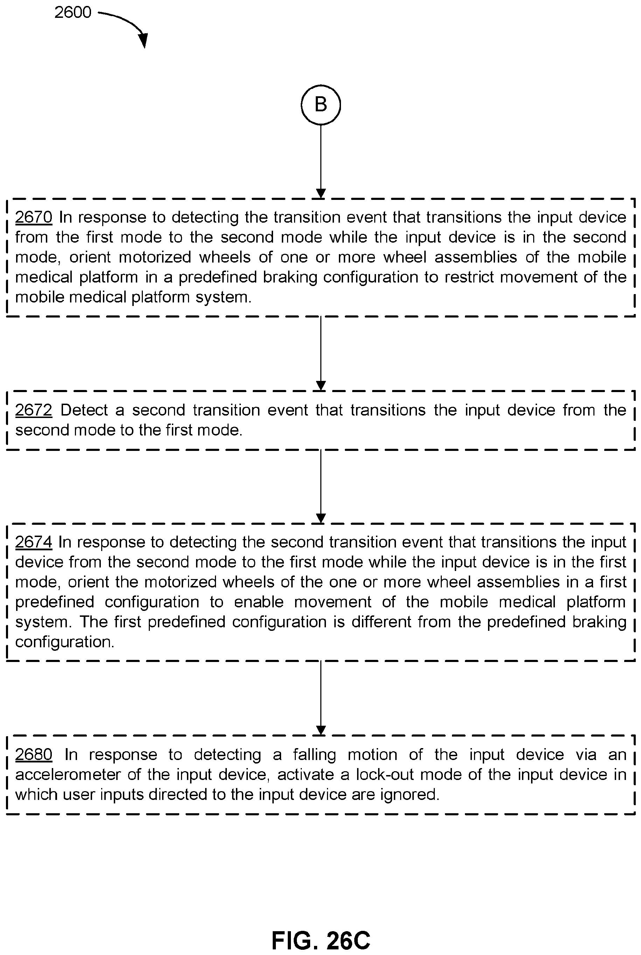

FIGS. 26A - 26C are a flowchart illustrating a method 2600 performed by an input device 201 for controlling operations of the mobile medical platform 200 of FIGs. 21A and 21B, in accordance with some embodiments. Method 2600 may be performed at an electronic device (such as mobile medical platform 200 or a computer system associated with mobile medical platform 200) having one or more processors and memory storing instructions for execution by the one or more processors (details of which are provided below with respect to FIG. 27). In some embodiments, the method 2500 is performed by executing instructions stored in the memory of the electronic device.

The method 2600 includes, in accordance with a determination that the input device 201 for controlling the mobile medical platform 200 is operating in a first mode (e.g., mobility mode, bed control mode) (e.g., determined in accordance with a current orientation of the input device being in a first orientation, and/or optionally, in accordance with activation of a motion control affordance 218), displaying (2610) a first view 230 of a user interface (e.g., user interface 230) that corresponds to the first mode via a display 214 of the input device 201. The input device includes a first input interface that is configured to receive user inputs of a first input type. The first view 230 provides visual feedback regarding first movement (e.g., rolling, steering, or both) of a rigid base 221 of the mobile medical platform system 100 that is initiated in accordance with a first user input of a input type received at the input device 201. FIG. 23A shows examples of the first view 230-1 and 230-2 of the user interface. The method 2600 also includes detecting a transition event that transitions the input device 201 from the first mode to a second mode (e.g., table top mode, bed status mode) that is distinct from the first mode. The method 2600 further includes, in response to detecting the transition event that transitions the input device 201 from the first mode to the second mode, displaying (2630) a second view 240 of the user interface (e.g., user interface 240) that corresponds to the second mode. The second view 240 provides visual feedback regarding a current positional status of a table top 225 and/or one or more robotic arms 205 of the mobile medical platform system 200 (e.g., robotic arm positions relative to the table top 225, arm and table top position 225 relative to the rigid base 221). FIG. 24A shows examples of the second view 240-1 and 240-2 of the user interface.

In some embodiments, the method 2600 includes, in accordance with a determination that the input device 201 is in a first physical orientation (e.g., horizontal orientation, landscape orientation) relative to a physical environment, operating the input device 201 in the first mode. The method 2600 also includes detecting a change in a physical orientation of the input device 201 from the first orientation to a second orientation (e.g., vertical orientation, portrait orientation) that is different from the first orientation, and in accordance with a determination that the input device 201 is in a second orientation relative to the physical environment, operating the input device 201 in the second mode. In some embodiments, operation of the input device 201 in the first mode corresponds to controlling power-assisted mobility functions of the mobile medical platform 200, such as driving (e.g., propelling, steering) the mobile medical platform 200. In some embodiments, operation of the input device 201 in the second mode corresponds to controlling table top functions of the mobile medical platform 200, such as reviewing table top 225 position, controlling table top functions and/or robotic arm functions, including stowing robotic arms, translating/tilting/pitching table top 225, and flexing or moving sections (e.g., sections 243 - 246) of the table top 225.

In some embodiments, displaying (2612) the first view 230 of the user interface corresponding to the first mode includes displaying the user interface in a first orientation (e.g., horizontal orientation, landscape orientation).

In some embodiments, displaying (2632) the second view 240 of the user interface corresponding to the second mode includes displaying the user interface in a second orientation (e.g., vertical orientation, portrait orientation) that is different from the first orientation.

In some embodiments, the method 2600 also includes, in response to receiving a movement input at a first joystick 212-1 of the input device 201, providing (2640) a first visual indication (e.g., via the visual indicator 211-1) in accordance with characteristics of the movement input received via the first joystick 212-1. The method also includes, in response to receiving a movement input at a second joystick 212-2 of the input device 201, providing (2642) a second visual indication (e.g., via the visual indicator 211-2) in accordance with characteristics of the movement input received via the second joystick 212-2. FIG. 22C illustrate an example of an input device 201 providing visual indications in accordance with detection of joystick movements.

In some embodiments, the method 2600 further includes in response to activating a motion control affordance 218 of the input device 201 is activated while receiving user inputs by at least one of the first joystick 212-1 and the second joystick 212-2, enabling (2650) a movement control function of the first joystick 212-1 and the second joystick 212-2. The method also includes, in accordance with a determination that the motion control affordance 218 is not activated while user inputs are received by at least one of the first joystick 212-1 and the second joystick 212-2, disable the movement control function of the first joystick 212-1 and the second joystick 212-2.

In some embodiments, the method 2600 also includes in response to activating a motion control affordance 218 of the input device 201 while the input device 201 is in the second mode, automatically transitioning (2660) the input device 201 from the second mode to the first mode.

In some embodiments, the method 2600 further includes, in response to detecting the transition event that transitions the input device 201 from the first mode to the second mode while the input device 201 is in the second mode, orienting (2670) motorized wheels of one or more wheel assemblies 227 of the mobile medical platform 200 in a predefined braking configuration to restrict movement of the mobile medical platform system 200. The method also includes detecting (2672) a second transition event that transitions the input device 201 from the second mode to the first mode, and in response to detecting the second transition event that transitions the input device 201 from the second mode to the first mode while the input device 201 is in the first mode, orienting (2674) the motorized wheels of the one or more wheel assemblies 227 in a first predefined configuration (such a linear motion configuration shown in FIG. 23B or a circular motion configuration shown in FIG. 23C) to enable movement of the mobile medical platform system 200. The first predefined configuration is different from the predefined braking configuration.

In some embodiments, the method 2600 also includes, in response to detecting a falling motion of the input device 201 via an accelerometer of the input device 201, activating (2680) a lock-out mode of the input device in which user inputs directed to the input device are ignored.

In some embodiments, the method 2600 includes, in response to detecting a falling motion of the input device 201, orienting the motorized wheels of the one or more wheel assemblies 227 in the predefined braking configuration shown in FIG. 23D.

Embodiments of the disclosure relate to systems and techniques for receiving user input at an input device for controlling functions of a mobile medical platform, such as a hospital bed or a surgical table.

### 3. Implementing Systems and Terminology.

FIG. 27 is a schematic diagram illustrating electronic components of a mobile medical platform in accordance with some embodiments.

The mobile medical platform 200 includes one or more processors 2700, which are in communication with a computer readable storage medium 2720 (e.g., computer memory devices, such as random-access memory, read-only memory, static random-access memory, and non-volatile memory, and other storage devices, such as a hard drive, an optical disk, a magnetic tape recording, or any combination thereof) storing instructions for performing any methods described herein (e.g., operations described with respect to FIGS. 25A - 25D and 26A - 26C). The one or more processors 2700 are also in communication with an input/output controller 2740 (via a system bus or any electrical circuit). The input/output controller 2740 receives instructions and/or data from an input device (e.g., a user input device 2760 that corresponds to input device 201) and relays the received instructions and/or data to the one or more processors 2700 (e.g., with or without any translation, conversion, and/or data processing). The input/output controller 2740 also receives instructions and/or data from the one or more processors 2700 and relays the instructions and/or data to one or more actuators, such as motors associated with the one or more wheel assemblies for providing power-assisted mobility to mobile medical platform, motors associated with positioning and orientation of the table top, and motors associated with movement of the one or more robotic arms. In some embodiments, the input/output controller 2740 is coupled to one or more actuator controllers 2790-1 through 2790-n and provides instructions and/or data to at least a subset of the one or more actuator controllers 2790-1 through 2790-n, which, in turn, provide control signals to selected actuators. In some embodiments, the one or more actuator controller 2790-1 through 2790-n are integrated with the input/output controller 2740 and the input/output controller 2740 provides control signals directly to the one or more actuators (without a separate actuator controller). Although FIG. 27 shows that there are separate actuator controllers 2790-1 through 2790-n (e.g., one actuator controller for each wheel assembly, one actuator for each robotic arm), in some embodiments, fewer actuator controllers may be used (e.g., one actuator controller for the entire mobile medical platform, or one actuator controller for a pair of wheel assemblies, etc.), additional actuator controllers may be used (e.g., one actuator controller for each actuator), or any combination thereof.

Embodiments disclosed herein provide systems, methods and apparatus for controlling operation of mobile medical platforms with power-assisted mobility and power-assisted table top positioning.

It should be noted that the terms "couple," "coupling," "coupled" or other variations of the word couple as used herein may indicate either an indirect connection or a direct connection. For example, if a first component is "coupled" to a second component, the first component may be either indirectly connected to the second component via another component or directly connected to the second component.

The functions of the user device to control operations for power-assisted mobilization and power-assisted table top positioning of a mobile medical platform described herein may be stored as one or more instructions on a processor-readable or computer-readable medium. The term "computer-readable medium" refers to any available medium that can be accessed by a computer or processor. By way of example, and not limitation, such a medium may comprise random access memory (RAM), read-only memory (ROM), electrically erasable programmable read-only memory (EEPROM), flash memory, compact disc read-only memory (CD-ROM) or other optical disk storage, magnetic disk storage or other magnetic storage devices, or any other medium that can be used to store desired program code in the form of instructions or data structures and that can be accessed by a computer. It should be noted that a computer-readable medium may be tangible and non-transitory. As used herein, the term "code" may refer to software, instructions, code or data that is/are executable by a computing device or processor.

The methods disclosed herein comprise one or more steps or actions for achieving the described method. The method steps and/or actions may be interchanged with one another without departing from the scope of the claims. In other words, unless a specific order of steps or actions is required for proper operation of the method that is being described, the order and/or use of specific steps and/or actions may be modified without departing from the scope of the claims.

As used herein, the term "plurality" denotes two or more. For example, a plurality of components indicates two or more components. The term "determining" encompasses a wide variety of actions and, therefore, "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing and the like.

The phrase "based on" does not mean "based only on," unless expressly specified otherwise. In other words, the phrase "based on" describes both "based only on" and "based at least on."

The previous description of the disclosed embodiments is provided to enable any person skilled in the art to make or use the present invention. Various modifications to these embodiments will be readily apparent to those skilled in the art, and the generic principles defined herein may be applied to other embodiments without departing from the scope of the invention.

## Claims

1. An input device (201) for controlling a mobile medical platform system (200) that includes a table top (225), a base (221), and one or more robotic arms (205), the input device comprising:
a first input interface that is configured to receive user inputs of a first input type;
a display (214);
one or more processors; and
memory storing instructions, which, when executed by the one or more processors, cause the processors to:
in accordance with a determination that the input device is operating in a first mode, display (2610), via the display, a first view (230) of a user interface that corresponds to the first mode, wherein the first view provides visual feedback regarding first movement of the base of the mobile medical platform system in a physical environment that is initiated in accordance with a first user input of the first input type received through the first input interface;
detect (2620) a transition event that transitions the input device from the first mode to a second mode distinct from the first mode; and
in response to detecting the transition event that transitions the input device from the first mode to the second mode, display (2630) a second view (240) of the user interface that corresponds to the second mode, wherein the second view provides visual feedback regarding a current positional status of the table top and/or the one or more robotic arms of the mobile medical platform system.

2. The input device of claim 1, wherein:
the first view of the user interface that corresponds to the first mode has a first orientation; and
the second view of the user interface that corresponds to the second mode has a second orientation that is different from the first orientation.

3. The input device of claim 1, further comprising:
a first joystick (212-1) and a second joystick (212-2), and
optionally:
a first visual indicator (211-1) disposed adjacent to the first joystick, and
a second visual indicator (211-2) disposed adjacent to the second joystick, wherein:
the first visual indicator is dynamically updated in accordance with characteristics of a movement input received via the first joystick, and the second visual indicator is dynamically updated in accordance with characteristics of a movement input received via the second joystick,
or:
a first control affordance (218), wherein the memory further stores instructions, which, when executed by the one or more processors, cause the processors to:
in accordance with a determination that the first control affordance is activated while user inputs are received by at least one of the first joystick and the second joystick, enable (2650) a movement control function of the first joystick and the second joystick; and
in accordance with a determination that the first control affordance is not activated while user inputs are received by at least one of the first joystick and the second joystick, disable (2652) the movement control function of the first joystick and the second joystick, and
optionally:
wherein the memory further stores instructions, which, when executed by the one or more processors, cause the processors to:
in accordance with a determination that the first control affordance is activated while the input device is in the second mode, automatically transition (2660) the input device from the second mode to the first mode.

4. The input device of claim 1, further comprising:
a second control affordance for transitioning the input device between the first mode and the second mode, wherein the transition event includes detection of a preset user input via the second control affordance of the input device.

5. The input device of claim 1, wherein:
the mobile medical platform system further includes one or more wheel assemblies (227), a respective wheel assembly of the one or more wheel assemblies including at least one motorized wheel, and
the memory storing instructions, which, when executed by the one or more processors, cause the processors to:
in response to detecting the transition event that transitions the input device from the first mode to the second mode and in accordance with a determination the input device is in the second mode, orient (2670) motorized wheels of the one or more wheel assemblies in a predefined braking configuration to restrict movement of the mobile medical platform system;
detect (2672) a second transition event that transitions the input device from the second mode to a first mode; and
in response to detecting the second transition event that transitions the input device from the second mode to the first mode and in accordance with a determination the input device is in the first mode, orient (2674) the motorized wheels of the one or more wheel assemblies in a first predefined configuration to enable movement of the table top, wherein the first predefined configuration is different from the predefined braking configuration.

6. The input device of claim 1, further comprising:
an accelerometer, wherein the memory further stores instructions, which, when executed by the one or more processors, cause the processors to:
in response to detecting a falling motion of the input device, activate a lock-out mode of the input device in which user inputs directed to the input device are ignored.

7. A mobile medical platform system (200), comprising:
a table with a base (221) and a table top (225);
one or more robotic arms (205) that are coupled to the table;
one or more wheel assemblies (227) that are coupled to the base to support and move the base in a physical environment, wherein a respective wheel assembly of the one or more wheel assemblies includes at least one motorized wheel;
the input device according to claim 1;
one or more processors; and
memory storing instructions, which, when executed by the one or more processors, cause the processors to:
receive (2510) a first user input of the first input type via the input device; and
in response to receiving the first user input of the first input type and in accordance with a determination that the first user input meets first criteria, initiate (2512) first movement of the at least one motorized wheel in accordance with the first user input of the first input type.

8. The mobile medical platform system of claim 7, wherein:
the input device is configured to receive user inputs of a second input type, and the memory further stores instructions, which, when executed by the one or more processors, cause the processors to:
receive (2520) a second user input of the second input type via the input device; and
in response to receiving the second user input of the second input type and in accordance with a determination that the second user input meets second criteria, initiate (2522) second movement of the table top relative to the base in accordance with the second user input of the second input type.

9. The mobile medical platform system of claim 7, wherein the input device is a pendant that is coupled to the table.

10. The mobile medical platform system of claim 7, wherein the display of the input device includes a touch sensitive display (214), and wherein the memory further stores instructions, which, when executed by the one or more processors, cause the processors to:
in accordance with a determination that the input device is in a first operation mode, present (2530) a first user interface (230) for controlling mobility of the mobile medical platform system, including one or more affordances (231) for controlling the at least one motorized wheel; and
in accordance with a determination that the input device is in a second operation mode, present (2532) a second user interface (240) for interacting with the table top, including one or more affordances (241) for changing a position of the table top relative to the base.

11. The mobile medical platform system of claim 7, wherein the input device includes a control affordance (216), and wherein the memory further stores instructions, which, when executed by the one or more processors, cause the processors to:
in accordance with a determination that the control affordance is activated at a first time, transition (2570) from operating the input device in a first mode to a second mode that is different from the first mode; and
in accordance with a determination that the control affordance is activated at a second time after the first time, transition (2572) from operating the input device from the second mode to the first mode,
optionally:
wherein the memory further stores instructions, which, when executed by the one or more processors, cause the processors to:
in accordance with a determination that the input device is operating in the first mode, display (2540) a first user interface with a first orientation on the display, wherein the first user interface includes one or more first affordances for controlling movement of the table; and
in accordance with a determination that the input device is operating in the second mode, display (2542) a second user interface via the display with a second orientation on the display, the second orientation being different from the first orientation, wherein the second user interface includes one or more second affordances for reviewing a status of the mobile medical platform system.

## Patentansprüche

1. Eingabevorrichtung (201) zum Steuern eines mobilen medizinischen Plattformsystems (200), das eine Tischplatte (225), eine Basis (221) und einen oder mehrere Roboterarme (205) einschließt, die Eingabevorrichtung umfassend:
eine erste Eingabeschnittstelle, die konfiguriert ist, um Benutzereingaben eines ersten Eingabetyps zu empfangen;
eine Anzeige (214);
einen oder mehrere Prozessoren; und
einen Speicher, der Anweisungen speichert, die, wenn sie durch den einen oder die mehreren Prozessoren ausgeführt werden, den Prozessor veranlassen zum:
gemäß einer Bestimmung, dass die Eingabevorrichtung in einem ersten Modus betrieben wird, Anzeigen (2610), über die Anzeige, einer ersten Ansicht (230) einer Benutzerschnittstelle, die dem ersten Modus entspricht, wobei die erste Ansicht visuelles Feedback bezüglich einer ersten Bewegung der Basis des mobilen medizinischen Plattformsystems in einer physischen Umgebung bereitstellt, die gemäß einer ersten Benutzereingabe des ersten Eingabetyps eingeleitet wird, die über die erste Eingabeschnittstelle empfangen wird;
Erkennen (2620) eines Überführungsereignisses, das die Eingabevorrichtung von dem ersten Modus in einen zweiten Modus überführt, der sich von dem ersten Modus unterscheidet; und
als Reaktion auf das Erkennen des Überführungsereignisses, das die Eingabevorrichtung von dem ersten Modus in den zweiten Modus überführt, Anzeigen (2630) einer zweiten Ansicht (240) der Benutzeroberfläche, die dem zweiten Modus entspricht, wobei die zweite Ansicht visuelles Feedback bezüglich eines aktuellen Positionsstatus der Tischplatte und/oder des einen oder der mehreren Roboterarme des mobilen medizinischen Plattformsystems bereitstellt.

2. Eingabevorrichtung nach Anspruch 1, wobei:
die erste Ansicht der Benutzeroberfläche, die dem ersten Modus entspricht, eine erste Ausrichtung aufweist; und
die zweite Ansicht der Benutzeroberfläche, die dem zweiten Modus entspricht, eine zweite Ausrichtung aufweist, die sich von der ersten Ausrichtung unterscheidet.

3. Eingabevorrichtung nach Anspruch 1, ferner umfassend:
einen ersten Joystick (212-1) und einen zweiten Joystick (212-2), und
optional:
eine erste visuelle Anzeige (211-1), die angrenzend an den ersten Joystick angeordnet ist, und
eine zweite visuelle Anzeige (211-2), die angrenzend an den zweiten Joystick angeordnet ist,
wobei:
der erste visuelle Indikator gemäß Eigenschaften einer Bewegungseingabe dynamisch aktualisiert wird, die über den ersten Joystick empfangen werden, und der zweite visuelle Indikator gemäß Eigenschaften einer Bewegungseingabe dynamisch aktualisiert wird, die über den zweiten Joystick empfangen werden,
oder:
eine erste Steueraffordanz (218), wobei der Speicher ferner Anweisungen speichert, die, wenn sie durch den einen oder die mehreren Prozessoren ausgeführt werden, die Prozessoren veranlassen zum:
gemäß einer Bestimmung, dass die erste Steueraffordanz freigegeben ist, während Benutzereingaben durch mindestens einen des ersten Joysticks und des zweiten Joysticks empfangen werden, Freigeben (2650) einer Bewegungssteuerungsfunktion des ersten Joysticks und des zweiten Joysticks; und
gemäß einer Bestimmung, dass die erste Steueraffordanz gesperrt ist, während Benutzereingaben durch mindestens einen des ersten Joysticks und des zweiten Joysticks empfangen werden, Sperren (2652) der Bewegungssteuerungsfunktion des ersten Joysticks und des zweiten Joysticks, und
optional:
wobei der Speicher ferner Anweisungen speichert, die, wenn sie durch den einen oder die mehreren Prozessoren ausgeführt werden, die Prozessoren veranlassen zum:
gemäß einer Bestimmung, dass die erste Steueraffordanz aktiviert ist, während sich die Eingabevorrichtung in dem zweiten Modus befindet, automatisches Überführen (2660) der Eingabevorrichtung von dem zweiten Modus in den ersten Modus.

4. Eingabevorrichtung nach Anspruch 1, ferner umfassend:
eine zweite Steueraffordanz zum Überführen der Eingabevorrichtung zwischen dem ersten Modus und dem zweiten Modus, wobei das Überführungsereignis die Erkennung einer voreingestellten Benutzereingabe über die zweite Steueraffordanz der Eingabevorrichtung umfasst.

5. Eingabevorrichtung nach Anspruch 1, wobei:
das mobile medizinische Plattformsystem ferner eine oder mehrere Radanordnungen (227) umfasst, wobei eine jeweilige Radanordnung der einen oder der mehreren Radanordnungen mindestens ein motorisiertes Rad einschließt, und
der Speicher Anweisungen speichert, die, wenn sie durch den einen oder die mehreren Prozessoren ausgeführt werden, den Prozessor veranlassen zum:
als Reaktion auf das Erkennen des Überführungsereignisses, das die Eingabevorrichtung von dem ersten Modus in den zweiten Modus überführt, und gemäß einer Bestimmung, dass sich die Eingabevorrichtung in dem zweiten Modus befindet, Ausrichten (2670) von motorisierten Rädern der einen oder der mehreren Radanordnungen in einer vordefinierten Bremskonfiguration, um eine Bewegung des mobilen medizinischen Plattformsystems einzuschränken;
Erkennen (2672) eines zweiten Überführungsereignisses, das die Eingabevorrichtung von dem zweiten Modus in einen ersten Modus überführt; und
als Reaktion auf das Erkennen des zweiten Überführungsereignisses, das die Eingabevorrichtung von dem zweiten Modus in den ersten Modus überführt, und gemäß einer Bestimmung, dass sich die Eingabevorrichtung in dem ersten Modus befindet, Ausrichten (2674) der motorisierten Räder der einen oder der mehreren Radanordnungen in einer ersten vordefinierten Konfiguration, um eine Bewegung der Tischplatte freizugeben, wobei sich die erste vordefinierte Konfiguration von der vordefinierten Bremskonfiguration unterscheidet.

6. Eingabevorrichtung nach Anspruch 1, ferner umfassend:
einen Beschleunigungsmesser, wobei der Speicher ferner Anweisungen speichert, die, wenn sie durch den einen oder die mehreren Prozessoren ausgeführt werden, die Prozessoren veranlassen zum:
als Reaktion auf das Erkennen einer Fallbewegung der Eingabevorrichtung, Aktivieren eines Aussperrmodus der Eingabevorrichtung, in dem Benutzereingaben ignoriert werden, die an die Eingabevorrichtung gerichtet sind.

7. Mobiles medizinisches Plattformsystem (200), umfassend:
einen Tisch mit einer Basis (221) und einer Tischplatte (225);
einen oder mehrere Roboterarme (205), die mit dem Tisch gekoppelt sind;
eine oder mehrere Radanordnungen (227), die mit der Basis gekoppelt sind, um die Basis in einer physischen Umgebung zu stützen und zu bewegen, wobei eine jeweilige Radanordnung der einen oder der mehreren Radanordnungen mindestens ein motorisiertes Rad einschließt;
die Eingabevorrichtung nach Anspruch 1;
einen oder mehrere Prozessoren; und
einen Speicher, der Anweisungen speichert, die, wenn sie durch den einen oder die mehreren Prozessoren ausgeführt werden, den Prozessor veranlassen zum:
Empfangen (2510) einer ersten Benutzereingabe des ersten Eingabetyps über die Eingabevorrichtung; und
als Reaktion auf das Empfangen der ersten Benutzereingabe des ersten Eingabetyps und gemäß einer Bestimmung, dass die erste Benutzereingabe erste Kriterien erfüllt, Einleiten (2512) einer ersten Bewegung des mindestens einen motorisierten Rads gemäß der ersten Benutzereingabe des ersten Eingabetyps.

8. Mobiles medizinisches Plattformsystem nach Anspruch 7, wobei:
die Eingabevorrichtung konfiguriert ist, um Benutzereingaben eines zweiten Eingabetyps zu empfangen, und der Speicher ferner Anweisungen speichert, die, wenn sie durch den einen oder die mehreren Prozessoren ausgeführt werden, die Prozessoren veranlassen zum:
Empfangen (2520) einer zweiten Benutzereingabe des zweiten Eingabetyps über die Eingabevorrichtung; und
als Reaktion auf das Empfangen der zweiten Benutzereingabe des zweiten Eingabetyps und gemäß einer Bestimmung, dass die zweite Benutzereingabe zweite Kriterien erfüllt, Einleiten (2522) einer zweiten Bewegung der Tischplatte relativ zu der Basis gemäß der zweiten Benutzereingabe des zweiten Eingabetyps.

9. Mobiles medizinisches Plattformsystem nach Anspruch 7, wobei die Eingabevorrichtung ein Schwenkarm ist, der mit dem Tisch gekoppelt ist.

10. Mobiles medizinisches Plattformsystem nach Anspruch 7, wobei die Anzeige der Eingabevorrichtung eine berührungsempfindliche Anzeige (214) einschließt und wobei der Speicher ferner Anweisungen speichert, die, wenn sie durch den einen oder die mehreren Prozessoren ausgeführt werden, die Prozessoren veranlassen zum:
gemäß einer Bestimmung, dass sich die Eingabevorrichtung in einem ersten Betriebsmodus befindet, Präsentieren (2530) einer ersten Benutzerschnittstelle (230) zum Steuern einer Mobilität des mobilen medizinischen Plattformsystems, einschließlich einer oder mehrerer Affordanzen (231) zum Steuern des mindestens einen motorisierten Rads; und
gemäß einer Bestimmung, dass sich die Eingabevorrichtung in einem zweiten Betriebsmodus befindet, Präsentieren (2532) einer zweiten Benutzerschnittstelle (240) zum Interagieren mit der Tischplatte, einschließlich einer oder mehrerer Affordanzen (241) zum Ändern einer Position der Tischplatte relativ zu der Basis.

11. Mobiles medizinisches Plattformsystem nach Anspruch 7, wobei die Eingabevorrichtung eine Steueraffordanz (216) einschließt und wobei der Speicher ferner Anweisungen speichert, die, wenn sie durch den einen oder die mehreren Prozessoren ausgeführt werden, die Prozessoren veranlassen zum:
gemäß einer Bestimmung, dass die Steueraffordanz zu einem ersten Zeitpunkt aktiviert ist, Überführen (2570) von dem Betreiben der Eingabevorrichtung in einem ersten Modus in einen zweiten Modus, der sich von dem ersten Modus unterscheidet; und
gemäß einer Bestimmung, dass die Steueraffordanz zu einem zweiten Zeitpunkt nach dem ersten Zeitpunkt aktiviert ist, Überführen (2572) von dem Betreiben der Eingabevorrichtung von dem zweiten Modus in den ersten Modus,
optional:
wobei der Speicher ferner Anweisungen speichert, die, wenn sie durch den einen oder die mehreren Prozessoren ausgeführt werden, die Prozessoren veranlassen zum:
gemäß einer Bestimmung, dass die Eingabevorrichtung in dem ersten Modus betrieben wird, Anzeigen (2540) einer ersten Benutzerschnittstelle mit einer ersten Ausrichtung auf der Anzeige, wobei die erste Benutzerschnittstelle eine oder mehrere erste Affordanzen zum Steuern der Bewegung des Tisches einschließt; und
gemäß einer Bestimmung, dass die Eingabevorrichtung in dem zweiten Modus betrieben wird, Anzeigen (2542) einer zweiten Benutzerschnittstelle über die Anzeige mit einer zweiten Ausrichtung auf der Anzeige, wobei sich die zweite Ausrichtung von der ersten Ausrichtung unterscheidet, wobei die zweite Benutzerschnittstelle eine oder mehrere zweite Affordanzen zum Überprüfen eines Status des mobilen medizinischen Plattformsystems einschließt.

## Revendications

1. Dispositif d'entrée (201) permettant de commander un système de plate-forme médicale mobile (200) qui comporte un plateau (225), une base (221), et un ou plusieurs bras robotisés (205), le dispositif d'entrée comprenant :
une première interface d'entrée qui est configurée pour recevoir des entrées utilisateur d'un premier type d'entrée ;
un écran (214) ;
un ou plusieurs processeurs ; et
une mémoire stockant des instructions qui, lorsqu'elles sont exécutées par les un ou plusieurs processeurs, amènent les processeurs à :
conformément à une détermination selon laquelle le dispositif d'entrée fonctionne dans un premier mode, afficher (2610), par le biais de l'écran, une première vue (230) d'une interface utilisateur qui correspond au premier mode, dans lequel la première vue fournit une rétroaction visuelle concernant un premier mouvement de la base du système de plate-forme médicale mobile dans un environnement physique qui est induit conformément à une première entrée utilisateur du premier type d'entrée reçue par l'intermédiaire de la première interface d'entrée ;
détecter (2620) un événement de transition qui fait passer le dispositif d'entrée du premier mode à un second mode distinct du premier mode ; et
en réponse à la détection de l'événement de transition qui fait passer le dispositif d'entrée du premier mode au second mode, afficher (2630) une seconde vue (240) de l'interface utilisateur qui correspond au second mode, dans lequel la seconde vue fournit une rétroaction visuelle concernant un état de position actuel du plateau et/ou des un ou plusieurs bras robotisés du système de plate-forme médicale mobile.

2. Dispositif d'entrée selon la revendication 1, dans lequel :
la première vue de l'interface utilisateur qui correspond au premier mode a une première orientation ; et
la seconde vue de l'interface utilisateur qui correspond au second mode a une seconde orientation qui est différente de la première orientation.

3. Dispositif d'entrée selon la revendication 1, comprenant en outre :
un premier levier de commande (212-1) et un second levier de commande (212-2), et
éventuellement :
un premier témoin visuel (211-1) disposé à côté du premier levier de commande, et
un second témoin visuel (211-2) disposé à côté du second levier de commande,
dans lequel :
le premier témoin visuel est mis à jour de manière dynamique conformément à des caractéristiques d'une entrée de mouvement reçue par le biais du premier levier de commande, et le second témoin visuel est mis à jour de manière dynamique conformément à des caractéristiques d'une entrée de mouvement reçue par le biais du second levier de commande,
ou :
une première affordance de commande (218), dans lequel la mémoire stocke en outre des instructions qui, lorsqu'elles sont exécutées par les un ou plusieurs processeurs, amènent les processeurs à :
conformément à une détermination selon laquelle la première affordance de commande est activée pendant que des entrées utilisateur sont reçues par au moins un du premier levier de commande et du second levier de commande, activer (2650) une fonction de commande de mouvement du premier levier de commande et du second levier de commande ; et
conformément à une détermination selon laquelle la première affordance de commande n'est pas activée pendant que des entrées utilisateur sont reçues par au moins un du premier levier de commande et du second levier de commande, désactiver (2652) la fonction de commande de mouvement du premier levier de commande et du second levier de commande, et
éventuellement :
dans lequel la mémoire stocke en outre des instructions qui, lorsqu'elles sont exécutées par les un ou plusieurs processeurs, amènent les processeurs à :
conformément à une détermination selon laquelle la première affordance de commande est activée pendant que le dispositif d'entrée est dans le second mode, faire passer automatiquement (2660) le dispositif d'entrée du second mode au premier mode.

4. Dispositif d'entrée selon la revendication 1, comprenant en outre :
une seconde affordance de commande pour faire passer le dispositif d'entrée du premier mode au second mode, dans lequel l'événement de transition comporte la détection d'une entrée utilisateur prédéfinie par le biais de la seconde affordance du dispositif d'entrée.

5. Dispositif d'entrée selon la revendication 1, dans lequel :
le système de plate-forme médicale mobile comporte en outre un ou plusieurs ensembles de roues (227), un ensemble de roues respectif des un ou plusieurs ensembles de roues comportant au moins une roue motorisée, et
la mémoire stockant des instructions qui, lorsqu'elles sont exécutées par les un ou plusieurs processeurs, amènent les processeurs à :
en réponse à la détection de l'événement de transition qui fait passer le dispositif d'entrée du premier mode au second mode et conformément à une détermination selon laquelle le dispositif d'entrée est dans le second mode, orienter (2670) des roues motorisées des un ou plusieurs ensembles de roues dans une configuration de freinage prédéfinie pour restreindre un mouvement du système de plate-forme médicale mobile ;
détecter (2672) un second événement de transition qui fait passer le dispositif d'entrée du second mode à un premier mode ; et
en réponse à la détection du second événement de transition qui fait passer le dispositif d'entrée du second mode au premier mode et conformément à une détermination selon laquelle le dispositif d'entrée est dans le premier mode, orienter (2674) les roues motorisées des un ou plusieurs ensembles de roues dans une première configuration prédéfinie pour permettre un mouvement du plateau, dans lequel la première configuration prédéfinie est différente de la configuration de freinage prédéfinie.

6. Dispositif d'entrée selon la revendication 1, comprenant en outre :
un accéléromètre, dans lequel la mémoire stocke en outre des instructions qui, lorsqu'elles sont exécutées par les un ou plusieurs processeurs, amènent les processeurs à :
en réponse à la détection d'un mouvement de chute du dispositif d'entrée, activer un mode de verrouillage du dispositif d'entrée dans lequel des entrées utilisateur concernant le dispositif d'entrée sont ignorées.

7. Système de plate-forme médicale mobile (200), comprenant :
une table équipée d'une base (221) et d'un plateau (225) ;
un ou plusieurs bras robotisés (205) qui sont accouplés à la table ;
un ou plusieurs ensembles de roues (227) qui sont accouplés à la base pour supporter et déplacer la base dans un environnement physique, dans lequel un ensemble de roues respectif des un ou plusieurs ensembles de roues comporte au moins une roue motorisée ;
le dispositif d'entrée selon la revendication 1 ;
un ou plusieurs processeurs ; et
une mémoire stockant des instructions qui, lorsqu'elles sont exécutées par les un ou plusieurs processeurs, amènent les processeurs à :
recevoir (2510) une première entrée utilisateur du premier type d'entrée par le biais du dispositif d'entrée ; et
en réponse à la réception de la première entrée utilisateur du premier type d'entrée et conformément à une détermination selon laquelle la première entrée utilisateur répond à de premiers critères, induire (2512) un premier mouvement de l'au moins une roue motorisée conformément à la première entrée utilisateur du premier type d'entrée.

8. Système de plate-forme médicale mobile selon la revendication 7, dans lequel :
le dispositif d'entrée est configuré pour recevoir des entrées utilisateur d'un second type d'entrée, et la mémoire stocke en outre des instructions qui, lorsqu'elles sont exécutées par les un ou plusieurs processeurs, amènent les processeurs à :
recevoir (2520) une seconde entrée utilisateur du second type d'entrée par le biais du dispositif d'entrée ; et
en réponse à la réception de la seconde entrée utilisateur du second type d'entrée et conformément à une détermination selon laquelle la seconde entrée utilisateur répond à de seconds critères, induire (2522) un second mouvement du plateau par rapport à la base conformément à la seconde entrée utilisateur du second type d'entrée.

9. Système de plate-forme médicale mobile selon la revendication 7, dans lequel le dispositif d'entrée est un élément suspendu accouplé à la table.

10. Système de plate-forme médicale mobile selon la revendication 7, dans lequel l'écran du dispositif d'entrée comporte un écran tactile (214), et dans lequel la mémoire stocke en outre des instructions qui, lorsqu'elles sont exécutées par les un ou plusieurs processeurs, amènent les processeurs à :
conformément à une détermination selon laquelle le dispositif d'entrée est dans un premier mode de fonctionnement, présenter (2530) une première interface utilisateur (230) pour commander la mobilité du système de plate-forme médicale mobile, y compris une ou plusieurs affordances (231) pour commander l'au moins une roue motorisée ; et
conformément à une détermination selon laquelle le dispositif d'entrée est dans un second mode de fonctionnement, présenter (2532) une seconde interface utilisateur (240) pour interagir avec le plateau, y compris une ou plusieurs affordances (241) pour changer une position du plateau par rapport à la base.

11. Système de plate-forme médicale mobile selon la revendication 7, dans lequel le dispositif d'entrée comporte une affordance de commande (216), et dans lequel la mémoire stocke en outre des instructions qui, lorsqu'elles sont exécutées par les un ou plusieurs processeurs, amènent les processeurs à :
conformément à une détermination selon laquelle l'affordance de commande est activée à un premier moment, faire passer (2570) le fonctionnement du dispositif d'entrée d'un premier mode à un second mode qui est différent du premier mode ; et
conformément à une détermination selon laquelle l'affordance de commande est activée à un second moment après le premier moment, faire passer (2572) le fonctionnement du dispositif d'entrée du second mode au premier mode,
éventuellement :
dans lequel la mémoire stocke en outre des instructions qui, lorsqu'elles sont exécutées par les un ou plusieurs processeurs, amènent les processeurs à :
conformément à une détermination selon laquelle le dispositif d'entrée fonctionne dans le premier mode, afficher (2540) une première interface utilisateur avec une première orientation sur l'écran, dans lequel la première interface utilisateur comporte une ou plusieurs premières affordances pour commander le mouvement de la table ; et
conformément à une détermination selon laquelle le dispositif d'entrée fonctionne dans le second mode, afficher (2542) une seconde interface utilisateur par le biais de l'écran avec une seconde orientation sur l'écran, la seconde orientation étant différente de la première orientation, dans lequel la seconde interface utilisateur comporte une ou plusieurs secondes affordances pour examiner un état du système de plate-forme médicale mobile.
